# EUROPEAN PATENT SPECIFICATION

(11) **EP 2 648 743 B1**
(45) Date of publication and mention of the grant of the patent: **14.01.2015**
(21) Application number: 11820837.0
(22) Date of filing: 07.12.2011
(51) Int. Cl.: A61K 38/16, C07K 7/06, C12N 15/00, C12N 15/80, C12N 15/82, C07K 14/415

(54) **AGENTS FOR INCREASED RESISTANCE AGAINST OXIDATIVE STRESS CONDITIONS**
MITTEL ZUR ERHÖHUNG DER RESISTENZ GEGEN OXIDATIVEN STRESS
AGENTS POUVANT CONFÉRER UNE RÉSISTANCE ACCRUE AUX CONDITIONS D'UN STRESS OXYDATIF

(30) Priority: 08.12.2010 GB 201020737
(43) Date of publication of application: 16.10.2013
(73) Proprietor: Katholieke Universiteit Leuven, 3000 Leuven (BE)
(72) Inventor: CAMMUE, Bruno, B-1652 Alsemberg (BE); CARRON, Delphine, B-1780 Wemmel (BE); CASSIMAN, David, B-3360 Korbeek-Lo (BE); DE CONINCK, Barbara, B-9400 Ninove (BE); MATHYS, Janick, B-3001 Heverlee (BE); SPINCEMAILLE, Pieter, B-3110 Rotselaar (BE); THEVISSEN, Karin, B-3360 Bierbeek (BE)
(74) Representative: Laenen, Bart Roger Albert
(86) International application number: PCT/BE2011/000070
(87) International publication number: WO 2012/075549

(56) References cited:
- XINXIN ZHANG ET AL: "Overexpression of a mitochondrial ATP synthase small subunit gene (AtMtATP6) confers tolerance to several abiotic stresses in Saccharomyces cerevisiae and Arabidopsis thaliana", BIOTECHNOLOGY LETTERS, SPRINGER NETHERLANDS, DORDRECHT, vol. 30, no. 7, 13 March 2008 (2008-03-13) , pages 1289-1294, XP019599953, ISSN: 1573-6776

### SEQUENCE LISTING

<110> Katholieke Universiteit Leuven - K.U.Leuven R&D
<120> Agents for increased resistance against oxidative stress conditions
<130> 2010106
<140> Not yet known
   <141> 2011-12-07
<150> GB 1020737.1
   <151> 2010-12-08
<160> 582
<170> PatentIn version 3.5
<210> 1
   <211> 10
   <212> PRT
   <213> Arabidopsis thaliana
<400> 1
   Met Leu Cys Val Leu Gln Gly Leu Arg Glu
   1               5                   10
<210> 2
   <211> 17
   <212> PRT
   <213> Arabidopsis thaliana
<400> 2
   Met Ile Ile Ile Asn Asn Asp Asn Tyr Leu Leu Leu Phe Tyr Asn Asn
   1               5                   10                  15
   Asn
<210> 3
   <211> 13
   <212> PRT
   <213> Arabidopsis thaliana
<400> 3
   Met Ile Ile Ile Tyr Tyr Cys Phe Ile Ile Ile Ile Asn
   1               5                   10
<210> 4
   <211> 11
   <212> PRT
   <213> Arabidopsis thaliana
<400> 4
   Met Leu Met Tyr Arg Met Arg Ser Gly Ala Asn
   1               5                   10
<210> 5
   <211> 18
   <212> PRT
   <213> Arabidopsis thaliana
<400> 5
   Met Gly Leu Asn Glu Asp Ser Val Phe Arg Ser Ile Lys Pro Phe Lys
   1               5                   10                  15
   Ser Pro
<210> 6
   <211> 39
   <212> PRT
   <213> Arabidopsis thaliana
<400> 6
   Met Asn Val Leu Ala Arg Ala Pro Arg Leu Arg His Gln Leu Gln Asn
   1               5                   10                  15
   Leu Thr Gln Asp Arg Arg Lys Thr Gln Met Gln Met Lys Gly Gln Arg
               20                  25                  30
   Val Arg Thr Thr Ser Leu Gln
           35
<210> 7
   <211> 21
   <212> PRT
   <213> Arabidopsis thaliana
<400> 7
   Met Ala Arg Glu Glu Lys Glu Gln Ser Val Tyr Asp Ile Tyr Thr Phe
   1               5                   10                  15
   Ala Ser Leu Leu Leu
               20
<210> 8
   <211> 23
   <212> PRT
   <213> Arabidopsis thaliana
<400> 8
   Met His Cys Leu Leu Ser Leu Leu Glu Met Val Pro Ser Asn Leu Leu
   1               5                   10                  15
   Ser Ala Leu Leu Asn Leu Asn
               20
<210> 9
   <211> 26
   <212> PRT
   <213> Arabidopsis thaliana
<400> 9
   Met Ser Val Glu Ile Cys Arg Arg Tyr Tyr Leu Pro Pro Pro Asn Asn
   1               5                   10                  15
   Cys Thr Tyr Thr Thr Pro Gln Thr Ser Ile
               20                  25
<210> 10
   <211> 13
   <212> PRT
   <213> Arabidopsis thaliana
<400> 10
   Met Ile Cys Tyr Lys Asn Leu Ala Arg Gln Asn Ile Asn
   1               5                   10
<210> 11
   <211> 10
   <212> PRT
   <213> Arabidopsis thaliana
<400> 11
   Met Ser Ser Tyr Gln Ser Ile Tyr Glu Cys
   1 5 10
<210> 12
   <211> 14
   <212> PRT
   <213> Arabidopsis thaliana
<400> 12
   Met Ile His Ile His Ile Asn Trp Arg Leu Ile Arg Ile Gly
   1 5 10
<210> 13
   <211> 24
   <212> PRT
   <213> Arabidopsis thaliana
<400> 13
   Met His Ile Tyr Ala Leu Ala Asn Ser Asn Gln Ser Pro Ile Asn Val
   1 5 10 15
   Tyr Met Tyr His Ser His Arg Pro
   20
<210> 14
   <211> 21
   <212> PRT
   <213> Arabidopsis thaliana
<400> 14
   Met Cys Ile Cys Ile Ile His Ile Gly Pro Arg Leu Ile Leu Thr Ile
   1 5 10 15
   Leu Gln Tyr Val Phe
   20
<210> 15
   <211> 75
   <212> PRT
   <213> Arabidopsis thaliana
<400> 15
   Met Ser Val Lys Lys Arg Ala Leu Glu Ser Lys Asn Asn Gly Ser Pro
   1               5                   10                  15
   Lys Ser Ser His Tyr Asn Arg Cys Leu Ser Phe Ser Phe Leu Glu Ser
               20                  25                  30
   Ser Thr Gly Glu Lys Lys Lys Pro Ser Ser Leu Asn Arg Met Asp Ser
           35                  40                  45
   Lys Ile Leu Lys Ala Glu Ile Val Lys Trp Ala Lys Arg Val Ala Ala
       50                  55                  60
   Tyr Ala Arg Gln Leu Ser Ser Arg Lys Gln Asp
   65                  70                  75
<210> 16
   <211> 13
   <212> PRT
   <213> Arabidopsis thaliana
<400> 16
   Met Lys Leu Val Gln Ala Asp Pro Phe Ser Asp Cys Phe
   1               5                   10
<210> 17
   <211> 30
   <212> PRT
   <213> Arabidopsis thaliana
<400> 17
   Met Glu Ala Pro Ser Arg Val Ile Ile Ile Asp Ala Cys His Ser Arg
   1               5                   10                  15
   Ser Trp Ser His Gln Arg Glu Arg Arg Arg Asn His His Arg
               20                  25                  30
<210> 18
   <211> 13
   <212> PRT
   <213> Arabidopsis thaliana
<400> 18
   Met Leu Val Asn Leu Ala Arg Glu Asn Lys Ile Lys Thr
   1               5                   10
<210> 19
   <211> 25
   <212> PRT
   <213> Arabidopsis thaliana
<400> 19
   Met Lys Lys Ala Tyr Gly Lys Ile Asp Met His Ser Phe Tyr Ile Asp
   1 5 10 15
   Leu Gly Pro His Ser Phe Asn His Ser
   20 25
<210> 20
   <211> 33
   <212> PRT
   <213> Arabidopsis thaliana
<400> 20
   Met Leu Val Ile Leu Val Leu Gly Val Ile Asn Gly Arg Glu Glu Glu
   1 5 10 15
   Thr Ile Ile Val Glu Pro Tyr Gly Phe Gln Asn Thr Gln Gly Arg Asp
   20 25 30
   Arg
<210> 21
   <211> 12
   <212> PRT
   <213> Arabidopsis thaliana
<400> 21
   Met Gly Arg Leu Ile Cys Thr His Phe Thr Leu Ile
   1 5 10
<210> 22
   <211> 13
   <212> PRT
   <213> Arabidopsis thaliana
<400> 22
   Met Leu Arg Ser His Glu Thr Cys Thr Ser Arg Ser Ile
   1 5 10
<210> 23
   <211> 15
   <212> PRT
   <213> Arabidopsis thaliana
<400> 23
   Met Ser Ala Tyr Gln Ser Ser His Lys Leu Ser Ser Ser Met Ser
   1 5 10 15
<210> 24
   <211> 37
   <212> PRT
   <213> Arabidopsis thaliana
<400> 24
   Met Asn Lys Thr Gln Ser His Leu Leu Phe Ser Leu Ser Thr Cys Leu
   1 5 10 15
   Asn Ser Tyr Val Asn Thr Lys Leu Pro Ser Ile Leu Val Asn Ser Lys
   20 25 30
   Leu Ser Ile Asn Thr
   35
<210> 25
   <211> 10
   <212> PRT
   <213> Arabidopsis thaliana
<400> 25
   Met Asn His Leu Gln Ile Ser Leu Ile Ser
   1 5 10
<210> 26
   <211> 32
   <212> PRT
   <213> Arabidopsis thaliana
<400> 26
   Met Pro Lys Leu Ile Cys Glu His Gln Thr Pro Ile His Ser Arg Gln
   1 5 10 15
   Phe Lys Ala Leu Tyr Gln Tyr Ile Thr Ser Ile Thr Leu Pro Met Arg
   20 25 30
<210> 27
   <211> 15
   <212> PRT
   <213> Arabidopsis thaliana
<400> 27
   Met Gly Val Trp Cys Ser His Met Ser Leu Gly Met Leu Ile Ser
   1 5 10 15
<210> 28
   <211> 16
   <212> PRT
   <213> Arabidopsis thaliana
<400> 28
   Met Gly Arg Gly Lys Val Trp Val Val Glu Gln Ser Arg Arg Lys Ile
   1 5 10 15
<210> 29
   <211> 22
   <212> PRT
   <213> Arabidopsis thaliana
<400> 29
   Met Asp Gly Ser Leu Val Phe Thr Tyr Glu Phe Arg His Val Asp Lys
   1 5 10 15
   Leu Lys Ser Lys Trp Asp
   20
<210> 30
   <211> 137
   <212> PRT
   <213> Arabidopsis thaliana
<400> 30
   Met Val Lys Trp Ser Gly Asp Val Thr Thr Asp Cys Ile Leu Cys Gln
   1 5 10 15
   Gly Asn Leu Glu Thr Arg Glu His Leu Phe Phe Asp Cys Gly Tyr Thr
   20 25 30
   Ser Ala Val Trp Ala Ala Leu Val Lys Gly Ile Leu Lys Ser Arg Tyr
   35 40 45
   Thr Ser Asn Trp Thr Ser Ile Met Asp His Leu Ala His Ala Gln Pro
   50 55 60
   His Arg Val Asp His Phe Leu Val Arg Tyr Ala Phe Gln Ala Thr Leu
   65 70 75 80
   Tyr Thr Val Trp Arg Glu Arg Asn Gly Arg Arg His Gly Glu Thr Leu
   85 90 95
   Asn Thr Ala Ser Gln Leu Val Gly Trp Ile Asp Lys Gln Ile Arg Asn
   100 105 110
   Gln Leu Ser Ser Ile Lys Leu Lys Gly Asp Arg Arg Tyr Asp Asp Ala
   115 120 125
   Leu Gln Leu Trp Phe Ser Thr Arg Val
   130 135
<210> 31
   <211> 41
   <212> PRT
   <213> Arabidopsis thaliana
<400> 31
   Met Leu Ser His Thr Ala Trp Thr Thr Ser Ser Phe Ala Met Pro Ser
   1 5 10 15
   Lys Arg Leu Tyr Thr Leu Cys Gly Gly Lys Glu Met Val Asp Asp Thr
   20 25 30
   Val Arg Pro Ser Thr Arg Pro Leu Asn
   35 40
<210> 32
   <211> 14
   <212> PRT
   <213> Arabidopsis thaliana
<400> 32
   Met Val Gly Arg Arg Tyr Asn Gly Leu His Ser Leu Ser Arg
   1 5 10
<210> 33
   <211> 21
   <212> PRT
   <213> Arabidopsis thaliana
<400> 33
   Met Asp Arg Gln Ala Asn Pro Lys Ser Ala Val Leu Asn Gln Ala Gln
   1 5 10 15
   Gly Arg Ser Thr Ile
   20
<210> 34
   <211> 28
   <212> PRT
   <213> Arabidopsis thaliana
<400> 34
   Met Ser Glu Val Val His Tyr Arg Gly Pro Val Gly Ser Ile Ser Arg
   1 5 10 15
   Phe Glu Asn Ser Leu Tyr Lys Arg Cys Pro Asn Cys
   20 25
<210> 35
   <211> 21
   <212> PRT
   <213> Arabidopsis thaliana
<400> 35
   Met Gln Arg Glu Leu Lys Glu Phe Lys Leu Lys Ile Glu Phe Glu Lys
   1 5 10 15
   Lys Glu Thr Lys Leu
   20
<210> 36
   <211> 31
   <212> PRT
   <213> Arabidopsis thaliana
<400> 36
   Met Asp Asn Arg Asp Lys Ser Cys Phe Ala Asn Lys His Ala Val Ala
   1 5 10 15
   Gly Lys Leu Ile Gly Phe Arg Thr Pro Ala Tyr Ser Leu Lys Pro
   20 25 30
<210> 37
   <211> 28
   <212> PRT
   <213> Arabidopsis thaliana
<400> 37
   Met Cys Ala Ser Arg Phe Lys Gly Val Gly Gly Cys Ser Lys Thr Asp
   1 5 10 15
   Gln Phe Thr Cys Asn Cys Met Phe Ile Cys Lys Thr
   20 25
<210> 38
   <211> 19
   <212> PRT
   <213> Arabidopsis thaliana
<400> 38
   Met Val Thr Lys Glu Thr Gln Ala Glu Gly Glu Lys Val Cys Val Ile
   1 5 10 15
   Ile Ile Leu
<210> 39
   <211> 16
   <212> PRT
   <213> Arabidopsis thaliana
<400> 39
   Met Leu Cys Cys Trp Asp Phe Asp Arg Ser Leu Pro Leu Ile Leu Leu
   1 5 10 15
<210> 40
   <211> 59
   <212> PRT
   <213> Arabidopsis thaliana
<400> 40
   Met Gly Ser Leu Ala Glu Glu Gly Gly Asp Leu Leu Leu Pro Arg Gly
   1 5 10 15
   Gly Val Ser Gly Gly Ala Lys Thr Gly Glu Gly Leu Cys Ser Gly Asp
   20 25 30
   Lys Gly Val Val Ser Val Glu Thr Ser Ser Arg Thr Ser Gly Ile Ile
   35 40 45
   Pro Met Leu Lys His Ile Ile Glu Leu Arg Thr
   50 55
<210> 41
   <211> 40
   <212> PRT
   <213> Arabidopsis thaliana
<400> 41
   Met Val Arg Cys Tyr Arg Thr Ser Arg Thr Arg Cys Ser Ile Asp Arg
   1 5 10 15
   Trp Gly Arg Val Leu Leu Gln Leu Gly Asp His Ile Ala Arg Pro Arg
   20 25 30
   Ile Met Ile Gly Glu Val Lys Arg
   35 40
<210> 42
   <211> 23
   <212> PRT
   <213> Arabidopsis thaliana
<400> 42
   Met Trp Ser Pro Ser Trp Arg Ser Thr Leu Pro Gln Arg Ser Met Leu
   1 5 10 15
   Gln Arg Val Arg Leu Val Arg
   20
<210> 43
   <211> 161
   <212> PRT
   <213> Arabidopsis thaliana
<400> 43
   Met Pro Pro Gln Lys Glu Asn His Arg Thr Leu Asn Lys Met Lys Thr
   1 5 10 15
   Asn Leu Phe Leu Phe Leu Ile Phe Ser Leu Leu Leu Ser Leu Ser Ser
   20 25 30
   Ala Glu Gln Cys Gly Arg Gln Ala Gly Gly Ala Leu Cys Pro Asn Gly
   35 40 45
   Leu Cys Cys Ser Glu Phe Gly Trp Cys Gly Asn Thr Glu Pro Tyr Cys
   50 55 60
   Lys Gln Pro Gly Cys Gln Ser Gln Cys Thr Pro Gly Gly Thr Pro Pro
   65 70 75 80
   Gly Pro Thr Gly Asp Leu Ser Gly Ile Ile Ser Ser Ser Gln Phe Asp
   85 90 95
   Asp Met Leu Lys His Arg Asn Asp Ala Ala Cys Pro Ala Arg Gly Phe
   100 105 110
   Tyr Thr Tyr Asn Ala Phe Ile Thr Ala Ala Lys Ser Phe Pro Gly Phe
   115 120 125
   Gly Thr Thr Gly Asp Thr Ala Thr Arg Lys Lys Glu Val Ala Ala Phe
   130 135 140
   Phe Gly Gln Thr Ser His Glu Thr Thr Gly Thr Gln Asn Ser Leu Thr
   145 150 155 160
   Ile
<210> 44
   <211> 63
   <212> PRT
   <213> Arabidopsis thaliana
<400> 44
   Met Ile Cys Leu Ser Ile Gly Met Met Pro Leu Val Leu Leu Glu Val
   1 5 10 15
   Ser Thr Leu Thr Thr Pro Leu Ser Pro Leu Gln Ser Pro Ser Pro Val
   20 25 30
   Leu Ala Pro Pro Glu Thr Pro Pro Arg Gly Arg Arg Arg Ser Pro Pro
   35 40 45
   Ser Ser Ala Arg Leu Pro Met Lys Leu Gln Val Pro Lys Thr His
   50 55 60
<210> 45
   <211> 42
   <212> PRT
   <213> Arabidopsis thaliana
<400> 45
   Met Ser Thr Thr Lys Asn Phe Phe Arg Leu Tyr Gln Asn Gln His Pro
   1 5 10 15
   Asn Thr Ser Ser Ser Leu Leu Phe Tyr Leu Ala Leu Ile Asp Ala Lys
   20 25 30
   Gly Leu Asn Arg Arg Glu Ser Lys Thr Pro
   35 40
<210> 46
   <211> 12
   <212> PRT
   <213> Arabidopsis thaliana
<400> 46
   Met Leu Tyr Ala Gln Ala Leu Ile Leu Cys Phe Ile
   1 5 10
<210> 47
   <211> 61
   <212> PRT
   <213> Arabidopsis thaliana
<400> 47
   Met Ile Met Cys Ser Leu Phe Ala Ser Val Phe Asp Cys Phe Val Pro
   1               5                   10                  15
   Lys Ser Asp Ser Lys Ile Ser Ser Thr Asp Glu Ser Asp Leu Lys Val
               20                  25                  30
   Leu Ser Ser Lys Lys Pro Lys Ser Lys Ser Pro Arg Ala Pro Ile Met
           35                  40                  45
   Val Ser Tyr Phe Pro Ala Gly Ser Asn Leu Ser Arg Leu
       50                  55                  60
<210> 48
   <211> 17
   <212> PRT
   <213> Arabidopsis thaliana
<400> 48
   Met Lys Tyr Trp Asp Val Asp Phe Asp Ile Asn Glu Arg Ser Phe Trp
   1               5                   10                  15
   Leu
<210> 49
   <211> 10
   <212> PRT
   <213> Arabidopsis thaliana
<400> 49
   Met Leu Tyr Lys Asn Ser Asn Asp Ser Met
   1               5                   10
<210> 50
   <211> 59
   <212> PRT
   <213> Arabidopsis thaliana
<400> 50
   Met Asp Arg Val Lys Ser Asn Ile Cys Ser His Ile Pro Leu Ile Gly
   1               5                   10                  15
   Cys Val Met Asp Val Leu Phe Leu Leu Phe Thr Ser Asp Ser Ser Ser
               20                  25                  30
   Asp Ala Gly Lys Glu Ile Val Asp Thr Gly Asn Leu Glu Ala Gln Lys
   35 40 45
   Asn Phe Arg Gln Leu Gln Cys Ile Lys Leu Gly
   50 55
<210> 51
   <211> 21
   <212> PRT
   <213> Arabidopsis thaliana
<400> 51
   Met Thr His Pro Ile Arg Gly Ile Cys Glu Gln Ile Leu Asp Phe Thr
   1 5 10 15
   Arg Ser Ile Lys Leu
   20
<210> 52
   <211> 14
   <212> PRT
   <213> Arabidopsis thaliana
<400> 52
   Met Pro Gln Leu Cys Leu Asp Leu Met Lys Ser Leu Lys Leu
   1 5 10
<210> 53
   <211> 67
   <212> PRT
   <213> Arabidopsis thaliana
<400> 53
   Met Ile Thr Val Leu Thr Phe Thr Leu Asn Phe Gln Leu Ser Ser Leu
   1 5 10 15
   Lys Gly Arg Arg Asn Tyr His Gly Leu Leu Lys Gly Phe Met Leu Arg
   20 25 30
   Asn Thr Leu Ser Ser Phe Lys Pro Ile Leu Tyr Asp Pro Asn Asn Ile
   35 40 45
   Gly Gln Leu Phe Ala Tyr Phe Pro Leu Ser Val Leu Glu Lys Glu Asn
   50 55 60
   Glu Val Asp
   65
<210> 54
   <211> 18
   <212> PRT
   <213> Arabidopsis thaliana
<400> 54
   Met Ile Ile Pro Ser Ser Leu Lys Thr Arg Lys Leu Lys Val Glu Ser
   1               5                   10                  15
   Lys Ser
<210> 55
   <211> 11
   <212> PRT
   <213> Arabidopsis thaliana
<400> 55
   Met Ser Arg Arg Met Ile Leu Thr Gln Tyr Trp
   1               5                   10
<210> 56
   <211> 57
   <212> PRT
   <213> Arabidopsis thaliana
<400> 56
   Met Ser Leu Leu Leu Asn Leu Phe Met Gly Leu Phe Leu Cys Gly Ile
   1               5                   10                  15
   Leu Lys Glu Val His Lys Asn Glu Gly Glu Glu Glu Glu Leu Val Glu
               20                  25                  30
   Thr Glu Leu Val Glu Met Glu Leu Val Glu Thr Thr Ser Phe Tyr Leu
           35                  40                  45
   Ile Thr Ile Leu Lys Ile Leu Phe His
       50                  55
<210> 57
   <211> 10
   <212> PRT
   <213> Arabidopsis thaliana
<400> 57
   Met Lys Pro Tyr Met Ile Asn Asn Phe Ser
   1               5                   10
<210> 58
   <211> 36
   <212> PRT
   <213> Arabidopsis thaliana
<400> 58
   Met Ser Ser Pro Pro Ile Pro Ser Pro Leu Ile Pro Ser Pro Pro Ile
   1               5                   10                  15
   Pro Pro Pro Pro Pro Arg Phe Tyr Val Pro Pro Ser Lys Ser Arg Arg
               20                  25                  30
   Gly Lys Gly Pro
           35
<210> 59
   <211> 28
   <212> PRT
   <213> Arabidopsis thaliana
<400> 59
   Met Val Ala Asn Tyr Leu Met Lys Gln Tyr Leu Gln Tyr Cys Tyr Gln
   1               5                   10                  15
   Val Lys Arg Cys Arg Leu His Gln Phe His Leu His
               20                  25
<210> 60
   <211> 17
   <212> PRT
   <213> Arabidopsis thaliana
<400> 60
   Met Tyr Leu Leu Gln Asn Pro Ala Glu Glu Lys Ala His Lys Gln Ile
   1               5                   10                  15
   Glu
<210> 61
   <211> 33
   <212> PRT
   <213> Arabidopsis thaliana
<400> 61
   Met Gln Glu Asn Lys Ala Arg Val Thr Thr Glu Lys Arg Ser Val Cys
   1               5                   10                  15
   Arg Thr Pro Pro Ile Asn Thr Glu Asn Arg Phe Asp Cys Leu Met Ile
               20                  25                  30
   Cys
<210> 62
   <211> 18
   <212> PRT
   <213> Arabidopsis thaliana
<400> 62
   Met His Leu Arg Lys Arg Arg Arg Leu Leu Ser Gln Lys Arg Thr Lys
   1 5 10 15
   Val Val
<210> 63
   <211> 22
   <212> PRT
   <213> Arabidopsis thaliana
<400> 63
   Met Ala Arg His Ser Leu Leu Ser Pro Pro Tyr Asp Ser Pro Leu Ser
   1 5 10 15
   Leu Tyr Ile Phe Ser Phe
   20
<210> 64
   <211> 15
   <212> PRT
   <213> Arabidopsis thaliana
<400> 64
   Met Thr Val Leu Phe His Cys Thr Tyr Ser Leu Phe Asp Asp Lys
   1 5 10 15
<210> 65
   <211> 14
   <212> PRT
   <213> Arabidopsis thaliana
<400> 65
   Met Leu Ser Arg Ser Ser Asn Asn Gln Ile Cys Ser Pro Cys
   1 5 10
<210> 66
   <211> 11
   <212> PRT
   <213> Arabidopsis thaliana
<400> 66
   Met Lys Lys Ile Cys Trp Ala Ser Gln Leu Ser
   1 5 10
<210> 67
   <211> 36
   <212> PRT
   <213> Arabidopsis thaliana
<400> 67
   Met Asp Pro Ala Met Glu Val Asp Met Val Lys Gly Met Glu Ala Asp
   1               5                   10                  15
   Thr Val Val Glu Ile Ile Thr Val Thr His Pro Lys Ser Phe Leu Leu
               20                  25                  30
   Leu Ile Val Ser
           35
<210> 68
   <211> 18
   <212> PRT
   <213> Arabidopsis thaliana
<400> 68
   Met Ile Ile Met Cys Ser Met Phe Cys Arg Tyr Ile Tyr Ile His Ala
   1               5                   10                  15
   Cys Ile
<210> 69
   <211> 22
   <212> PRT
   <213> Arabidopsis thaliana
<400> 69
   Met Tyr Ile Tyr Val Thr Thr Lys His Arg Thr His Tyr Tyr His Lys
   1               5                   10                  15
   Phe Arg Ile Met Lys Gln
               20
<210> 70
   <211> 12
   <212> PRT
   <213> Arabidopsis thaliana
<400> 70
   Met Phe Cys Lys Phe Ala Ile Phe His Phe Tyr Ala
   1               5                   10
<210> 71
   <211> 11
   <212> PRT
   <213> Arabidopsis thaliana
<400> 71
   Met Glu Glu Thr Leu Tyr Thr Gln His Gln Lys
   1 5 10
<210> 72
   <211> 10
   <212> PRT
   <213> Arabidopsis thaliana
<400> 72
   Met Pro Glu Asn Gln His Lys Ser Gly Glu
   1 5 10
<210> 73
   <211> 36
   <212> PRT
   <213> Arabidopsis thaliana
<400> 73
   Met Glu Asp Cys Glu Leu Ala Glu His Asp Leu Val Ile Glu Thr Ala
   1 5 10 15
   Val Ser Ser Ala Gly Lys Leu Asn Pro Phe Leu Met Phe Ser Tyr Phe
   20 25 30
   Trp Cys Cys Val
   35
<210> 74
   <211> 19
   <212> PRT
   <213> Arabidopsis thaliana
<400> 74
   Met Arg Phe Leu Tyr Asn Thr Leu Pro Gly Phe Gln Ile Asp Phe Phe
   1 5 10 15
   Ser Ser Val
<210> 75
   <211> 33
   <212> PRT
   <213> Arabidopsis thaliana
<400> 75
   Met Cys Cys Gln Pro Leu Pro Leu Ala Ile Pro Leu Arg Thr Ala Val
   1               5                   10                  15
   Lys Pro Ser Arg Ile Lys Glu Arg Gln Ser Lys Gly Val Arg Ser Ala
               20                  25                  30
   Thr
<210> 76
   <211> 31
   <212> PRT
   <213> Arabidopsis thaliana
<400> 76
   Met Met Phe Leu Gln Val Asn Arg Ser Met Leu Lys Lys Ala Asp Ser
   1               5                   10                  15
   Thr Ile Gln Arg Gln Ser Leu Ala Asn Thr Glu Thr Leu Ala Thr
               20                  25                  30
<210> 77
   <211> 45
   <212> PRT
   <213> Arabidopsis thaliana
<400> 77
   Met Ile Ser Ile Leu Ser Phe Arg Thr Ser Ser Ser Tyr Tyr Arg Gln
   1               5                   10                  15
   Ser Phe His Lys Asp Trp Phe Leu Ala Ala His Val Tyr Gln Thr Ile
               20                  25                  30
   Trp Glu Leu Gly Ile Leu Lys Leu Leu Gly Ser Gln Tyr
           35                  40                  45
<210> 78
   <211> 31
   <212> PRT
   <213> Arabidopsis thaliana
<400> 78
   Met Gly Leu Leu Gly Glu Val Val Gly Asn Thr Leu Glu Leu Gly Thr
   1               5                   10                  15
   Ile Leu Phe Leu Ile Ile Ser Tyr Glu Leu Leu Leu Glu Phe Thr
               20                  25                  30
<210> 79
   <211> 12
   <212> PRT
   <213> Arabidopsis thaliana
<400> 79
   Met Ser Ser Ser Trp Asn Leu Leu Ser Phe Gln Ser
   1 5 10
<210> 80
   <211> 11
   <212> PRT
   <213> Arabidopsis thaliana
<400> 80
   Met Asn Glu Leu Gln Arg Leu Tyr Leu Lys Thr
   1 5 10
<210> 81
   <211> 30
   <212> PRT
   <213> Arabidopsis thaliana
<400> 81
   Met Ile Tyr Glu Ser Gln Asp Thr Thr Asp Lys Leu Ile Pro Glu His
   1 5 10 15
   Met Ser Asn Arg Leu Thr Met Thr Gly Ser Lys Asp Ile Lys
   20 25 30
<210> 82
   <211> 24
   <212> PRT
   <213> Arabidopsis thaliana
<400> 82
   Met Ile Tyr Glu Ser Gln Asp Thr Thr Asp Lys Leu Ile Ser Lys His
   1 5 10 15
   Met Ser Asn Arg Leu Lys Gln Thr
   20
<210> 83
   <211> 14
   <212> PRT
   <213> Arabidopsis thaliana
<400> 83
   Met Cys Leu Asp Ile Asn Leu Ser Val Val Ser Trp Asp Ser
   1 5 10
<210> 84
   <211> 10
   <212> PRT
   <213> Arabidopsis thaliana
<400> 84
   Met Ile Val Ser Lys Tyr Leu Gln Pro Ile
   1               5                   10
<210> 85
   <211> 26
   <212> PRT
   <213> Arabidopsis thaliana
<400> 85
   Met Ser Leu Asp Pro Val Ile Val Ser Leu Leu Leu Met Cys Ser Gly
   1               5                   10                  15
   Ile Ser Leu Ser Val Val Ser Trp Asp Ser
               20                  25
<210> 86
   <211> 81
   <212> PRT
   <213> Arabidopsis thaliana
<400> 86
   Met Arg Ile Met Trp Lys Phe Ile Ile Val Met Leu Cys Phe Ile Ala
   1               5                   10                  15
   Leu Val Gly Ser Arg Gly Thr Ser Ala Thr Ser Arg Leu Arg Met Lys
               20                  25                  30
   Lys Glu Asp Ile Gly Arg Arg Phe Ala Leu Gln Asn Lys Leu Gln Arg
           35                  40                  45
   Gly Pro Val Pro Pro Ser Gln Pro Ser Pro Cys His Asn Lys Leu Asn
       50                  55                  60
   Pro Leu Ser His Ser Gln Val Tyr Ser Ser His Thr Tyr Val Thr Cys
   65                  70                  75                  80
   Pro
<210> 87
   <211> 43
   <212> PRT
   <213> Arabidopsis thaliana
<400> 87
   Met Ile Lys Trp Leu Asn Asn Thr Lys Ser Cys Ile Ile Tyr Val Gln
   1               5                   10                  15
   Lys Val Leu Gln Phe Arg Leu Phe Arg Val Trp Arg Ser Arg Ile Val
               20                  25                  30
   Asp Thr Tyr Met Tyr Glu Lys Arg Asp Asn Lys
           35                  40
<210> 88
   <211> 43
   <212> PRT
   <213> Arabidopsis thaliana
<400> 88
   Met Ser Lys Arg Tyr Tyr Asn Phe Asp Ser Phe Glu Phe Gly Gly Val
   1               5                   10                  15
   Glu Ser Trp Ile His Ile Cys Met Arg Arg Glu Thr Ile Asn Lys Arg
               20                  25                  30
   Met Trp Leu Ile Lys Leu Gln Gly Gln Val Thr
           35                  40
<210> 89
   <211> 23
   <212> PRT
   <213> Arabidopsis thaliana
<400> 89
   Met Asn Phe His Met Ile Leu Met Ile Thr Arg Pro Cys Asn Ala Gly
   1               5                   10                  15
   Met Asn Gly Leu Val Val Asp
               20
<210> 90
   <211> 44
   <212> PRT
   <213> Arabidopsis thaliana
<400> 90
   Met Glu Lys Ala Val Lys Glu Glu Leu Asp Leu Val Gly Ala Tyr Ser
   1               5                   10                  15
   Ala Lys Gln Thr Phe Tyr Leu Tyr Leu Pro Ser Ser Phe Ser Ala Leu
               20                  25                  30
   Thr Leu Pro Met Phe Leu Leu Ile Pro Pro Lys Leu
           35                  40
<210> 91
   <211> 12
   <212> PRT
   <213> Arabidopsis thaliana
<400> 91
   Met Arg Asp Gln Phe Asn Leu Gln Ile Ser Ala Trp
   1 5 10
<210> 92
   <211> 35
   <212> PRT
   <213> Arabidopsis thaliana
<400> 92
   Met Asn Gln Ile Gln Leu Ile Tyr Phe Asn Phe Leu Gln Pro Gln Cys
   1 5 10 15
   Thr Ile Tyr Leu Ile Phe Leu Phe Thr Lys Val Phe Ile Tyr Asn Thr
   20 25 30
   Lys Asn Ile
   35
<210> 93
   <211> 28
   <212> PRT
   <213> Arabidopsis thaliana
<400> 93
   Met Gly His His Arg Ile Leu Arg Lys Thr His Thr Ser Tyr Ile Phe
   1 5 10 15
   Arg Val Ile Tyr Lys Asn Phe Gly Glu Gln Lys Asn
   20 25
<210> 94
   <211> 26
   <212> PRT
   <213> Arabidopsis thaliana
<400> 94
   Met Ile His His Leu Tyr Leu Ala Arg Asn Pro Arg Ile Ser Asp Thr
   1 5 10 15
   Thr Ser Leu Ile Thr Pro Ile Leu Lys Cys
   20 25
<210> 95
   <211> 12
   <212> PRT
   <213> Arabidopsis thaliana
<400> 95
   Met Gln Asn Gln Ile Glu Lys Arg Cys Gln Phe Tyr
   1 5 10
<210> 96
   <211> 28
   <212> PRT
   <213> Arabidopsis thaliana
<400> 96
   Met Asn Asn Glu Leu Gln Gln Leu Tyr Leu Lys Thr Lys Ser Lys Ser
   1 5 10 15
   His Lys Gln Asp Ile Ile Ser Phe Phe Leu Ile Gln
   20 25
<210> 97
   <211> 60
   <212> PRT
   <213> Arabidopsis thaliana
<400> 97
   Met Ser Pro Arg Thr Gln Val Thr Asn Thr Ala Thr Gly Thr Gln Ala
   1 5 10 15
   Thr Val Arg Ile Val Asp Gln Cys Ser Asn Gly Gly Leu Asp Leu Glu
   20 25 30
   Glu Gly Val Phe Arg Gln Leu Asp Thr Asn Ser Gln Gly Asn Ala Arg
   35 40 45
   Gly His Leu Ile Val Asn Tyr Glu Phe Val Asn Cys
   50 55 60
<210> 98
   <211> 19
   <212> PRT
   <213> Arabidopsis thaliana
<400> 98
   Met Leu Phe Ser Tyr Thr Asn Leu Leu Asn Val Cys Ile Cys Pro Leu
   1 5 10 15
   Glu Arg Arg
<210> 99
   <211> 27
   <212> PRT
   <213> Arabidopsis thaliana
<400> 99
   Met Cys Ala Tyr Val Pro Ser Asn Ala Gly Asp Glu Tyr Cys Asp Arg
   1 5 10 15
   Asn Ala Ser Asp Cys Glu Asn Arg Gly Ser Val
   20 25
<210> 100
   <211> 19
   <212> PRT
   <213> Arabidopsis thaliana
<400> 100
   Met Ser Cys Ser Asp Glu Asn Leu Leu Thr Leu Asp Gly Val Phe Asn
   1 5 10 15
   Cys Val Leu
<210> 101
   <211> 14
   <212> PRT
   <213> Arabidopsis thaliana
<400> 101
   Met Thr Ile Ser Tyr Ile Phe Phe Leu Leu Lys Thr Ala Ile
   1 5 10
<210> 102
   <211> 42
   <212> PRT
   <213> Arabidopsis thaliana
<400> 102
   Met Thr Ala Asp Leu Gly Lys Ser Ser Ser Phe Ser Ser Arg Arg Pro
   1 5 10 15
   Gly Ser Leu Asp Val Leu Phe Arg Arg Glu Ser Ala His Ile Gly Arg
   20 25 30
   Arg Leu Gln Leu Cys Ser Met Ile Arg Tyr
   35 40
<210> 103
   <211> 11
   <212> PRT
   <213> Arabidopsis thaliana
<400> 103
   Met Cys Asn Arg Ile Phe Pro Asp Ile Cys Pro
   1               5                   10
<210> 104
   <211> 36
   <212> PRT
   <213> Arabidopsis thaliana
<400> 104
   Met Met Met Ile Phe Gln Asp Pro Leu Ser Ser His His Tyr Ile Ala
   1               5                   10                  15
   Val Leu Arg Arg Lys Lys Ile Tyr Asp Ile Val Ile Val Glu Glu Ile
               20                  25                  30
   Leu Val Arg Ser
           35
<210> 105
   <211> 18
   <212> PRT
   <213> Arabidopsis thaliana
<400> 105
   Met Ile Leu Ser Leu Ser Arg Arg Tyr Leu Phe Asp Arg Glu Arg Glu
   1               5                   10                  15
   Lys Tyr
<210> 106
   <211> 43
   <212> PRT
   <213> Arabidopsis thaliana
<400> 106
   Met Lys Lys Leu Arg Gln Arg Val Ala Lys Lys Ile Lys Gln Asp Ile
   1               5                   10                  15
   Cys Gly Asp Asn Ser Thr Val Phe Phe Phe Leu Val Ile Leu Thr Thr
               20                  25                  30
   Phe Leu Ile Leu Asn Tyr Leu Ile Asn Ser Leu
           35                  40
<210> 107
   <211> 28
   <212> PRT
   <213> Arabidopsis thaliana
<400> 107
   Met Trp Ser Ile Ser Pro Arg Lys Lys Lys Gln Tyr Cys Cys His His
   1 5 10 15
   Lys Cys Leu Val Leu Phe Ser Leu Pro Leu Phe Ala
   20 25
<210> 108
   <211> 21
   <212> PRT
   <213> Arabidopsis thaliana
<400> 108
   Met Thr Trp Ser Asn Val Ile Gln Leu Ser Leu Glu Leu Lys Gln Pro
   1 5 10 15
   Ala Thr Ser Gly Thr
   20
<210> 109
   <211> 14
   <212> PRT
   <213> Arabidopsis thaliana
<400> 109
   Met Pro Leu Pro Val Gln Ala Leu Ser Leu Gln Val Trp Asp
   1 5 10
<210> 110
   <211> 12
   <212> PRT
   <213> Arabidopsis thaliana
<400> 110
   Met Trp Cys Lys His Ser Ser Leu Leu Phe Leu Arg
   1 5 10
<210> 111
   <211> 19
   <212> PRT
   <213> Arabidopsis thaliana
<400> 111
   Met Asp Gln Ser Gln Thr Trp Arg Asp Lys Ala Cys Thr Gly Lys Gly
   1 5 10 15
   Ile Val Asp
<210> 112
   <211> 45
   <212> PRT
   <213> Arabidopsis thaliana
<400> 112
   Met Arg Asn Ser Gln Val Pro Glu Val Ala Gly Cys Phe Asn Ser Ser
   1 5 10 15
   Glu Ser Trp Met Thr Phe Asp Gln Val Ile Cys Leu Asp Val Ala Asn
   20 25 30
   Gly Asn Ala Ser Ser Phe Phe His His Tyr Cys Ser Arg
   35 40 45
<210> 113
   <211> 17
   <212> PRT
   <213> Arabidopsis thaliana
<400> 113
   Met Ile Phe Thr Leu Glu Thr Gly Gly Arg Ser Val Tyr Thr Thr Ser
   1 5 10 15
   Gly
<210> 114
   <211> 41
   <212> PRT
   <213> Arabidopsis thaliana
<400> 114
   Met Gly Tyr Gly Arg Ser Leu Ile Trp Leu Ala Glu Asp Thr Phe Gly
   1 5 10 15
   Glu Ile Ser Arg Arg Cys Val Arg Glu Val Asn Asp Arg Tyr Lys Gln
   20 25 30
   Ser Phe Ala Cys Leu Ala Ser Asp Met
   35 40
<210> 115
   <211> 28
   <212> PRT
   <213> Arabidopsis thaliana
<400> 115
   Met Thr Asp Ile Asn Lys Val Ser Pro Val Trp Leu Leu Ile Cys Arg
   1 5 10 15
   Lys Leu Arg Thr Arg Lys Ile Val Ser Ser Phe Ser
   20 25
<210> 116
   <211> 41
   <212> PRT
   <213> Arabidopsis thaliana
<400> 116
   Met Lys Leu Pro Asn Ser Asp Glu Phe Gly Gln Asp Leu Arg Lys Arg
   1 5 10 15
   Arg Asn Asn Phe Ser Cys Ser Gln Leu Ser Thr Tyr Gln Lys Pro Asn
   20 25 30
   Arg Arg Asn Phe Val Tyr Ile Gly His
   35 40
<210> 117
   <211> 13
   <212> PRT
   <213> Arabidopsis thaliana
<400> 117
   Met Lys Phe Lys Tyr Met Asn Thr Lys Leu Lys Asn Glu
   1 5 10
<210> 118
   <211> 42
   <212> PRT
   <213> Arabidopsis thaliana
<400> 118
   Met Asn Ser Asp Lys Ile Tyr Glu Asn Glu Glu Thr Ile Phe Leu Val
   1 5 10 15
   Leu Asn Phe Leu His Ile Arg Ser Gln Thr Gly Glu Thr Leu Phe Ile
   20 25 30
   Ser Val Ile Asp Phe Ser Asp Ala Ser Pro
   35 40
<210> 119
   <211> 14
   <212> PRT
   <213> Arabidopsis thaliana
<400> 119
   Met Gln Gln Arg Val Asn Cys Gly His His Leu Asp Leu Leu
   1               5                   10
<210> 120
   <211> 29
   <212> PRT
   <213> Arabidopsis thaliana
<400> 120
   Met Val Asn His Leu Gln Gln Ile Gln Met Val Thr Ala Ile Asp Ser
   1               5                   10                  15
   Leu Leu His Arg Leu Tyr Gln Gly Arg Gln Arg Leu Asn
               20                  25
<210> 121
   <211> 10
   <212> PRT
   <213> Arabidopsis thaliana
<400> 121
   Met Lys Glu Arg Ser Lys Thr Gln Leu Asn
   1               5                   10
<210> 122
   <211> 15
   <212> PRT
   <213> Arabidopsis thaliana
<400> 122
   Met Gly Tyr Arg Asn Arg Ser Tyr Leu Val Lys Ser Leu Pro Phe
   1               5                   10                  15
<210> 123
   <211> 19
   <212> PRT
   <213> Arabidopsis thaliana
<400> 123
   Met Arg Ser Arg Arg Ile Lys Lys Thr Arg Arg Ile Lys Arg Thr Arg
   1               5                   10                  15
   Arg Ile Asn
<210> 124
   <211> 24
   <212> PRT
   <213> Arabidopsis thaliana
<400> 124
   Met Phe Phe Glu Gln Arg Leu Gly Phe His Leu Leu Ala Gly Leu Val
   1               5                   10                  15
   Trp Leu Val Asn Pro Ser Arg Phe
               20
<210> 125
   <211> 18
   <212> PRT
   <213> Arabidopsis thaliana
<400> 125
   Met Leu Ser Asn Asp Ala Asn Arg Glu Thr Gly Lys Ser Leu Tyr Leu
   1               5                   10                  15
   Lys Gly
<210> 126
   <211> 26
   <212> PRT
   <213> Arabidopsis thaliana
<400> 126
   Met Met Gln Thr Val Lys Leu Glu Ser Leu Tyr Ile Ser Arg Asp Lys
   1               5                   10                  15
   Thr Pro Glu Leu Ala Leu Leu Val Val Leu
               20                  25
<210> 127
   <211> 45
   <212> PRT
   <213> Arabidopsis thaliana
<400> 127
   Met Pro Ile Thr Ser Ser Leu Pro Ile Leu Gly Ser Glu Ala Gly Lys
   1               5                   10                  15
   Asp Gly Gln Glu Glu Asp Asn Thr Ala Ala Ser Gly Gly Ile Met Ala
               20                  25                  30
   Ala Gly Thr Pro Val Thr His Pro Lys Gly Lys Pro Val
           35                  40                  45
<210> 128
   <211> 17
   <212> PRT
   <213> Arabidopsis thaliana
<400> 128
   Met Ser Phe Leu Ala Glu Asn Val Glu Ser Arg Phe Leu Ala Lys Lys
   1 5 10 15
   Cys
<210> 129
   <211> 10
   <212> PRT
   <213> Arabidopsis thaliana
<400> 129
   Met Leu Asn Cys Phe Leu Ala Gly Lys Tyr
   1 5 10
<210> 130
   <211> 18
   <212> PRT
   <213> Arabidopsis thaliana
<400> 130
   Met Asn Val Val Phe Gly Gly Lys Met Leu Asn Cys Val Leu Ala Gly
   1 5 10 15
   Lys Tyr
<210> 131
   <211> 18
   <212> PRT
   <213> Arabidopsis thaliana
<400> 131
   Met Leu Ile Val Val Phe Gly Glu Lys Met Leu Asn Cys Val Leu Ala
   1 5 10 15
   Gly Lys
<210> 132
   <211> 10
   <212> PRT
   <213> Arabidopsis thaliana
<400> 132
   Met Phe Asn Val Val Phe Gly Gly Lys Cys
   1 5 10
<210> 133
   <211> 10
   <212> PRT
   <213> Arabidopsis thaliana
<400> 133
   Met Leu Asn Cys Val Leu Ala Arg Lys Tyr
   1 5 10
<210> 134
   <211> 10
   <212> PRT
   <213> Arabidopsis thaliana
<400> 134
   Met Leu Asn Cys Val Leu Ala Gly Lys Cys
   1 5 10
<210> 135
   <211> 23
   <212> PRT
   <213> Arabidopsis thaliana
<400> 135
   Met Ser Leu Ile Ile Gln Ile Cys Phe Ser Leu Ser Glu Arg His Leu
   1 5 10 15
   Val Val Ala Tyr Ile His Phe
   20
<210> 136
   <211> 35
   <212> PRT
   <213> Arabidopsis thaliana
<400> 136
   Met Ala Leu Asn Asn Tyr Arg Leu Gln Ile Pro Leu Lys His Leu Pro
   1 5 10 15
   Ile Pro Arg His Lys Leu Arg Asn Glu Leu Asn Asn Thr Asn Leu Leu
   20 25 30
   Leu Leu Glu
   35
<210> 137
   <211> 31
   <212> PRT
   <213> Arabidopsis thaliana
<400> 137
   Met Met Ile Ile His Leu Gln Val Thr Gln His Ser Glu Lys Asp Arg
   1 5 10 15
   Leu Arg Lys Asn His Thr Tyr Lys Leu Phe Cys Lys Asn Ile Phe
   20 25 30
<210> 138
   <211> 14
   <212> PRT
   <213> Arabidopsis thaliana
<400> 138
   Met Tyr Asp Phe Phe Ser Ala Tyr Leu Phe His Tyr Val Val
   1 5 10
<210> 139
   <211> 36
   <212> PRT
   <213> Arabidopsis thaliana
<400> 139
   Met Ile Cys Arg Leu Arg Arg Leu Gly Arg Glu Trp Ser Leu Val Lys
   1 5 10 15
   Ile Asp Trp Leu Lys Leu Arg Met Phe Leu Arg Ala Ile Ser Asn Tyr
   20 25 30
   Phe Cys Arg Cys
   35
<210> 140
   <211> 15
   <212> PRT
   <213> Arabidopsis thaliana
<400> 140
   Met Ile Phe Ser Gln Pro Ile Phe Phe Thr Met Leu Cys Asn Leu
   1 5 10 15
<210> 141
   <211> 20
   <212> PRT
   <213> Arabidopsis thaliana
<400> 141
   Met Val Ser Cys Ser Asn Val Arg Cys Gly Leu Ser Gly Cys Phe Gly
   1 5 10 15
   Arg Lys Arg Thr
   20
<210> 142
   <211> 27
   <212> PRT
   <213> Arabidopsis thaliana
<400> 142
   Met Ser Arg Leu Ser Leu Cys Val Phe Val Leu Leu Cys Ala Phe Ala
   1 5 10 15
   Ala Lys Ala Ala Ala Gln Ser Ala Pro Asn Val
   20 25
<210> 143
   <211> 19
   <212> PRT
   <213> Arabidopsis thaliana
<400> 143
   Met Cys Val Cys Val Ala Met Cys Val Cys Gly Gln Ser Ser Arg Ser
   1 5 10 15
   Ile Arg Thr
<210> 144
   <211> 26
   <212> PRT
   <213> Arabidopsis thaliana
<400> 144
   Met Met Ile Ala Ser Gln Ala Val Leu His Ser Gln Leu Gln Ala Thr
   1 5 10 15
   Trp Leu Gln Gln Ser Gln Leu Lys Gln Asn
   20 25
<210> 145
   <211> 32
   <212> PRT
   <213> Arabidopsis thaliana
<400> 145
   Met Gly Leu Asp Gly Val Thr Asn Val Asp Phe Asp Glu Leu Gly Val
   1               5                   10                  15
   Asp Val Ser Lys Leu Leu Leu Ser Leu Lys Trp Phe Trp Leu Leu Asp
               20                  25                  30
<210> 146
   <211> 15
   <212> PRT
   <213> Arabidopsis thaliana
<400> 146
   Met Trp Ile Leu Met Asn Trp Val Leu Thr Cys Arg Asn Cys Cys
   1               5                   10                  15
<210> 147
   <211> 12
   <212> PRT
   <213> Arabidopsis thaliana
<400> 147
   Met Asn Gly Ser Arg Trp Ser Asn Glu Cys Gly Phe
   1               5                   10
<210> 148
   <211> 18
   <212> PRT
   <213> Arabidopsis thaliana
<400> 148
   Met Val Leu Val Ala Arg Leu Arg Leu Arg Ala Gln Ala Arg Ser Cys
   1               5                   10                  15
   Trp Cys
<210> 149
   <211> 18
   <212> PRT
   <213> Arabidopsis thaliana
<400> 149
   Met His Gly Thr Asp Val Thr Phe Arg Lys Asp Ala Arg Ser Gly Cys
   1               5                   10                  15
   Asp Leu
<210> 150
   <211> 68
   <212> PRT
   <213> Arabidopsis thaliana
<400> 150
   Met Asp Ala Ser Arg Ser Ile Thr Ser Leu Pro Gly Thr Leu Pro Lys
   1               5                   10                  15
   Gly His Ile Arg Ser Leu His Pro Ser Glu Arg Ser His Leu Phe His
               20                  25                  30
   Ala Ser Phe Gly Asp Ala Thr Val Arg Leu Tyr Pro Leu Asp Leu Thr
           35                  40                  45
   Pro Phe Gly Arg Asp Thr Ser Ser Pro Val Ser Tyr Gln Ser Thr Ser
       50                  55                  60
   Ser Ser Val Ser
   65
<210> 151
   <211> 56
   <212> PRT
   <213> Arabidopsis thaliana
<400> 151
   Met Leu Pro Glu Ala Ser His Pro Phe Leu Ala Pro Ser Leu Lys Val
   1               5                   10                  15
   Thr Ser Ala Pro Cys Ile Leu Pro Lys Gly His Ile Cys Ser Met His
               20                  25                  30
   Leu Leu Ala Met Gln Pro Phe Val Cys Thr Arg Leu Thr Leu His Leu
           35                  40                  45
   Leu Ala Glu Thr Pro Leu His Leu
       50                  55
<210> 152
   <211> 10
   <212> PRT
   <213> Arabidopsis thaliana
<400> 152
   Met Ala Glu Leu Leu Gln Gln Val Arg Tyr
   1               5                   10
<210> 153
   <211> 12
   <212> PRT
   <213> Arabidopsis thaliana
<400> 153
   Met Thr Leu Thr Cys Ile Ser Gln Leu Leu Ser Ser
   1 5 10
<210> 154
   <211> 38
   <212> PRT
   <213> Arabidopsis thaliana
<400> 154
   Met Ser Val Leu Val Ala Asp Leu Leu Glu Gln Phe Arg His His Leu
   1 5 10 15
   Val Glu Leu Gln Ile Ser Met Leu Ser Tyr Lys Leu Leu Met Arg Phe
   20 25 30
   Ser Leu Arg Ile Leu Leu
   35
<210> 155
   <211> 25
   <212> PRT
   <213> Arabidopsis thaliana
<400> 155
   Met Ile Leu Lys Cys Trp Ser Ser Arg Phe Leu Arg Val Ser Pro Tyr
   1 5 10 15
   Gln Asn Ala His Ser Leu Ser Leu Gly
   20 25
<210> 156
   <211> 17
   <212> PRT
   <213> Arabidopsis thaliana
<400> 156
   Met Arg Ile Leu Ile Trp Thr His Ser Gln Glu Pro Gly Thr Pro Ala
   1 5 10 15
   Leu
<210> 157
   <211> 49
   <212> PRT
   <213> Arabidopsis thaliana
<400> 157
   Met Ala Phe Phe Thr Asn His Phe Thr Ile Val Leu Leu Tyr Gln Phe
   1               5                   10                  15
   Ile Phe Leu Ile Arg Thr Ile Asn Phe Val Ile Ile Met Tyr Glu Pro
               20                  25                  30
   Lys Asn Pro Tyr Ser Val Tyr Met Lys Val Ser Ile Trp Gln Leu Leu
           35                  40                  45
   Ile
<210> 158
   <211> 11
   <212> PRT
   <213> Arabidopsis thaliana
<400> 158
   Met Ser Thr Lys Val Asn Gly Gly Gly Asp Glu
   1               5                   10
<210> 159
   <211> 29
   <212> PRT
   <213> Arabidopsis thaliana
<400> 159
   Met Asp Met Met Val Val Asn Thr Glu Ala Val Val Phe Thr Asp Leu
   1               5                   10                  15
   Val Ile Leu Asn Thr Glu Glu Ala Val Val Leu Arg Ile
               20                  25
<210> 160
   <211> 11
   <212> PRT
   <213> Arabidopsis thaliana
<400> 160
   Met Asn Arg Glu Glu Ala Val Val Leu Trp Ile
   1               5                   10
<210> 161
   <211> 25
   <212> PRT
   <213> Arabidopsis thaliana
<400> 161
   Met Gly Ile Arg Tyr Leu Asn Gly Pro Lys Arg Thr Arg Pro Thr Ser
   1 5 10 15
   Cys Lys Pro Asn Phe Pro Ser Asp Lys
   20 25
<210> 162
   <211> 62
   <212> PRT
   <213> Arabidopsis thaliana
<400> 162
   Met Gly Gln Lys Glu Pro Gly Pro Pro Ala Val Asn Leu Ile Ser Leu
   1 5 10 15
   Gln Thr Asn Glu Gly Arg Asp Gly Gly Gly Cys Cys Thr Glu Met Thr
   20 25 30
   Arg Gly Lys Arg Pro Ser Pro Val Lys Glu Arg Ser Thr Phe Ala Gly
   35 40 45
   Phe Gly Arg Glu Val Arg Gln Arg Arg Arg Thr Arg Gly Ser
   50 55 60
<210> 163
   <211> 12
   <212> PRT
   <213> Arabidopsis thaliana
<400> 163
   Met Lys Val Val Met Val Ala Ala Val Val Arg Arg
   1 5 10
<210> 164
   <211> 11
   <212> PRT
   <213> Arabidopsis thaliana
<400> 164
   Met Val Phe Leu Arg Ala Ile Ser Asn Tyr Leu
   1 5 10
<210> 165
   <211> 11
   <212> PRT
   <213> Arabidopsis thaliana
<400> 165
   Met Ile Cys Arg Pro Arg Arg Gln Gly Arg Asp
   1 5 10
<210> 166
   <211> 14
   <212> PRT
   <213> Arabidopsis thaliana
<400> 166
   Met Asn Met Leu Leu Lys Ser Gln Arg Tyr Met His Tyr Pro
   1 5 10
<210> 167
   <211> 32
   <212> PRT
   <213> Arabidopsis thaliana
<400> 167
   Met Ile Leu Cys Glu Tyr Ile Tyr Glu His Ala Leu Lys Val Thr Lys
   1 5 10 15
   Ile Tyr Ala Leu Pro Val Asp Ala Pro Val Thr Val Gly Phe Val Phe
   20 25 30
<210> 168
   <211> 10
   <212> PRT
   <213> Arabidopsis thaliana
<400> 168
   Met Phe Leu Ser Val Leu Glu Phe Gly Ser
   1 5 10
<210> 169
   <211> 20
   <212> PRT
   <213> Arabidopsis thaliana
<400> 169
   Met Tyr Leu Trp Gly Met Gln Glu Ala Met Met Asn Asp Ile Val Pro
   1 5 10 15
   Ser Met Met Gln
   20
<210> 170
   <211> 12
   <212> PRT
   <213> Arabidopsis thaliana
<400> 170
   Met Glu Arg Cys Thr Cys Gly Ala Cys Lys Lys Arg
   1 5 10
<210> 171
   <211> 22
   <212> PRT
   <213> MCSHWKREWRDVLVGHARSDDE
<400> 171
   Met Cys Ser His Trp Lys Arg Glu Trp Arg Asp Val Leu Val Gly His
   1 5 10 15
   Ala Arg Ser Asp Asp Glu
   20
<210> 172
   <211> 28
   <212> PRT
   <213> Arabidopsis thaliana
<400> 172
   Met Asn His Leu Asn Arg Pro Lys Val Leu Thr Gln Gly Pro Leu Ser
   1 5 10 15
   Lys Ile Phe Asn Ser Ser Asn Arg Tyr Ser Leu Leu
   20 25
<210> 173
   <211> 16
   <212> PRT
   <213> Arabidopsis thaliana
<400> 173
   Met Ser Gly Tyr Ser Tyr Asp Cys Thr Gly Leu Phe Pro Val Leu Leu
   1 5 10 15
<210> 174
   <211> 33
   <212> PRT
   <213> Arabidopsis thaliana
<400> 174
   Met Ile Ala Arg Val Cys Ser Gln Ser Tyr Cys Glu Glu Glu Leu Pro
   1 5 10 15
   Ile Asn Ile Gln Arg His Val Cys Trp Lys Val Phe Ala Val Gln Ala
   20 25 30
   Ala
<210> 175
   <211> 17
   <212> PRT
   <213> Arabidopsis thaliana
<400> 175
   Met Ser Thr Ser Ser Thr Trp Asn His Met Gln His Ala Ile Lys His
   1 5 10 15
   Asn
<210> 176
   <211> 15
   <212> PRT
   <213> Arabidopsis thaliana
<400> 176
   Met Arg Lys Tyr Asn Asn Ile Glu Ser Ile Thr Ser Ser Asn Gln
   1 5 10 15
<210> 177
   <211> 10
   <212> PRT
   <213> Arabidopsis thaliana
<400> 177
   Met Glu Pro His Ala Thr Arg Asn Gln Thr
   1 5 10
<210> 178
   <211> 25
   <212> PRT
   <213> Arabidopsis thaliana
<400> 178
   Met Phe Asp Cys Val Leu His Val Val Pro Cys Thr Arg Arg Thr His
   1 5 10 15
   Gly Asn Gln Leu Asp Cys Leu Phe Tyr
   20 25
<210> 179
   <211> 48
   <212> PRT
   <213> Arabidopsis thaliana
<400> 179
   Met Trp Phe His Val Leu Asp Val Leu Met Val Thr Ser Leu Ile Val
   1 5 10 15
   Tyr Phe Ile Asp Cys Leu Met Leu Cys Phe Gln Tyr Cys Tyr Thr Ser
   20 25 30
   Ala Cys Cys Leu Leu Phe Trp Val Leu Gly Trp Glu Ile Ile Val Cys
   35 40 45
<210> 180
   <211> 17
   <212> PRT
   <213> Arabidopsis thaliana
<400> 180
   Met Gln Gln Arg Leu Met Met Asn Leu Leu Asp Gln Trp Glu Leu Val
   1 5 10 15
   Ala
<210> 181
   <211> 64
   <212> PRT
   <213> Arabidopsis thaliana
<400> 181
   Met His Lys Leu Thr Ser Leu His Asn Pro Arg Arg Asn Arg Ser Trp
   1 5 10 15
   Arg Arg Arg Leu Trp Arg Arg Gly Leu Arg Ile Met Asp Leu Arg Val
   20 25 30
   Ser Asn Ser Trp Gly Asn Gly Phe Trp Arg Arg Asp Ile Ser Leu Arg
   35 40 45
   Arg His Phe Arg Arg Ser Arg Pro Asn Lys Arg Arg Cys Asn Arg Gly
   50 55 60
<210> 182
   <211> 22
   <212> PRT
   <213> Arabidopsis thaliana
<400> 182
   Met Leu Ser Phe Gly Phe Arg Gln Asp Leu Phe Asp Thr Thr Arg Leu
   1 5 10 15
   Ser Lys Asn Gly Lys Lys
   20
<210> 183
   <211> 40
   <212> PRT
   <213> Arabidopsis thaliana
<400> 183
   Met Pro Glu Arg Leu Met Gly Thr Asp Cys Lys Phe Val Gly Asn Met
   1 5 10 15
   Ser Thr Leu Val Gln Ile Gln Leu Gly Pro Ile Ile Ser Cys Leu His
   20 25 30
   Asn His Phe Phe Phe Leu His Lys
   35 40
<210> 184
   <211> 10
   <212> PRT
   <213> Arabidopsis thaliana
<400> 184
   Met Glu Lys Lys Val Thr Lys His Lys Lys
   1 5 10
<210> 185
   <211> 20
   <212> PRT
   <213> Arabidopsis thaliana
<400> 185
   Met Arg Lys Ala Leu Asp Ile Thr Arg Lys Pro Leu Ser Trp Ser Thr
   1 5 10 15
   Gly His Thr Lys
   20
<210> 186
   <211> 16
   <212> PRT
   <213> Arabidopsis thaliana
<400> 186
   Met Glu Lys Ala Glu His Leu Ser Ser Ser Ala His Glu Lys Ser Ser
   1               5                   10                  15
<210> 187
   <211> 16
   <212> PRT
   <213> Arabidopsis thaliana
<400> 187
   Met Ile Gln Lys Ser Gln Thr Arg Leu Val Ile Lys Leu Tyr Phe Val
   1               5                   10                  15
<210> 188
   <211> 10
   <212> PRT
   <213> Arabidopsis thaliana
<400> 188
   Met Asn Pro Thr Ile Asp Pro Thr Thr Ile
   1               5                   10
<210> 189
   <211> 24
   <212> PRT
   <213> Arabidopsis thaliana
<400> 189
   Met Asp Phe Gly Ser Tyr Gly Phe Asn Leu Pro Val Leu Val Met Ser
   1               5                   10                  15
   Arg Ser Tyr Gly Gly Gly Gly Gly
               20
<210> 190
   <211> 12
   <212> PRT
   <213> Arabidopsis thaliana
<400> 190
   Met His Ser Gln Tyr Tyr Ser Pro Asn His Gly Tyr
   1               5                   10
<210> 191
   <211> 15
   <212> PRT
   <213> Arabidopsis thaliana
<400> 191
   Met Ala Thr Asn Lys Ile Arg Leu Gly Thr Lys Asn Gln Thr Glu
   1               5                   10                  15
<210> 192
   <211> 53
   <212> PRT
   <213> Arabidopsis thaliana
<400> 192
   Met Lys Ala Phe His Tyr Tyr Gln Pro Lys Glu Ile Arg Pro Leu Leu
   1               5                   10                  15
   Asp Ile Arg Phe Met Pro Glu Arg Tyr Glu Val Gly Leu Ile Trp Lys
               20                  25                  30
   His Trp Ala Ile Tyr Phe Ile Pro Thr Ile Leu Ser Gly Phe Trp Phe
           35                  40                  45
   Leu Val Leu Phe Tyr
       50
<210> 193
   <211> 13
   <212> PRT
   <213> Arabidopsis thaliana
<400> 193
   Met Tyr Ser Asp Glu Met Leu Leu Ile Leu Gly Gly Leu
   1               5                   10
<210> 194
   <211> 20
   <212> PRT
   <213> Arabidopsis thaliana
<400> 194
   Met Ala Asp Leu Ser Pro Asn Leu Leu Lys Gln Pro Lys Gly Leu Ala
   1               5                   10                  15
   Leu Ser Arg Phe
               20
<210> 195
   <211> 15
   <212> PRT
   <213> Arabidopsis thaliana
<400> 195
   Met Val Leu Ala Ser Ser Leu Leu Gly Ala Arg Pro Phe Val Gly
   1               5                   10                  15
<210> 196
   <211> 17
   <212> PRT
   <213> Arabidopsis thaliana
<400> 196
   Met Ser Arg Asn Leu Thr His Arg Pro Lys Arg Arg Glu Asn Thr Arg
   1               5                   10                  15
   Thr
<210> 197
   <211> 23
   <212> PRT
   <213> Arabidopsis thaliana
<400> 197
   Met Asp Glu Leu Gln Gly Gly Arg Arg Gln Ala Pro Cys Phe Gln Arg
   1               5                   10                  15
   Ser Gly Gly Pro Asn Leu Ile
               20
<210> 198
   <211> 17
   <212> PRT
   <213> Arabidopsis thaliana
<400> 198
   Met Leu Ser Glu Lys Trp Arg Ser Glu Ser Tyr Leu Asn Cys Glu Asn
   1               5                   10                  15
   Asn
<210> 199
   <211> 79
   <212> PRT
   <213> Arabidopsis thaliana
<400> 199
   Met Arg Asn Gln Ser His Val Lys Thr Arg His Phe Ala Glu Arg Phe
   1               5                   10                  15
   Phe Asn Asp Ser Lys Ile Phe Phe Ser Phe Pro Asp Lys Ile Ser Cys
               20                  25                  30
   Phe Tyr Glu Tyr Pro Phe Leu Asp Ser Phe Arg Lys Leu Leu Phe Phe
           35                  40                  45
   Val Asp Leu Glu Ile Cys Ile Asp Asp Leu Lys Ser Lys Val Glu Ser
       50                  55                  60
   Leu Ser Leu Leu Leu Ile Leu Phe Gln Phe Val Leu Gln Leu Ile
   65                  70                  75
<210> 200
   <211> 20
   <212> PRT
   <213> Arabidopsis thaliana
<400> 200
   Met Gly Thr Arg Lys Asn Thr Arg Phe Tyr Gln Gly Arg Arg Arg Lys
   1               5                   10                  15
   Phe Trp Ser Arg
               20
<210> 201
   <211> 15
   <212> PRT
   <213> Arabidopsis thaliana
<400> 201
   Met Glu Tyr Lys Cys Phe Asn Ser Asp Phe Thr Ala Val Glu Lys
   1               5                   10                  15
<210> 202
   <211> 28
   <212> PRT
   <213> Arabidopsis thaliana
<400> 202
   Met Leu Met Val Lys Met Asp Gln Lys Asp Pro Asp Thr Ile Ser Met
   1               5                   10                  15
   Ser Leu Phe Ser Cys Phe Leu Thr Phe Pro Leu Gln
               20                  25
<210> 203
   <211> 28
   <212> PRT
   <213> Arabidopsis thaliana
<400> 203
   Met Ala Tyr Asp Val Arg Gly Asn Asn Val Thr Thr His Val Met Gly
   1               5                   10                  15
   Pro Phe Ser Gly Pro Ser Glu Val Trp Leu Gly Pro
               20                  25
<210> 204
   <211> 23
   <212> PRT
   <213> Arabidopsis thaliana
<400> 204
   Met Leu Trp Ala His Leu Val Ala Gln Val Lys Ser Gly Leu Ala Leu
   1               5                   10                  15
   Lys Ser Met Glu Gly Leu Met
               20
<210> 205
   <211> 36
   <212> PRT
   <213> Arabidopsis thaliana
<400> 205
   Met Pro Pro Pro Pro Lys Arg Gln Ala Val Gly Pro Arg Val Tyr Ala
   1               5                   10                  15
   Ile Ala Gly Glu Glu Asp Val Asp Glu Asp Gly Ala Asp Pro Ile Val
               20                  25                  30
   Gly Lys Ser Ser
           35
<210> 206
   <211> 20
   <212> PRT
   <213> Arabidopsis thaliana
<400> 206
   Met Leu Met Arg Met Ala Pro Thr Arg Leu Leu Val Ser Leu Leu Lys
   1               5                   10                  15
   Asn Leu Phe Lys
               20
<210> 207
   <211> 14
   <212> PRT
   <213> Arabidopsis thaliana
<400> 207
   Met Ser Ile Ala Cys Ala Cys Phe Trp Cys Ser Cys Leu Ser
   1 5 10
<210> 208
   <211> 21
   <212> PRT
   <213> Arabidopsis thaliana
<400> 208
   Met Ala Tyr Thr Arg Gly Pro Thr Ala Cys Arg Phe Gly Gly Gly Gly
   1 5 10 15
   Ile Ile Pro Leu Phe
   20
<210> 209
   <211> 22
   <212> PRT
   <213> Arabidopsis thaliana
<400> 209
   Met Met Pro Arg Arg Pro Thr Arg Arg Leu Tyr Ser Ser Leu Lys Thr
   1 5 10 15
   Pro Leu Arg His Asp Ala
   20
<210> 210
   <211> 59
   <212> PRT
   <213> Arabidopsis thaliana
<400> 210
   Met Arg Arg Leu Gly Ser Leu Asp Leu Leu Ala Thr Gly Phe Phe Ser
   1 5 10 15
   Asp Leu Glu Ile Arg Trp Cys Ile Ala Leu Val Leu Phe Phe Leu Leu
   20 25 30
   Phe Val Lys Pro Pro Arg Phe Phe Ile Val Phe Ala Arg Ser Trp Ser
   35 40 45
   Leu Trp Cys Leu Leu Leu Leu Leu Phe Cys Thr
   50 55
<210> 211
   <211> 20
   <212> PRT
   <213> Arabidopsis thaliana
<400> 211
   Met Phe Leu Leu Leu Ser Cys Ser Ile Val Leu Phe Gln Ile Ser Ile
   1 5 10 15
   Ala His Arg Leu
   20
<210> 212
   <211> 10
   <212> PRT
   <213> Arabidopsis thaliana
<400> 212
   Met Asn Val Arg Ile His Pro Ile Thr Asn
   1 5 10
<210> 213
   <211> 16
   <212> PRT
   <213> Arabidopsis thaliana
<400> 213
   Met Ser Gly Tyr Ile Arg Ser Gln Thr Asn Thr Arg Ala His Glu Tyr
   1 5 10 15
<210> 214
   <211> 13
   <212> PRT
   <213> Arabidopsis thaliana
<400> 214
   Met Phe Phe Leu Gln Leu Gln Leu His Arg Gly Leu Val
   1 5 10
<210> 215
   <211> 26
   <212> PRT
   <213> Arabidopsis thaliana
<400> 215
   Met Lys Arg Thr Leu Lys Ala Gln Arg Met Ile Leu Lys Leu Leu Pro
   1               5                   10                  15
   Val Ser Lys Met His Cys Leu Ser Asn Leu
               20                  25
<210> 216
   <211> 23
   <212> PRT
   <213> Arabidopsis thaliana
<400> 216
   Met Ser Leu Val Tyr Trp Val Ala Leu Phe His Val His Ala Val Asn
   1               5                   10                  15
   Asn Phe His Leu Pro Gln Pro
               20
<210> 217
   <211> 64
   <212> PRT
   <213> Arabidopsis thaliana
<400> 217
   Met His Met Glu Glu Gly His Pro Ile Asp Gln Ala His Gln Ile Tyr
   1               5                   10                  15
   Gln Ala Arg Gln Asn Asp Val Gln Asn Pro Ser Ala Leu Ala Thr Thr
               20                  25                  30
   Ile Tyr Asn Arg His Phe Phe Gly Phe Ser Ile Val Gln Leu Lys Leu
           35                  40                  45
   Val Pro Asn Cys Asn Met Leu Ser Ile Gly Ser Met Val Phe Phe Leu
       50                  55                  60
<210> 218
   <211> 30
   <212> PRT
   <213> Arabidopsis thaliana
<400> 218
   Met Phe Phe Thr Leu Thr Ile Glu Ser Ala Val Cys Phe Ile Asn Arg
   1               5                   10                  15
   Arg Arg Gly Leu Gly Ser Lys Gly Asp His Arg Asp Gln Gly
               20                  25                  30
<210> 219
   <211> 14
   <212> PRT
   <213> Arabidopsis thaliana
<400> 219
   Met Pro Cys Cys Leu Ala Pro Ser Pro Asp Trp Thr Arg His
   1 5 10
<210> 220
   <211> 26
   <212> PRT
   <213> Arabidopsis thaliana
<400> 220
   Met Ser Gly Pro Val Arg Arg Trp Ser Glu Ala Thr Arg His Val Arg
   1 5 10 15
   Arg Asp Arg Ser Gly Asn Ala Leu Phe Met
   20 25
<210> 221
   <211> 22
   <212> PRT
   <213> Arabidopsis thaliana
<400> 221
   Met Glu Ala Ser Gly Thr Lys Val Pro Ser Glu Ser Arg Asn Ile Ala
   1 5 10 15
   Leu Leu Leu Asp Arg Leu
   20
<210> 222
   <211> 24
   <212> PRT
   <213> Arabidopsis thaliana
<400> 222
   Met Ser Asn Pro Glu Leu Ile Ser His Thr Gln Gln Lys Glu Leu Arg
   1 5 10 15
   Trp Gln Arg Leu His Leu Val Met
   20
<210> 223
   <211> 24
   <212> PRT
   <213> Arabidopsis thaliana
<400> 223
   Met Ile Arg Cys Phe Cys Val Thr Thr Lys Val Leu Ser Phe Phe Arg
   1 5 10 15
   Phe Lys Cys Cys Ile Thr Leu Val
   20
<210> 224
   <211> 12
   <212> PRT
   <213> Arabidopsis thaliana
<400> 224
   Met Phe Leu Cys Asp Tyr Gln Ser Phe Glu Leu Phe
   1 5 10
<210> 225
   <211> 14
   <212> PRT
   <213> Arabidopsis thaliana
<400> 225
   Met Leu Tyr His Phe Gly Val Gly Ser Trp Trp Phe Ile Arg
   1 5 10
<210> 226
   <211> 13
   <212> PRT
   <213> Arabidopsis thaliana
<400> 226
   Met Thr Arg Cys Ser Leu Cys His Arg Ser Ser Phe Cys
   1 5 10
<210> 227
   <211> 10
   <212> PRT
   <213> Arabidopsis thaliana
<400> 227
   Met Asn Pro Glu Ser Ser Ser Ala Glu Ser
   1 5 10
<210> 228
   <211> 13
   <212> PRT
   <213> Arabidopsis thaliana
<400> 228
   Met Arg Met Arg Arg Gly Thr Arg Arg Arg Ala Ser Arg
   1 5 10
<210> 229
   <211> 23
   <212> PRT
   <213> Arabidopsis thaliana
<400> 229
   Met Ile Ser Asp Pro Ser Phe Ala Glu Ala Leu His Lys Arg Arg Leu
   1 5 10 15
   Ser Ser Ala Lys Arg Arg Trp
   20
<210> 230
   <211> 20
   <212> PRT
   <213> Arabidopsis thaliana
<400> 230
   Met Ile Val Ser Ser Cys Ala Lys Pro Gln Arg Met Arg Asp Gln Arg
   1 5 10 15
   Ser Cys Val Ala
   20
<210> 231
   <211> 21
   <212> PRT
   <213> Arabidopsis thaliana
<400> 231
   Met Gly Lys Pro Arg Ile Lys Ser Ser Thr Ile Pro Ser His Leu Leu
   1 5 10 15
   Phe Leu Gly Leu Arg
   20
<210> 232
   <211> 42
   <212> PRT
   <213> Arabidopsis thaliana
<400> 232
   Met Leu Trp Pro Ile Pro Ser Arg Arg His Pro Arg Gln Thr Ser Lys
   1 5 10 15
   Asp Pro Lys Glu Ala Lys Ala Ile Ala Lys Gln Thr Val Met Ser Gln
   20 25 30
   Lys Leu Leu Gly Glu Gly Ser Ser Gln Phe
   35 40
<210> 233
   <211> 14
   <212> PRT
   <213> Arabidopsis thaliana
<400> 233
   Met Pro Ser Cys Leu Asp Arg Lys Pro Asn Leu Leu Lys Leu
   1 5 10
<210> 234
   <211> 16
   <212> PRT
   <213> Arabidopsis thaliana
<400> 234
   Met Tyr Tyr Pro Asn Ile Lys Pro Glu Asn Asn Leu Asn Phe Val Phe
   1 5 10 15
<210> 235
   <211> 23
   <212> PRT
   <213> Arabidopsis thaliana
<400> 235
   Met Asn His Asp Leu Tyr Leu Leu Lys Tyr Ser Val Leu Cys Glu Val
   1 5 10 15
   Asn Ile Gly Pro Val Leu Gly
   20
<210> 236
   <211> 12
   <212> PRT
   <213> Arabidopsis thaliana
<400> 236
   Met Ser Ser Val Leu Ile Leu Ile Ala Phe Gln Asp
   1 5 10
<210> 237
   <211> 16
   <212> PRT
   <213> Arabidopsis thaliana
<400> 237
   Met Tyr Thr Leu Val Leu Leu Asn Val Lys Cys Arg Arg Cys Leu Tyr
   1 5 10 15
<210> 238
   <211> 33
   <212> PRT
   <213> Arabidopsis thaliana
<400> 238
   Met Leu Asn Val Val Gly Ala Tyr Ile Asn Cys Phe Ser Arg Leu Glu
   1 5 10 15
   Asn Lys Val Lys Ile Val Phe Arg Phe Asn Val Trp Val Val His Ile
   20 25 30
   Tyr
<210> 239
   <211> 19
   <212> PRT
   <213> Arabidopsis thaliana
<400> 239
   Met Tyr Thr Ile Met Arg Phe Ser Tyr Arg Ile Ser Ser Thr Ser Ser
   1 5 10 15
   Arg Phe Arg
<210> 240
   <211> 18
   <212> PRT
   <213> Arabidopsis thaliana
<400> 240
   Met Ile Leu His Leu Lys Val His Leu Asn Leu Glu Leu Val Leu Glu
   1 5 10 15
   Ile Arg
<210> 241
   <211> 21
   <212> PRT
   <213> Arabidopsis thaliana
<400> 241
   Met Phe Arg Ala Asp Glu Leu Leu Glu Lys Asp Glu Val Thr Ser Phe
   1 5 10 15
   Val Arg Arg Ala Ser
   20
<210> 242
   <211> 16
   <212> PRT
   <213> Arabidopsis thaliana
<400> 242
   Met Thr Lys Leu Cys Phe Glu Leu Thr Ser Phe Leu Arg Arg Thr Arg
   1 5 10 15
<210> 243
   <211> 10
   <212> PRT
   <213> Arabidopsis thaliana
<400> 243
   Met Tyr Arg Thr Leu Ser Thr Arg Ser Ser
   1 5 10
<210> 244
   <211> 11
   <212> PRT
   <213> Arabidopsis thaliana
<400> 244
   Met Ile Glu Ala Arg Thr Leu Ser Gly Gln Ala
   1 5 10
<210> 245
   <211> 12
   <212> PRT
   <213> Arabidopsis thaliana
<400> 245
   Met Met Thr Trp Ser Arg Glu Tyr Asp Thr Ser Val
   1 5 10
<210> 246
   <211> 54
   <212> PRT
   <213> Arabidopsis thaliana
<400> 246
   Met Leu Pro Pro Ser Tyr Phe Pro Leu Thr Arg Gly Val Tyr Leu Pro
   1               5                   10                  15
   Arg Phe Leu Leu Asp His Leu Asn Thr Ser Val Ile Val Thr His Gly
               20                  25                  30
   Arg Arg Cys Glu Thr His Leu Ile Leu Glu Glu Gly Gln Val Cys Met
           35                  40                  45
   Pro Pro Pro Leu Leu Val
       50
<210> 247
   <211> 27
   <212> PRT
   <213> Arabidopsis thaliana
<400> 247
   Met Ile Ser Pro Leu Ser Glu Leu Ile Ala Ser Ser Phe Ser Leu Val
   1               5                   10                  15
   Arg Asp Ser Thr Phe Phe Ser Arg Thr Lys Ser
               20                  25
<210> 248
   <211> 25
   <212> PRT
   <213> Arabidopsis thaliana
<400> 248
   Met Ile Arg Thr Gly His Ala Ser Thr Val Ile Phe Ser Phe Asn Pro
   1               5                   10                  15
   Gly Gly Ile Ser Ser Thr Leu Ser Ser
               20                  25
<210> 249
   <211> 158
   <212> PRT
   <213> Arabidopsis thaliana
<400> 249
   Met Thr Tyr Lys Gly Ala Gly Arg Phe Thr Ser Arg Lys Lys Leu Ser
   1               5                   10                  15
   Pro Arg Tyr Val Gly Pro Tyr Lys Val Ile Lys Arg Val Gly Ala Val
               20                  25                  30
   Ala Tyr Lys Leu Asp Leu Pro Pro Lys Leu Asn Ala Phe His Asn Val
           35                  40                  45
   Phe His Val Ser Gln Leu Arg Lys Cys Leu Ser Asp Gln Glu Glu Ser
       50                  55                  60
   Val Glu Asp Ile Pro Pro Gly Leu Lys Glu Asn Met Thr Val Glu Ala
   65                  70                  75                  80
   Trp Pro Val Arg Ile Met Asp Arg Met Thr Lys Gly Thr Arg Glu Lys
                   85                  90                  95
   Ser Arg Asp Leu Leu Lys Val Leu Trp Asn Cys Gly Gly Arg Glu Glu
               100                 105                 110
   Tyr Thr Trp Glu Thr Glu Asn Lys Met Lys Ala Asn Phe Pro Glu Trp
           115                 120                 125
   Phe Lys Glu Met Gly Lys Asp Gln Leu Asp Ala Asp Ser Arg Thr Asn
       130                 135                 140
   Pro Ile Gln Gly Gly Glu Thr Cys Asn Ala Arg Asp Pro Gln
   145                 150                 155
<210> 250
   <211> 15
   <212> PRT
   <213> Arabidopsis thaliana
<400> 250
   Met Arg Phe Thr Thr Ser Ser Met Cys His Asn Tyr Gly Ser Val
   1               5                   10                  15
<210> 251
   <211> 25
   <212> PRT
   <213> Arabidopsis thaliana
<400> 251
   Met Arg Ile Arg Gly Arg Ile Gln Phe Lys Gly Gly Arg Leu Val Thr
   1               5                   10                  15
   Pro Ala Ile Leu Asn Arg Ile Val Gly
               20                  25
<210> 252
   <211> 30
   <212> PRT
   <213> Arabidopsis thaliana
<400> 252
   Met Ala Gly Thr Asp His Gly Pro Asn Asp Glu Arg Asp Ser Gly Lys
   1               5                   10                  15
   Ile Lys Gly Phe Val Lys Ser Leu Val Glu Leu Trp Arg Pro
               20                  25                  30
<210> 253
   <211> 42
   <212> PRT
   <213> Arabidopsis thaliana
<400> 253
   Met Ile Leu Phe Cys Leu Arg Leu Leu Lys Leu Ser Thr Ala His Ser
   1               5                   10                  15
   Arg Leu Lys Arg Ser Ala Ser Lys Arg Asp Ala Ile Phe Glu Tyr Asp
               20                  25                  30
   Arg Arg Leu Trp Asn Arg Gly Leu Pro Glu
           35                  40
<210> 254
   <211> 14
   <212> PRT
   <213> Arabidopsis thaliana
<400> 254
   Met Leu His Leu Pro Lys Leu Ser Ile Gly Leu Leu Gln Val
   1               5                   10
<210> 255
   <211> 31
   <212> PRT
   <213> Arabidopsis thaliana
<400> 255
   Met Gln Phe Ser Asn Met Ile Asp Asp Tyr Gly Thr Glu Val Tyr Leu
   1               5                   10                  15
   Asn Asp His Glu Glu Ser Arg Arg Pro His Ser Ser Ser Leu Val
               20                  25                  30
<210> 256
   <211> 50
   <212> PRT
   <213> Arabidopsis thaliana
<400> 256
   Met Gln Arg Arg Gly Trp Gln Gln Thr Asn Gln Met His Leu Gln Gln
   1               5                   10                  15
   Ile His Pro Ser Pro His Leu Leu Ile Pro Ile Ile Leu Gly Glu Ala
               20                  25                  30
   Arg Cys Leu Val Leu Val Ser Val Thr Leu Asp Ala Leu Gly Leu Asn
           35                  40                  45
   Lys Arg
       50
<210> 257
   <211> 19
   <212> PRT
   <213> Arabidopsis thaliana
<400> 257
   Met Thr Gly Ile Ser Cys Asp Ile Trp Met Thr Ser Phe Leu Gln Arg
   1               5                   10                  15
   Thr Ile Gly
<210> 258
   <211> 61
   <212> PRT
   <213> Arabidopsis thaliana
<400> 258
   Met Thr Ser Trp Val Leu Ile Glu Val Leu Ile Ser Leu Arg Ser Asn
   1               5                   10                  15
   Gln Pro Ile Val Arg Cys Arg Asn Asp Val Ile Gln Ile Ser Gln Glu
               20                  25                  30
   Ile Pro Val Met Ile Trp Pro Ser Phe Leu Thr Arg Leu Thr Ser Phe
           35                  40                  45
   Ser Leu Ile Arg Ser Arg Ala Lys Pro Met Thr Arg Leu
       50                  55                  60
<210> 259
   <211> 12
   <212> PRT
   <213> Arabidopsis thaliana
<400> 259
   Met Ala Met Phe Asn Ile Ser Glu Ala His His Arg
   1               5                   10
<210> 260
   <211> 11
   <212> PRT
   <213> Arabidopsis thaliana
<400> 260
   Met Ser Ser Arg Tyr Arg Arg Arg Ser Arg Ser
   1               5                   10
<210> 261
   <211> 21
   <212> PRT
   <213> Arabidopsis thaliana
<400> 261
   Met Gln Pro Phe Val Gly Thr Arg Ser Thr Leu His Val Leu Ala Glu
   1               5                   10                  15
   Thr Pro Leu His Pro
               20
<210> 262
   <211> 26
   <212> PRT
   <213> Arabidopsis thaliana
<400> 262
   Met Gln Gly Met Asp Val Thr Phe Arg Lys Gly Glu Arg Asn Gly Cys
   1               5                   10                  15
   Asp Leu Ser Glu Gly Cys Lys Glu Arg Met
               20                  25
<210> 263
   <211> 21
   <212> PRT
   <213> Arabidopsis thaliana
<400> 263
   Met Gln Lys Thr Asp Val Thr Phe Trp Lys Asp Ala Arg Asn Gly Cys
   1               5                   10                  15
   Asp Leu Ser Glu Gly
               20
<210> 264
   <211> 20
   <212> PRT
   <213> Arabidopsis thaliana
<400> 264
   Met Leu Trp Glu Ala Ser Ile Arg Pro Leu Tyr Lys Gly Gly Arg Pro
   1               5                   10                  15
   Arg Pro Leu Glu
               20
<210> 265
   <211> 12
   <212> PRT
   <213> Arabidopsis thaliana
<400> 265
   Met Thr Gly Ile Lys Arg Arg Arg Arg Leu Leu Phe
   1               5                   10
<210> 266
   <211> 12
   <212> PRT
   <213> Arabidopsis thaliana
<400> 266
   Met Phe Ile Phe Asp Gly Leu Lys Phe Lys Lys Ile
   1               5                   10
<210> 267
   <211> 19
   <212> PRT
   <213> Arabidopsis thaliana
<400> 267
   Met Lys Phe Met Asp Leu Val Asp Pro Ile Leu Val His Gly Phe Ser
   1               5                   10                  15
   Ala Glu Ile
<210> 268
   <211> 17
   <212> PRT
   <213> Arabidopsis thaliana
<400> 268
   Met Glu Ala Leu Arg Pro Lys Ser Cys Ser Ser Thr Pro Ser Thr Phe
   1 5 10 15
   His
<210> 269
   <211> 29
   <212> PRT
   <213> Arabidopsis thaliana
<400> 269
   Met His Glu Leu Ile Leu Leu Tyr Thr Lys Gln Lys Arg Pro Lys Thr
   1 5 10 15
   Ile Lys Ala Pro Gly Ile Pro Ala Pro Thr Ala Ile Ala
   20 25
<210> 270
   <211> 19
   <212> PRT
   <213> Arabidopsis thaliana
<400> 270
   Met Gly Leu Ser Val Val Asp Leu Trp Gly Trp Asp Glu Thr Thr Pro
   1 5 10 15
   Phe Asn Ala
<210> 271
   <211> 31
   <212> PRT
   <213> Arabidopsis thaliana
<400> 271
   Met Ala Thr Thr Arg Leu Leu Thr Cys Ala Arg Glu Arg Arg Arg Ile
   1 5 10 15
   Leu Arg Pro Ser Lys Arg Asp Gln Arg Lys Asn Lys Arg Asp Thr
   20 25 30
<210> 272
   <211> 15
   <212> PRT
   <213> Arabidopsis thaliana
<400> 272
   Met Val Ile Ser Phe Ala Gly Tyr Ser Pro Lys Glu Ser Ala Arg
   1 5 10 15
<210> 273
   <211> 11
   <212> PRT
   <213> Arabidopsis thaliana
<400> 273
   Met Ile Ser Pro Arg Val Leu Gly Ile His Leu
   1 5 10
<210> 274
   <211> 14
   <212> PRT
   <213> Arabidopsis thaliana
<400> 274
   Met Asp Arg Ser Lys Pro Leu Arg Ile Ala Tyr Tyr Gly Lys
   1 5 10
<210> 275
   <211> 11
   <212> PRT
   <213> Arabidopsis thaliana
<400> 275
   Met Thr Phe Leu Ile Trp Thr Asp Arg Ser Pro
   1 5 10
<210> 276
   <211> 17
   <212> PRT
   <213> Arabidopsis thaliana
<400> 276
   Met Ile Val Thr Arg Lys Leu Gly Ile Gly Ser Gly Ser Ser Tyr Leu
   1 5 10 15
   Pro
<210> 277
   <211> 29
   <212> PRT
   <213> Arabidopsis thaliana
<400> 277
   Met Thr Ala Gln Arg Met Ser Thr Ile Gly Met Lys Trp Ile Ala Phe
   1 5 10 15
   Ser Ala Asp Leu Asn Arg Asp Leu Phe Phe Phe Pro Gly
   20 25
<210> 278
   <211> 10
   <212> PRT
   <213> Arabidopsis thaliana
<400> 278
   Met Leu Asn Val Val Leu Thr Glu Lys Cys
   1 5 10
<210> 279
   <211> 10
   <212> PRT
   <213> Arabidopsis thaliana
<400> 279
   Met Leu Asn Cys Val Leu Ala Gly Lys Cys
   1 5 10
<210> 280
   <211> 27
   <212> PRT
   <213> Arabidopsis thaliana
<400> 280
   Met Leu Asn Cys Val Leu Ala Glu Asn Val Glu Leu Arg Phe Gly Gly
   1 5 10 15
   Lys Met Leu Asn Val Val Leu Ala Gly Lys Cys
   20 25
<210> 281
   <211> 28
   <212> PRT
   <213> Arabidopsis thaliana
<400> 281
   Met Leu Ile Val Val Phe Gly Gly Lys Met Leu Asn Val Phe Phe Gly
   1 5 10 15
   Gly Lys Met Leu Asn Cys Val Leu Ala Gly Lys Cys
   20 25
<210> 282
   <211> 19
   <212> PRT
   <213> Arabidopsis thaliana
<400> 282
   Met Leu Asn Cys Val Phe Asp Gly Lys Met Leu Asn Cys Val Leu Val
   1 5 10 15
   Glu Lys Cys
<210> 283
   <211> 10
   <212> PRT
   <213> Arabidopsis thaliana
<400> 283
   Met Leu Asn Cys Val Leu Ala Glu Lys Cys
   1 5 10
<210> 284
   <211> 34
   <212> PRT
   <213> Arabidopsis thaliana
<400> 284
   Met Ser Phe Trp Arg Glu Asn Val Glu Cys Arg Phe Asp Gly Lys Met
   1 5 10 15
   Leu Asn Val Val Phe Gly Arg Lys Met Leu Asn Cys Phe Leu Ala Gly
   20 25 30
   Lys Cys
<210> 285
   <211> 19
   <212> PRT
   <213> Arabidopsis thaliana
<400> 285
   Met Leu Ile Val Val Phe Gly Gly Lys Met Leu Asn Val Phe Phe Gly
   1 5 10 15
   Gly Lys Met
<210> 286
   <211> 28
   <212> PRT
   <213> Arabidopsis thaliana
<400> 286
   Met Val Val Val Ser Val Arg Asp Ser Lys Glu Val His Asp Gly Arg
   1 5 10 15
   Val Met Val Glu Ala Met Ile Ser Trp Leu Arg Val
   20 25
<210> 287
   <211> 18
   <212> PRT
   <213> Arabidopsis thaliana
<400> 287
   Met Tyr Trp Ile Ile Val Gly Phe Leu Ala Arg Phe Gly Asp Ala Val
   1 5 10 15
   Glu Cys
<210> 288
   <211> 11
   <212> PRT
   <213> Arabidopsis thaliana
<400> 288
   Met Leu Met Leu Leu Lys His Asp Leu Phe Leu
   1 5 10
<210> 289
   <211> 14
   <212> PRT
   <213> Arabidopsis thaliana
<400> 289
   Met Gln Lys Leu Leu His Gly Gly Leu Phe Gly Ile Leu Ser
   1 5 10
<210> 290
   <211> 29
   <212> PRT
   <213> Arabidopsis thaliana
<400> 290
   Met Ile Phe Ala Met Thr Lys Phe Gln Asn Gly Lys Lys Lys Lys Lys
   1 5 10 15
   Gln Ile Pro Lys Trp Val Glu Leu Met Gly Cys Asp Ala
   20 25
<210> 291
   <211> 14
   <212> PRT
   <213> Arabidopsis thaliana
<400> 291
   Met Glu Lys Lys Lys Lys Asn Lys Phe Gln Asn Gly Trp Asn
   1 5 10
<210> 292
   <211> 33
   <212> PRT
   <213> Arabidopsis thaliana
<400> 292
   Met Arg Ala Ser Lys Ser Thr Lys Lys Val Phe Tyr Ser Ile Ile Glu
   1 5 10 15
   Leu Ser Phe Lys Gly Gly Gln Glu Thr Leu Cys Cys Val Arg Met Ser
   20 25 30
   Arg
<210> 293
   <211> 46
   <212> PRT
   <213> Arabidopsis thaliana
<400> 293
   Met Leu Ser Cys Glu Arg Val Ser Arg Arg Arg Lys Ser Ser Ile Leu
   1 5 10 15
   Leu Ser Ser Cys Arg Ser Lys Ala Val Lys Lys Pro Phe Val Ala Phe
   20 25 30
   Glu Cys Pro Val Glu Gly Ser Thr Ile Ser Tyr Cys Cys Cys
   35 40 45
<210> 294
   <211> 10
   <212> PRT
   <213> Arabidopsis thaliana
<400> 294
   Met Gln Phe Pro Val Ile Thr Gln Cys Tyr
   1 5 10
<210> 295
   <211> 36
   <212> PRT
   <213> Arabidopsis thaliana
<400> 295
   Met Ser Thr Thr Lys Gln Ser Gly Val Gly Arg Leu Met Leu Leu Arg
   1               5                   10                  15
   Phe Phe Ser Leu Asn Cys Phe Gly Cys Tyr Val Val Gly Ile Leu Ile
               20                  25                  30
   Asp Leu Cys His
           35
<210> 296
   <211> 47
   <212> PRT
   <213> Arabidopsis thaliana
<400> 296
   Met His Arg Lys Lys Ser Ile Phe His Tyr Gln Tyr Cys Val Lys Ile
   1               5                   10                  15
   Met Arg Leu Leu Ile Leu Leu Gln Ile Tyr Asn Tyr Arg Ser Ile Asn
               20                  25                  30
   Gly Arg Asp Leu Ser Lys Ser Gln Gln His Asn Ile Gln Asn Ser
           35                  40                  45
<210> 297
   <211> 22
   <212> PRT
   <213> Arabidopsis thaliana
<400> 297
   Met Ser Tyr Gly Leu Thr Gln Leu Ile Leu Leu Asn Gly Leu Asp Gln
   1               5                   10                  15
   Pro Ile Thr His Leu Ile
               20
<210> 298
   <211> 30
   <212> PRT
   <213> Arabidopsis thaliana
<400> 298
   Met Ser Leu Lys Leu Asn Glu Leu Trp Val Asp Pro Thr His Phe Val
   1               5                   10                  15
   Lys Trp Val Gly Ser Thr His Asn Ser Phe Asn Leu Met Gly
               20                  25                  30
<210> 299
   <211> 13
   <212> PRT
   <213> Arabidopsis thaliana
<400> 299
   Met Val Asp Thr Gly Pro Val Arg Ser Val Leu Tyr Ser
   1               5                   10
<210> 300
   <211> 23
   <212> PRT
   <213> Arabidopsis thaliana
<400> 300
   Met Leu Arg Phe Leu Trp Trp Ile Leu Val Leu Leu Gly Arg Tyr Cys
   1               5                   10                  15
   Thr Val Asp Gly Ala Ala Thr
               20
<210> 301
   <211> 49
   <212> PRT
   <213> Arabidopsis thaliana
<400> 301
   Met Gly Leu Leu Pro Lys Cys Trp Thr Phe Thr Asp Trp Tyr Gln Pro
   1               5                   10                  15
   Leu Met Gly Glu Ile Met Leu Leu Leu Gly Leu Thr Phe Ser Trp Pro
               20                  25                  30
   Ile Val Gln Glu Lys Val Ile Thr Ser Ser Leu Arg His Arg Tyr Arg
           35                  40                  45
   Asn
<210> 302
   <211> 14
   <212> PRT
   <213> Arabidopsis thaliana
<400> 302
   Met Ser Asp Pro Ile Ala Cys Leu Glu Cys Gln His Glu Thr
   1 5 10
<210> 303
   <211> 13
   <212> PRT
   <213> Arabidopsis thaliana
<400> 303
   Met Lys Pro Asn Tyr Asp Ser Gly Gly Glu Asp Leu Lys
   1 5 10
<210> 304
   <211> 33
   <212> PRT
   <213> Arabidopsis thaliana
<400> 304
   Met Ala Phe Lys Ser Arg Lys Ile Gly Lys Asn Lys Ser Asn Ile Lys
   1 5 10 15
   Glu Asn Lys Arg Lys Gly Lys Glu Lys Ser Asn Gln Trp Val Ala Ser
   20 25 30
   His
<210> 305
   <211> 15
   <212> PRT
   <213> Arabidopsis thaliana
<400> 305
   Met Pro Leu Gly Thr Met Ser Phe Lys Ser Arg Gly Gly Ser Tyr
   1 5 10 15
<210> 306
   <211> 20
   <212> PRT
   <213> Arabidopsis thaliana
<400> 306
   Met Asn Ala Gly Arg Asp Arg Tyr Ser Val Pro Leu His Arg Lys Ser
   1 5 10 15
   Asn Asn Pro Ser
   20
<210> 307
   <211> 14
   <212> PRT
   <213> Arabidopsis thaliana
<400> 307
   Met Tyr Leu Ser Leu Met Ile Leu Gln Val Met Gln Ser Tyr
   1 5 10
<210> 308
   <211> 19
   <212> PRT
   <213> Arabidopsis thaliana
<400> 308
   Met His Glu Pro Val Ala Val Asp Gly Pro Leu Gly Leu Lys Pro Gly
   1 5 10 15
   Leu Ala Leu
<210> 309
   <211> 32
   <212> PRT
   <213> Arabidopsis thaliana
<400> 309
   Met Ser Leu Trp Arg Leu Met Asp His Trp Ala Ser Ser Leu Ala Leu
   1 5 10 15
   Leu Cys Arg Leu Cys Ser Ile Glu Pro Glu Arg Pro Leu Met Cys Ile
   20 25 30
<210> 310
   <211> 48
   <212> PRT
   <213> Arabidopsis thaliana
<400> 310
   Met Arg Leu Gly Glu Ser Glu Val Phe Asn Ser Ala Gln Gln Ser Ala
   1 5 10 15
   Glu Ile Arg Leu Phe Arg Arg Met Ala Phe Gly Ser Gln Asn Tyr Ser
   20 25 30
   Thr Asp Phe Phe Ser His Ser Ser Tyr Asn Ser Arg Asp Ala Asn Val
   35 40 45
<210> 311
   <211> 21
   <212> PRT
   <213> Arabidopsis thaliana
<400> 311
   Met Ala Glu Ile Tyr Gln Asn Pro Asn Asn Ile Thr Ser Lys Thr Val
   1               5                   10                  15
   Lys Thr Lys Glu Thr
               20
<210> 312
   <211> 30
   <212> PRT
   <213> Arabidopsis thaliana
<400> 312
   Met Ile Phe Thr Arg Leu Arg Leu Cys Tyr Cys Asn Tyr Tyr Ala Arg
   1               5                   10                  15
   Lys Asn Leu Tyr Cys Asn Phe Gly Tyr Gln Met Pro Phe Phe
               20                  25                  30
<210> 313
   <211> 25
   <212> PRT
   <213> Arabidopsis thaliana
<400> 313
   Met Ser Thr Asn Gln Glu Lys Ala Lys Pro Glu Gly Asp Ser Tyr Cys
   1               5                   10                  15
   Phe Asn His Ser Glu Lys Ala Gln Leu
               20                  25
<210> 314
   <211> 16
   <212> PRT
   <213> Arabidopsis thaliana
<400> 314
   Met Val Cys Ala Thr Asn Gly Glu Arg Arg Lys Arg Thr Ile Cys Val
   1               5                   10                  15
<210> 315
   <211> 23
   <212> PRT
   <213> Arabidopsis thaliana
<400> 315
   Met Arg Glu Lys Ile Cys Thr Val Ile Leu Ala Thr Lys Cys His Ser
   1 5 10 15
   Ser Lys Gln Ser Asp Leu Cys
   20
<210> 316
   <211> 27
   <212> PRT
   <213> Arabidopsis thaliana
<400> 316
   Met Ala Thr Pro Phe Phe Pro Ile Asp Pro Leu Ser Phe Ser Gln Leu
   1 5 10 15
   Thr His Pro Arg Glu Thr Asp Phe Ala Glu Ser
   20 25
<210> 317
   <211> 15
   <212> PRT
   <213> Arabidopsis thaliana
<400> 317
   Met Met Ile Ser Met Ile Leu Pro Trp Leu Leu Leu Phe Phe Gln
   1 5 10 15
<210> 318
   <211> 22
   <212> PRT
   <213> Arabidopsis thaliana
<400> 318
   Met Tyr Lys Met Tyr Ala Cys Met Asp Val Cys Met Asn Ala Cys Met
   1 5 10 15
   Asp Val Trp Met Tyr Ala
   20
<210> 319
   <211> 33
   <212> PRT
   <213> Arabidopsis thaliana
<400> 319
   Met His Arg Tyr Phe Val Leu Gly Cys Arg Phe Ser Gly Phe Val Lys
   1               5                   10                  15
   Leu Asp Val Glu Glu Ala Asp Gln Val Leu Ile Val Leu Cys Gly Leu
               20                  25                  30
   Thr
<210> 320
   <211> 15
   <212> PRT
   <213> Arabidopsis thaliana
<400> 320
   Met Leu Leu Tyr Leu Val Tyr Gln Val Gly Thr Gly Thr Tyr Gln
   1               5                   10                  15
<210> 321
   <211> 49
   <212> PRT
   <213> Arabidopsis thaliana
<400> 321
   Met His Val Trp Met Tyr Gly Cys Met His Glu Cys Met Tyr Gly Cys
   1               5                   10                  15
   Met His Glu Cys Met Tyr Gly Cys Met His Glu Cys Met Tyr Gly Cys
               20                  25                  30
   Met His Glu Cys Thr Asp Ile Leu Phe Trp Asp Ala Asp Phe Pro Gly
           35                  40                  45
   Ser
<210> 322
   <211> 14
   <212> PRT
   <213> Arabidopsis thaliana
<400> 322
   Met Trp Lys Arg Leu Ile Lys Tyr Ser Ser Cys Phe Val Gly
   1               5                   10
<210> 323
   <211> 63
   <212> PRT
   <213> Arabidopsis thaliana
<400> 323
   Met Tyr Gly Cys Met His Glu Cys Met Tyr Gly Cys Met Asp Val Cys
   1 5 10 15
   Met Asn Ala Cys Met Asp Val Cys Met Asn Ala Cys Met Asp Val Cys
   20 25 30
   Met Asn Ala Cys Met Asp Val Cys Met Asn Ala Pro Ile Phe Cys Phe
   35 40 45
   Gly Met Gln Ile Phe Arg Val Arg Glu Thr Gly Cys Gly Arg Gly
   50 55 60
<210> 324
   <211> 49
   <212> PRT
   <213> Arabidopsis thaliana
<400> 324
   Met Ala Ser Ser Cys Gln Leu Leu Ala Ile Asn Val Gly Glu Gly Phe
   1 5 10 15
   Ala Ala Val Val Ala Ala Ser Phe Gly Arg Ser Arg Ala Asn Ser Phe
   20 25 30
   Asn Ser Glu Val Val Pro Asp Val Arg Leu Leu Pro Pro Ala Leu Gly
   35 40 45
   Gly
<210> 325
   <211> 20
   <212> PRT
   <213> Arabidopsis thaliana
<400> 325
   Met Arg Ser Val Arg Phe Leu Asp Ser Ile Leu Ser Tyr Asn Asn Phe
   1 5 10 15
   Val Leu Ser Asn
   20
<210> 326
   <211> 15
   <212> PRT
   <213> Arabidopsis thaliana
<400> 326
   Met Phe Gly Leu Arg Leu Leu Arg Arg Asn Leu Leu Gln Cys Phe
   1 5 10 15
<210> 327
   <211> 13
   <212> PRT
   <213> Arabidopsis thaliana
<400> 327
   Met Gln Val Arg Gly Glu Leu Phe Asn Arg Cys Leu Val
   1 5 10
<210> 328
   <211> 68
   <212> PRT
   <213> Arabidopsis thaliana
<400> 328
   Met Gln Ile Ala Lys Glu Tyr Thr Cys Thr Pro Ser His Ser Phe Ser
   1 5 10 15
   Leu Phe Ile Cys Cys Met Leu Arg Ser Lys Ser Phe Leu Arg Cys Lys
   20 25 30
   Leu Glu Gly Asn Ser Ser Ile Asp Val Leu Ser Arg Lys Ser Thr Glu
   35 40 45
   Ile Leu Phe Glu Ile Ser Gly Asn Ile Leu Lys Arg Glu His Pro Leu
   50 55 60
   Arg Ser Arg Arg
   65
<210> 329
   <211> 24
   <212> PRT
   <213> Arabidopsis thaliana
<400> 329
   Met Ser Cys Leu Glu Lys Ala Leu Arg Tyr Cys Leu Arg Tyr Gln Glu
   1 5 10 15
   Thr Ser Ser Asn Val Ser Ile Leu
   20
<210> 330
   <211> 20
   <212> PRT
   <213> Arabidopsis thaliana
<400> 330
   Met Leu Lys Asp Ile Ser Met Arg Gln Glu Thr Arg Pro Leu Thr Lys
   1 5 10 15
   Gln Asn Phe Thr
   20
<210> 331
   <211> 21
   <212> PRT
   <213> Arabidopsis thaliana
<400> 331
   Met Phe Met Phe Glu Phe Cys Ser Leu Trp Met Cys Phe Glu Phe Glu
   1 5 10 15
   Cys Ile Val Phe Glu
   20
<210> 332
   <211> 15
   <212> PRT
   <213> Arabidopsis thaliana
<400> 332
   Met Gln Ile Thr Tyr Arg Asp Asp Thr Ile Asn Met Arg Ile Glu
   1 5 10 15
<210> 333
   <211> 72
   <212> PRT
   <213> Arabidopsis thaliana
<400> 333
   Met Ser Gly Gln Ile Ser Leu Thr Leu Phe Ser Cys Leu Leu Cys His
   1 5 10 15
   Leu Asp Arg Leu Phe Ala Phe Gln Glu Pro Ser Leu Gly Leu Phe Gly
   20 25 30
   Leu Leu Thr Leu Ser Lys Asn Thr Pro Tyr Leu Glu Thr Asp Thr Phe
   35 40 45
   Thr Asp Thr Lys Ile Thr Gln Thr Gln Cys Thr Gln Thr Gln Asn Thr
   50 55 60
   Ser Lys Asp Tyr Lys Thr Gln Thr
   65 70
<210> 334
   <211> 49
   <212> PRT
   <213> Arabidopsis thaliana
<400> 334
   Met Leu Ile Ser Leu Ser Ile Cys Gln Val Arg Tyr Leu Ser Leu Tyr
   1 5 10 15
   Ser His Val Tyr Cys Val Ile Ser Ile Gly Tyr Leu His Phe Lys Asn
   20 25 30
   Arg Val Leu Ala Cys Leu Gly Tyr Ser His Tyr Pro Lys Thr His Leu
   35 40 45
   Ile
<210> 335
   <211> 15
   <212> PRT
   <213> Arabidopsis thaliana
<400> 335
   Met Asn Ile Gly Thr Leu Leu Leu Met Phe Tyr Val Ser Lys Arg
   1 5 10 15
<210> 336
   <211> 14
   <212> PRT
   <213> Arabidopsis thaliana
<400> 336
   Met Asn Arg Leu Val Phe Leu Ala Lys Glu Val Asn Asn His
   1 5 10
<210> 337
   <211> 17
   <212> PRT
   <213> Arabidopsis thaliana
<400> 337
   Met Leu Ala Asn Ala Glu Val Ser Ile Tyr Lys Ser Leu Ser Arg Thr
   1 5 10 15
   Leu
<210> 338
   <211> 17
   <212> PRT
   <213> Arabidopsis thaliana
<400> 338
   Met Phe Met Ser Ser Gly Phe Ile Asp Ser Thr Lys Arg Val Ser Ser
   1               5                   10                  15
   Ile
<210> 339
   <211> 15
   <212> PRT
   <213> Arabidopsis thaliana
<400> 339
   Met Tyr His Lys Asp Ser Ile Phe Leu Leu Leu Leu Val Val Leu
   1               5                   10                  15
<210> 340
   <211> 77
   <212> PRT
   <213> Arabidopsis thaliana
<400> 340
   Met Lys Thr Gly Asp Ala Tyr Leu Ser Ser Gly Met Tyr Ile His Glu
   1               5                   10                  15
   Ile Glu Met Lys Lys Phe Phe Asn Gly Tyr Ser Leu Leu Trp Leu Arg
               20                  25                  30
   Arg Leu Thr Gly Ser Tyr Thr Arg Asp Thr Phe Ser Gly Val Tyr Lys
           35                  40                  45
   Thr Arg Gly His Glu His Arg Leu Gln Cys Thr Thr Lys Ile Leu Ser
       50                  55                  60
   Phe Phe Phe Phe Leu Trp Phe Ser Glu Phe Leu Phe Leu
   65                  70                  75
<210> 341
   <211> 11
   <212> PRT
   <213> Arabidopsis thaliana
<400> 341
   Met Phe Lys Leu Trp Gly Phe Leu Thr Gln Gly
   1               5                   10
<210> 342
   <211> 14
   <212> PRT
   <213> Arabidopsis thaliana
<400> 342
   Met Leu Ile Phe Pro Leu Glu Cys Thr Ser Thr Arg Leu Lys
   1               5                   10
<210> 343
   <211> 40
   <212> PRT
   <213> Arabidopsis thaliana
<400> 343
   Met Asn Ile Val Phe Asn Val Pro Gln Arg Phe Tyr Leu Ser Ser Ser
   1               5                   10                  15
   Ser Cys Gly Ser Leu Ser Phe Tyr Phe Cys Lys Ser Thr Gln Cys Ile
               20                  25                  30
   Tyr Arg Val Leu Asp Asn Asp Leu
           35                  40
<210> 344
   <211> 17
   <212> PRT
   <213> Arabidopsis thaliana
<400> 344
   Met Val Phe Ile Glu Ser Ile Phe Lys Asp Trp Arg Ala Glu Glu Ala
   1               5                   10                  15
   Ser
<210> 345
   <211> 25
   <212> PRT
   <213> Arabidopsis thaliana
<400> 345
   Met Leu Thr Gln Ile Leu Pro Lys His Leu Gln Phe Lys Thr Asn Ser
   1               5                   10                  15
   Leu Thr Thr Arg Ile Arg Asp Thr Pro
               20                  25
<210> 346
   <211> 78
   <212> PRT
   <213> Arabidopsis thaliana
<400> 346
   Met Met Val Leu Ile Ile Arg Phe Ala Arg His Ser Tyr Asp Ala Leu
   1 5 10 15
   Ile Leu Phe Leu Met Ile Tyr Gln Tyr Arg Val Ser Ile Ser Cys Val
   20 25 30
   Phe Leu Cys Phe Pro Glu Leu Leu Trp Ser Ile Ser Asn Ser Ser Ser
   35 40 45
   Gln Thr Ile Gly Phe Glu Leu Gln Met Phe Trp Gln Asn Leu Gly Gln
   50 55 60
   His Phe Leu Val Lys Ile Cys Phe Phe Ser Gln His Phe Ser
   65 70 75
<210> 347
   <211> 14
   <212> PRT
   <213> Arabidopsis thaliana
<400> 347
   Met Phe Phe Phe His Ser Leu Ser Ser Cys Asp Tyr Arg Ser
   1 5 10
<210> 348
   <211> 40
   <212> PRT
   <213> Arabidopsis thaliana
<400> 348
   Met Val Cys Phe Ser Phe Ile His Ser Leu His Val Ile Ile Asp His
   1 5 10 15
   Asp Gly Ser Asn Tyr Pro Phe Cys Lys Thr Phe Ile Arg Arg Ser Tyr
   20 25 30
   Phe Ile Phe Asn Asp Leu Ser Val
   35 40
<210> 349
   <211> 21
   <212> PRT
   <213> Arabidopsis thaliana
<400> 349
   Met Met Thr Tyr Leu Ser Phe Ser Glu Ala Leu Val Ile Tyr His Pro
   1 5 10 15
   His Ile Ile Ser Ser
   20
<210> 350
   <211> 34
   <212> PRT
   <213> Arabidopsis thaliana
<400> 350
   Met Thr Lys Val Arg Ser Ile Ser Ser Leu Val Met Ile Thr Thr Pro
   1 5 10 15
   Lys Ala Thr Ser Ser Ile Ile Pro Ile Arg Arg Arg Gln Leu Leu Val
   20 25 30
   Glu Ile
<210> 351
   <211> 44
   <212> PRT
   <213> Arabidopsis thaliana
<400> 351
   Met Lys Lys Glu Asn Gly Ile Gly Thr Gln Met Lys Lys Ile Ile Thr
   1 5 10 15
   Ser Phe His Ile Leu Lys Lys Met Ser Arg Ser Gln Gln Glu Arg Ser
   20 25 30
   His Gln Val Lys Ser Leu Leu His Asp Gln Leu His
   35 40
<210> 352
   <211> 10
   <212> PRT
   <213> Arabidopsis thaliana
<400> 352
   Met Trp Lys Glu Val Ile Ile Phe Phe Ile
   1 5 10
<210> 353
   <211> 10
   <212> PRT
   <213> Arabidopsis thaliana
<400> 353
   Met Ser Met Ser Asn Thr Ser Lys Asp Ser
   1 5 10
<210> 354
   <211> 26
   <212> PRT
   <213> Arabidopsis thaliana
<400> 354
   Met Arg Asn Thr Gly Leu Ser Ser Ala Pro Ser Phe Leu Trp Cys Phe
   1 5 10 15
   Ser Gly Asp Thr Phe Cys Trp Arg Ser Ile
   20 25
<210> 355
   <211> 30
   <212> PRT
   <213> Arabidopsis thaliana
<400> 355
   Met Ala Phe Leu Gln Val Phe Phe Ser Met Ala Ser Trp Lys Ser Met
   1 5 10 15
   Gly Ser His Ser Ala Asn Lys Gln Asn Lys Val Arg Phe Ser
   20 25 30
<210> 356
   <211> 19
   <212> PRT
   <213> Arabidopsis thaliana
<400> 356
   Met Lys Ile Tyr Phe Ser Leu Leu Ser Ser Ser Leu Leu Arg Phe Ser
   1 5 10 15
   Ser Gly Thr
<210> 357
   <211> 19
   <212> PRT
   <213> Arabidopsis thaliana
<400> 357
   Met Leu Leu Ala Ile Ser Arg Ile Val Leu Phe Phe Leu Ser Ala Thr
   1 5 10 15
   Pro Phe Cys
<210> 358
   <211> 11
   <212> PRT
   <213> Arabidopsis thaliana
<400> 358
   Met Leu Arg Arg Met Ile Leu Gln Arg Asn Arg
   1               5                   10
<210> 359
   <211> 10
   <212> PRT
   <213> Arabidopsis thaliana
<400> 359
   Met Ser Arg Ala Asn Ile Ser Gln Ser Ile
   1               5                   10
<210> 360
   <211> 10
   <212> PRT
   <213> Arabidopsis thaliana
<400> 360
   Met Phe Ala Arg Leu Met Ile Lys Val Ser
   1               5                   10
<210> 361
   <211> 13
   <212> PRT
   <213> Arabidopsis thaliana
<400> 361
   Met Ser Leu Ser Ile Leu Arg Lys Leu Gly Pro His Arg
   1               5                   10
<210> 362
   <211> 27
   <212> PRT
   <213> Arabidopsis thaliana
<400> 362
   Met Asn His Ser Asn Ala Pro Arg Ile Cys Ser Pro Asn Leu Ile Thr
   1               5                   10                  15
   Ile Ser Arg Lys Lys Ala Ile Ile Phe Phe Ile
               20                  25
<210> 363
   <211> 13
   <212> PRT
   <213> Arabidopsis thaliana
<400> 363
   Met Lys Asn Cys Trp Lys Leu Pro Ser Thr Gly Leu Pro
   1 5 10
<210> 364
   <211> 15
   <212> PRT
   <213> Arabidopsis thaliana
<400> 364
   Met Ser Leu His Ile Cys Ser Ser Lys Gln Ser Cys Pro Ile Gln
   1 5 10 15
<210> 365
   <211> 21
   <212> PRT
   <213> Arabidopsis thaliana
<400> 365
   Met Thr Arg Glu Gln Gln Pro Leu Tyr Val Leu Phe Pro Ser Met Leu
   1 5 10 15
   Ala Cys Met Leu Val
   20
<210> 366
   <211> 49
   <212> PRT
   <213> Arabidopsis thaliana
<400> 366
   Met Lys Lys Ile Ile Ala Phe Phe Leu Asp Ile Val Ile Lys Leu Gly
   1 5 10 15
   Glu Gln Ile Leu Gly Ala Leu Leu Trp Phe Met Glu Ala Gln Leu Met
   20 25 30
   Gly Ala Ser Asn Asn Ser Ser Phe Leu Phe Lys His Gln Ser Val Val
   35 40 45
   Phe
<210> 367
   <211> 13
   <212> PRT
   <213> Arabidopsis thaliana
<400> 367
   Met Leu Val Asp Leu Leu Leu Asp Trp Thr Gly Leu Phe
   1               5                   10
<210> 368
   <211> 15
   <212> PRT
   <213> Arabidopsis thaliana
<400> 368
   Met Glu Gly Lys Arg Thr Tyr Asn Gly Cys Cys Ser Arg Val Ile
   1               5                   10                  15
<210> 369
   <211> 51
   <212> PRT
   <213> Arabidopsis thaliana
<400> 369
   Met Ile Cys His Val Ile Lys Leu Glu Lys Ser Val Glu Gly Gly Lys
   1               5                   10                  15
   Val Thr Arg Ala Trp Val Ala Arg Lys Val Leu Thr Pro Ser Arg Asn
               20                  25                  30
   Gly Gly Lys Val Val Asp Ser Pro Leu Ile Ser Ala Val Gly Lys Val
           35                  40                  45
   Glu Lys Ser
       50
<210> 370
   <211> 79
   <212> PRT
   <213> Arabidopsis thaliana
<400> 370
   Met Val Leu Lys Leu Gly Leu Leu Cys Thr Asn Leu Val Pro Glu Ser
   1               5                   10                  15
   Arg Pro Asp Met Val Lys Val Val Gln Tyr Leu Asp Arg Gln Val Ser
               20                  25                  30
   Leu Pro Asp Phe Ser Pro Asp Ser Pro Gly Ile Gly Ile Val Thr Pro
   35 40 45
   Val Leu Val Gly Gly Ser Ser Thr Val Ile Ser Asn Ile Ser Ser Pro
   50 55 60
   Val Thr Glu Phe Ile Thr His Ser Ile Gln Tyr Gly Ile Gly Arg
   65 70 75
<210> 371
   <211> 18
   <212> PRT
   <213> Arabidopsis thaliana
<400> 371
   Met Asn Val Val Asn Lys Lys Leu Ile Trp Pro Ser Trp Ser Lys Ala
   1 5 10 15
   Pro Asp
<210> 372
   <211> 48
   <212> PRT
   <213> Arabidopsis thaliana
<400> 372
   Met Leu Thr Val Gly Phe Glu Pro Thr Pro Phe Arg Thr Arg Thr Leu
   1 5 10 15
   Ile Trp Arg Leu Gly Pro Thr Arg Pro Tyr Gln Leu Phe Val Asn Asn
   20 25 30
   Val His Leu Gln Ala Ser Asn Phe Thr Ser His Arg Pro Ile Pro Tyr
   35 40 45
<210> 373
   <211> 63
   <212> PRT
   <213> Arabidopsis thaliana
<400> 373
   Met Leu Glu Ile Thr Val Glu Leu Pro Pro Thr Lys Thr Gly Val Thr
   1 5 10 15
   Ile Pro Ile Pro Gly Glu Ser Gly Glu Lys Ser Gly Asn Glu Thr Cys
   20 25 30
   Leu Ser Arg Tyr Cys Thr Thr Phe Thr Met Ser Gly Leu Asp Ser Gly
   35 40 45
   Thr Arg Phe Val Gln Ser Asn Pro Asn Phe Asn Thr Ile Ser Ile
   50 55 60
<210> 374
   <211> 14
   <212> PRT
   <213> Arabidopsis thaliana
<400> 374
   Met Ala Leu Arg Ile Pro Lys Leu Tyr Leu Val Leu Pro Val
   1 5 10
<210> 375
   <211> 16
   <212> PRT
   <213> Arabidopsis thaliana
<400> 375
   Met Arg Arg Ile Pro Glu Pro Val Gln Ile Thr Gln Gly Met Gln Ile
   1 5 10 15
<210> 376
   <211> 31
   <212> PRT
   <213> Arabidopsis thaliana
<400> 376
   Met Asp Cys Phe Asn Ala Asn Thr Asn Gln Arg Arg Glu Ala Thr Glu
   1 5 10 15
   Arg Asn Arg Thr Cys Lys Val Thr Ser Gly Leu Ser Ala Ser Gln
   20 25 30
<210> 377
   <211> 10
   <212> PRT
   <213> Arabidopsis thaliana
<400> 377
   Met Cys Phe Arg Asn Pro Ala Val Glu Ile
   1 5 10
<210> 378
   <211> 11
   <212> PRT
   <213> Arabidopsis thaliana
<400> 378
   Met Gly Asn Ser Cys Ala Ser Glu Thr Gln Leu
   1 5 10
<210> 379
   <211> 66
   <212> PRT
   <213> Arabidopsis thaliana
<400> 379
   Met Thr Val Thr Lys Ala Val Thr Leu Lys Pro Arg Arg Phe Met Val
   1 5 10 15
   Asn Leu Lys Met Val Lys Pro Phe His Leu Trp Met Gln Val Val Gln
   20 25 30
   Gly Val Arg Trp Thr Lys Lys Leu Gly Ser Val Leu Lys Val Leu Ser
   35 40 45
   Lys Val His Thr Leu Leu Lys Ala Val Leu Ala Leu Lys Gly Gln Arg
   50 55 60
   Met Gln
   65
<210> 380
   <211> 15
   <212> PRT
   <213> Arabidopsis thaliana
<400> 380
   Met Leu Met Val Leu Asp Lys Phe Cys Ser Ser Ser Leu Arg Asn
   1 5 10 15
<210> 381
   <211> 29
   <212> PRT
   <213> Arabidopsis thaliana
<400> 381
   Met Ser Leu Gly His Ser Gln Arg Gln Ser Ile His Ile Leu Leu His
   1 5 10 15
   Leu Tyr Cys Leu His Trp Leu Arg Tyr Pro Arg Leu Asp
   20 25
<210> 382
   <211> 13
   <212> PRT
   <213> Arabidopsis thaliana
<400> 382
   Met Gln Gln Asp Val Tyr Ala Leu Ser Leu Arg Val Ser
   1 5 10
<210> 383
   <211> 33
   <212> PRT
   <213> Arabidopsis thaliana
<210> 384
   <211> 21
   <212> PRT
   <213> Arabidopsis thaliana
<210> 385
   <211> 24
   <212> PRT
   <213> Arabidospsis thaliana
<210> 386
   <211> 18
   <212> PRT
   <213> Arabidopsis thaliana
<400> 386
   Met Gly Gln Tyr Ile Pro Lys Gly Ser Lys Lys Glu Ser Leu Ser Arg
   1 5 10 15
   Glu Ser
<210> 387
   <211> 32
   <212> PRT
   <213> Arabidopsis thaliana
<400> 387
   Met Lys Ile His Asp Gln Asn Ser Pro Ile Ser Thr Ile Asp Ser Val
   1 5 10 15
   Asn Glu Gln Leu Pro Phe Leu Ile Thr Asp Ser Asn Pro Phe Ala Gln
   20 25 30
<210> 388
   <211> 12
   <212> PRT
   <213> Arabidopsis thaliana
<400> 388
   Met Gly Asp Phe Asn Ser Ala Ala Val Met Ile His
   1 5 10
<210> 389
   <211> 19
   <212> PRT
   <213> Arabidopsis thaliana
<400> 389
   Met Val His Gly Phe Thr Pro His Thr Arg Ile Thr Gly Gln Lys Asp
   1 5 10 15
   Leu Ser Gln
<210> 390
   <211> 14
   <212> PRT
   <213> Arabidopsis thaliana
<400> 390
   Met Asp Leu Leu Leu Ile Leu Glu Leu Leu Gly Lys Arg Ile
   1 5 10
<210> 391
   <211> 21
   <212> PRT
   <213> Arabidopsis thaliana
<400> 391
   Met Asn Phe His Gly Arg Phe Gln Leu Gly Gly Gly Asp Asp Ser Leu
   1 5 10 15
   Thr Ser Phe Gln Ser
   20
<210> 392
   <211> 23
   <212> PRT
   <213> Arabidopsis thaliana
<400> 392
   Met Lys Lys Thr Leu Ile Ile Met Pro Gly Lys Asn Gln Asn Gln Arg
   1 5 10 15
   Lys Lys Glu Gln Arg Glu Ile
   20
<210> 393
   <211> 16
   <212> PRT
   <213> Arabidopsis thaliana
<400> 393
   Met Ile Asp Ser Ser Pro Phe Ser Asn Tyr Ala Ile Phe Asn Asn Val
   1 5 10 15
<210> 394
   <211> 15
   <212> PRT
   <213> Arabidopsis thaliana
<400> 394
   Met Pro His Gln Thr Gln Arg Glu Glu Gln Asp Leu Leu Tyr Leu
   1 5 10 15
<210> 395
   <211> 46
   <212> PRT
   <213> Arabidopsis thaliana
<400> 395
   Met Arg Thr Met Gln Arg Cys His Ile Lys Leu Arg Glu Lys Asn Arg
   1 5 10 15
   Ile Ser Tyr Thr Tyr Arg Gln Gly Lys Tyr Asn Arg Cys Ser Ile Tyr
   20 25 30
   Leu Phe Gln Lys Asp Thr Tyr Ser Arg Asn Ser Cys Phe Gln
   35 40 45
<210> 396
   <211> 10
   <212> PRT
   <213> Arabidopsis thaliana
<400> 396
   Met Leu His Leu Leu Tyr Phe Pro Cys Leu
   1 5 10
<210> 397
   <211> 30
   <212> PRT
   <213> Arabidopsis thaliana
<400> 397
   Met Trp His Leu Cys Met Val Leu Ile Ser Leu Arg Ser Ile Trp Asn
   1 5 10 15
   Tyr Val Leu Ser Phe Leu Val Lys Ala Ile Asn Thr Ser Phe
   20 25 30
<210> 398
   <211> 32
   <212> PRT
   <213> Arabidopsis thaliana
<400> 398
   Met Ser Phe Leu Asn Arg Tyr Leu Ser Glu Lys Gly Arg Cys Cys Ile
   1 5 10 15
   Cys Tyr Ile Phe Pro Val Tyr Lys Tyr Lys Arg Ser Cys Ser Ser Leu
   20 25 30
<210> 399
   <211> 63
   <212> PRT
   <213> Arabidopsis thaliana
<400> 399
   Met Leu Lys Arg Val Gly Asn Asn Ser Lys His Ala Ser Gly Asn Asn
   1               5                   10                  15
   Ile Ser Leu Ile Gly Glu Thr Leu Pro Thr Thr Ser Leu Ala Ser Phe
               20                  25                  30
   Ser Cys Leu Leu Leu Val Cys Ser Val Ala Phe Phe Ser Gly Lys Lys
           35                  40                  45
   Leu Arg Lys Cys Ser Cys Leu Pro Pro Ser Phe Leu Asn Arg Leu
       50                  55                  60
<210> 400
   <211> 18
   <212> PRT
   <213> Arabidopsis thaliana
<400> 400
   Met Leu Phe Pro Glu Ala Cys Phe Glu Leu Phe Pro Thr Arg Leu Ser
   1               5                   10                  15
   Ile Thr
<210> 401
   <211> 32
   <212> PRT
   <213> Arabidopsis thaliana
<400> 401
   Met Gly Glu Asp Thr Asn Ile Ser Leu Ala Phe Cys Gln Lys Arg Met
   1               5                   10                  15
   Pro Gln Ser Ile Gln Val Lys Asp Met Lys Met Met Arg Val Arg Leu
               20                  25                  30
<210> 402
   <211> 26
   <212> PRT
   <213> Arabidopsis thaliana
<400> 402
   Met Lys Lys Ala Arg Leu Gln Ser Pro Leu Cys Ser Arg Val Asn Asn
   1               5                   10                  15
   Lys Ile Ala Ala Tyr Gln Ser His Thr Val
               20                  25
<210> 403
   <211> 17
   <212> PRT
   <213> Arabidopsis thaliana
<400> 403
   Met Ala Tyr Phe Arg Ser Ala Ser Arg Asn Ser Pro Thr Phe Leu Asn
   1               5                   10                  15
   Arg
<210> 404
   <211> 10
   <212> PRT
   <213> Arabidopsis thaliana
<400> 404
   Met His Arg Leu Leu Leu Ile His Pro Asn
   1               5                   10
<210> 405
   <211> 13
   <212> PRT
   <213> Arabidopsis thaliana
<400> 405
   Met Leu Gly Ser Phe Val Lys Arg Tyr Gly Asn Thr Pro
   1               5                   10
<210> 406
   <211> 35
   <212> PRT
   <213> Arabidopsis thaliana
<400> 406
   Met Asn Lys Arg Arg Pro Pro Thr Tyr Asn Lys Met Glu Arg Leu Met
   1               5                   10                  15
   Glu Ser Lys Tyr Met Met Ile Trp Ile Leu Lys Met Lys Met Glu Val
               20                  25                  30
   Phe Lys Arg
           35
<210> 407
   <211> 31
   <212> PRT
   <213> Arabidopsis thaliana
<400> 407
   Met Val Ile Gly Tyr Leu Leu Asn Thr Ser Ile Phe Ile Leu Ser Ile
   1               5                   10                  15
   His Ile Ile Met Tyr Leu Asp Ser Ile Asn Leu Ser Ile Leu Leu
               20                  25                  30
<210> 408
   <211> 123
   <212> PRT
   <213> Arabidopsis thaliana
<400> 408
   Met Lys Lys Arg Arg Arg Arg Lys Lys Ile Ser Ser Asn Lys Ser Ser
   1               5                   10                  15
   Ile Cys Lys Leu Gln Lys Lys Lys Thr Ala Lys Val Thr Lys Glu Glu
               20                  25                  30
   Val Val Val Lys Tyr Glu Asp Glu Val Val Val Asp Met Glu Met Asp
           35                  40                  45
   Val Val Gly Gly His Met Lys Thr Thr Gln Thr Lys Glu Val Lys Thr
       50                  55                  60
   His Gln Glu Val Val Gly Lys Asp Thr Gln Asn Gln Asp Thr Ile Asn
   65                  70                  75                  80
   Gln Val Ser Asn Ala Thr Ile Val Gly Ser Leu Asp Ile Met Leu Leu
                   85                  90                  95
   Asn Val Lys Leu Leu Ala Thr Lys Asn Leu Arg Arg Arg Pro Thr Thr
               100                 105                 110
   Leu Lys Lys Lys Phe Lys Lys Lys Thr Cys Tyr
           115                 120
<210> 409
   <211> 32
   <212> PRT
   <213> Arabidopsis thaliana
<400> 409
   Met Asn Lys Lys Arg Ile Ile Ser Gly Thr Ser Ile Val Val Gln Val
   1               5                   10                  15
   Ile Thr Cys Ala Gly Glu Lys Val Cys Ser Arg Ser Leu Met Asn Arg
               20                  25                  30
<210> 410
   <211> 15
   <212> PRT
   <213> Arabidopsis thaliana
<400> 410
   Met Glu Ile Ile Asn Leu Phe Pro Thr Phe Thr Ile Phe Arg Ala
   1               5                   10                  15
<210> 411
   <211> 24
   <212> PRT
   <213> Arabidopsis thaliana
<400> 411
   Met Thr Leu His Asn Val Leu Arg Cys Val Thr Lys Arg Ser Leu Gly
   1               5                   10                  15
   Tyr Gly Ile Phe Asp Ser Asp Ile
               20
<210> 412
   <211> 32
   <212> PRT
   <213> Arabidopsis thaliana
<400> 412
   Met Cys Val Val Gln Ser Ser Arg Asn His Leu Val Lys Val Ile Thr
   1               5                   10                  15
   Ser Phe Ser Leu Leu Met Ile Phe Gln Glu Lys His Gly Tyr Ile Phe
               20                  25                  30
<210> 413
   <211> 11
   <212> PRT
   <213> Arabidopsis thaliana
<400> 413
   Met Leu Met Tyr Arg Met Arg Ser Gly Ala Asn
   1               5                   10
<210> 414
   <211> 34
   <212> PRT
   <213> Arabidopsis thaliana
<400> 414
   Met Thr Lys Val Arg Ser Ile Ser Ser Leu Val Met Ile Thr Thr Pro
   1 5 10 15
   Lys Ala Thr Ser Ser Ile Ile Pro Ile Arg Arg Arg Gln Leu Leu Val
   20 25 30
   Glu Ile
<210> 415
   <211> 49
   <212> PRT
   <213> Arabidopsis thaliana
<400> 415
   Met Lys Lys Glu Asn Gly Ile Gly Thr Gln Met Lys Lys Ile Ile Thr
   1 5 10 15
   Ser Phe His Ile Leu Lys Lys Met Ser Arg Ser Gln Gln Glu Arg Ser
   20 25 30
   His Gln Val Lys Ser Leu Leu His His Gln Leu His Gln Gln Val Leu
   35 40 45
   Lys
<210> 416
   <211> 17
   <212> PRT
   <213> Arabidopsis thaliana
<400> 416
   Met Pro Trp Met Lys Arg Ser Asn Gln Tyr Lys Arg Met Thr His Gly
   1 5 10 15
   Ser
<210> 417
   <211> 12
   <212> PRT
   <213> Arabidopsis thaliana
<400> 417
   Met Leu Gln Leu Trp Glu Val Trp Thr Leu Cys Phe
   1 5 10
<210> 418
   <211> 35
   <212> PRT
   <213> Arabidopsis thaliana
<400> 418
   Met Ala Ser Ser Ile Arg Thr Ser Lys Phe Trp Arg Ile Gly Val Ala
   1               5                   10                  15
   Phe Lys Glu Gly Asn Gly Glu Arg Ala Thr Leu Tyr Lys Ser Ser Lys
               20                  25                  30
   Ser Ser Val
           35
<210> 419
   <211> 40
   <212> PRT
   <213> Arabidopsis thaliana
<400> 419
   Met Ser Thr Trp Lys Ala Ile Gln Asn Glu Leu Ser Lys Gly Val Lys
   1               5                   10                  15
   Phe Lys Ser Thr Lys Ile Val Gly Ala Asn Thr Tyr Arg Cys Val Trp
               20                  25                  30
   Ser Asn Gln Ala Glu Ile Thr Trp
           35                  40
<210> 420
   <211> 18
   <212> PRT
   <213> Arabidopsis thaliana
<400> 420
   Met Gly Ile Phe Phe Glu Arg Lys Ile Arg Gly Val Arg Asn Phe Gln
   1               5                   10                  15
   Lys Val
<210> 421
   <211> 19
   <212> PRT
   <213> Arabidopsis thaliana
<400> 421
   Met Gly Val Asn Phe Thr Ser Lys Trp Thr Gln Asp Asn Trp Arg Glu
   1               5                   10                  15
   Val Gly Val
<210> 422
   <211> 45
   <212> PRT
   <213> Arabidopsis thaliana
<400> 422
   Met Ser Phe His Leu Phe Gly Leu Cys Cys Leu His Val Ala Ser Asn
   1               5                   10                  15
   His Val His Phe His Ile His His Asp Leu Val Leu Val Leu Asp His
               20                  25                  30
   His Leu Phe Phe Gly Asn Phe Gly Arg Phe Leu Leu Leu
           35                  40                  45
<210> 423
   <211> 10
   <212> PRT
   <213> Arabidopsis thaliana
<400> 423
   Met Trp Lys Glu Val Ile Ile Phe Phe Ile
   1               5                   10
<210> 424
   <211> 10
   <212> PRT
   <213> Arabidopsis thaliana
<400> 424
   Met Ser Met Ser Asn Thr Ser Lys Asp Ser
   1               5                   10
<210> 425
   <211> 26
   <212> PRT
   <213> Arabidopsis thaliana
<400> 425
   Met Arg Asn Thr Gly Leu Ser Ser Ala Pro Ser Phe Leu Trp Cys Phe
   1               5                   10                  15
   Ser Gly Asp Thr Phe Cys Trp Arg Ser Ile
               20                  25
<210> 426
   <211> 24
   <212> PRT
   <213> Arabidopsis thaliana
<400> 426
   Met Gly Phe Lys Leu Phe Glu Asn Phe Glu His Leu Gly Phe Phe Phe
   1 5 10 15
   Gln Lys Ile Tyr Pro Cys Phe Ser
   20
<210> 427
   <211> 23
   <212> PRT
   <213> Arabidopsis thaliana
<400> 427
   Met Ile Ser Ile Leu Gln Ser Asn Glu Asn Val Ser Phe Thr Phe Tyr
   1 5 10 15
   Leu His Leu Arg Phe Ile Ser
   20
<210> 428
   <211> 13
   <212> PRT
   <213> Arabidopsis thaliana
<400> 428
   Met Ile Leu Phe Leu Phe Ile Phe Leu Val Ala Ser His
   1 5 10
<210> 429
   <211> 12
   <212> PRT
   <213> Arabidopsis thaliana
<400> 429
   Met Ser Lys Leu Pro Thr Ile Val Ala Phe Asp Thr
   1 5 10
<210> 430
   <211> 56
   <212> PRT
   <213> Arabidopsis thaliana
<400> 430
   Met Trp Pro Pro Thr Thr Ser Ile Ser Ile Ser Thr Thr Thr Ser Ser
   1 5 10 15
   Ser Tyr Leu Thr Thr Thr Ser Ser Leu Val Thr Leu Ala Val Phe Phe
   20 25 30
   Phe Cys Asn Leu His Ile Glu Asp Leu Phe Asp Asp Ile Phe Phe Leu
   35 40 45
   Leu Leu Leu Phe Phe Ile Ser Leu
   50 55
<210> 431
   <211> 30
   <212> PRT
   <213> Arabidopsis thaliana
<400> 431
   Met Ala Phe Leu Gln Val Phe Phe Ser Met Ala Ser Trp Lys Ser Met
   1 5 10 15
   Gly Ser His Ser Ala Asn Lys Gln Asn Lys Val Arg Phe Ser
   20 25 30
<210> 432
   <211> 19
   <212> PRT
   <213> Arabidopsis thaliana
<400> 432
   Met Lys Ile Tyr Phe Ser Leu Leu Ser Ser Ser Leu Leu Arg Phe Ser
   1 5 10 15
   Ser Gly Thr
<210> 433
   <211> 19
   <212> PRT
   <213> Arabidopsis thaliana
<400> 433
   Met Leu Leu Ala Ile Ser Arg Ile Val Leu Phe Phe Leu Ser Ala Thr
   1 5 10 15
   Pro Phe Cys
<210> 434
   <211> 67
   <212> PRT
   <213> Arabidopsis thaliana
<400> 434
   Met Pro Leu Ser Ser Gln Tyr Leu Arg Asn Ser Leu Asp Val Asn Ser
   1               5                   10                  15
   Pro Pro Arg Ser Asp Leu Met Val Leu Ile Thr Arg Pro Leu Ser Phe
               20                  25                  30
   Ser Thr Trp Ala Leu Asn Phe Leu Lys Ile Ser Asn Thr Ser Asp Phe
           35                  40                  45
   Ser Phe Lys Lys Tyr Thr His Val Phe Leu Glu Lys Ser Ser Ile Lys
       50                  55                  60
   Arg Arg Lys
   65
<210> 435
   <211> 38
   <212> PRT
   <213> Arabidopsis thaliana
<400> 435
   Met Ser Phe Arg Met Leu Arg Thr Asn Ile Leu Phe Phe Asp Ile Gly
   1               5                   10                  15
   Thr Leu Val Met Arg Leu Leu Ser Trp Ser Leu Ile Glu Arg Leu Leu
               20                  25                  30
   Ser Phe Asn Leu Ile Ser
           35
<210> 436
   <211> 37
   <212> PRT
   <213> Arabidopsis thaliana
<400> 436
   Met Arg Met Phe Pro Leu Pro Phe Thr Ser Ile Phe Asp Ser Ser Pro
   1               5                   10                  15
   Lys Ala Thr Phe Pro Leu Thr Asp Ser Ser Ser Ser Ala Asn Ile Leu
               20                  25                  30
   Phe Leu Pro His Met
           35
<210> 437
   <211> 11
   <212> PRT
   <213> Arabidopsis thaliana
<400> 437
   Met Ile Ser Ser Phe Phe Phe Phe Phe Ser Ser
   1               5                   10
<210> 438
   <211> 51
   <212> PRT
   <213> Arabidopsis thaliana
<400> 438
   Met Ser Pro Phe Ser Phe Ser Arg Thr Thr Gly Ala Pro Ser Val Gln
   1               5                   10                  15
   Arg Ile Phe Asn Phe Pro Phe Arg Ile Lys Arg Arg Ile Lys Lys Gly
               20                  25                  30
   Trp Ser Val Lys Ser Pro Val Pro Ser Arg Thr Lys Phe Arg Ile Asn
           35                  40                  45
   Lys Gly Ser
       50
<210> 439
   <211> 32
   <212> PRT
   <213> Arabidopsis thaliana
<400> 439
   Met Arg Thr Phe Leu Gly Thr Tyr Asp Lys Cys Leu Ala Val Ser Glu
   1               5                   10                  15
   Asn Asp Phe Ser Asp Asn Pro Thr Val Cys Gly Ala Gly Cys Asn Gly
               20                  25                  30
<210> 440
   <211> 26
   <212> PRT
   <213> Arabidopsis thaliana
<400> 440
   Met Ile Lys Gln Ile Val Ile Ser Ser Val Val His Lys Met Ser Arg
   1               5                   10                  15
   His Cys Asp Pro Phe Leu His His Thr Arg
               20                  25
<210> 441
   <211> 37
   <212> PRT
   <213> Arabidopsis thaliana
<400> 441
   Met Thr Glu Arg Val Gln Val Val Leu Cys Phe Thr Leu Glu Thr Arg
   1               5                   10                  15
   Leu Ser His Gly Cys Arg Arg Asn Asn Gln Leu Ser Leu Tyr Pro Leu
               20                  25                  30
   Val Lys Arg Ser Met
           35
<210> 442
   <211> 27
   <212> PRT
   <213> Arabidopsis thaliana
<400> 442
   Met Ile Glu Val Asn Thr Leu Thr His Ala Ile Thr Thr Leu Glu Ser
   1               5                   10                  15
   Val Leu Ala Lys Arg Thr Cys Asn Trp Ser Met
               20                  25
<210> 443
   <211> 14
   <212> PRT
   <213> Arabidopsis thaliana
<400> 443
   Met Asp Val Glu Glu Thr Thr Asn Cys His Ser Ile His Leu
   1               5                   10
<210> 444
   <211> 42
   <212> PRT
   <213> Arabidopsis thaliana
<400> 444
   Met Cys Met Pro Cys Tyr Leu Val Lys Lys Pro Leu Glu Gly Val Lys
   1               5                   10                  15
   Leu Thr Thr Arg Gly Thr Asn Glu Asp Leu Cys Gly Gln Gln Val Gly
               20                  25                  30
   Asn Ser Phe Gly Glu Glu Pro Gly Leu Pro
           35                  40
<210> 445
   <211> 55
   <212> PRT
   <213> Arabidopsis thaliana
<400> 445
   Met Cys Leu His Ile Leu Gln Leu His Val Leu Phe Ala Asn Thr Leu
   1               5                   10                  15
   Ser Asn Val Val Ile Ala Cys Val Asn Val Phe Thr Ser Ile Met Glu
               20                  25                  30
   Asp Arg Val Leu Arg Gln Ser Tyr Cys Arg Leu Val Val His Lys Asp
           35                  40                  45
   Leu Arg Trp Phe Leu Leu Trp
       50                  55
<210> 446
   <211> 78
   <212> PRT
   <213> Arabidopsis thaliana
<400> 446
   Met Cys Leu Leu Arg Ser Trp Lys Thr Gly Phe Phe Ala Lys Ala Ile
   1               5                   10                  15
   Ala Asp Leu Leu Ser Thr Lys Ile Phe Val Gly Ser Ser Cys Gly Lys
               20                  25                  30
   Leu Asn Ser Phe Lys Arg Phe Leu Asn Gln Ile Ala Trp His Thr His
           35                  40                  45
   Asp Val Ala Ala Thr Tyr Ser Ala Ser Gln Val Asp Arg Val Thr Ile
       50                  55                  60
   Gly Cys Phe Phe Asp Ile His Val Lys Ala Val Ser Pro Met
   65                  70                  75
<210> 447
   <211> 78
   <212> PRT
   <213> Arabidopsis thaliana
<400> 447
   Met Leu Asp Ser Glu Cys Leu Leu Glu Ile Tyr Ser Leu Cys Leu Ser
   1 5 10 15
   Leu Leu Leu Leu Lys Glu Leu Asn Leu Ile Val Trp Arg Phe Met Arg
   20 25 30
   Lys Arg Ser Ile Arg Phe Ser Lys Arg Trp Ile Ser Tyr Leu Gly Arg
   35 40 45
   Leu Val Leu Val Leu Met Cys Gly Val Val Arg Val Ile Leu Met Asn
   50 55 60
   Ser Cys Val Trp Gln Arg Ile Ile Leu Met Lys Asp Gly Asn
   65 70 75
<210> 448
   <211> 48
   <212> PRT
   <213> Arabidopsis thaliana
<400> 448
   Met Arg Trp Ile Asn His Lys Glu Val Gln Asn Ser Leu Phe Gln Phe
   1 5 10 15
   Pro Ser Phe Ile Asn Ile Met Arg Cys Gln Thr Gln Glu Phe Ile Arg
   20 25 30
   Ile Thr Arg Thr Thr Pro His Ile Asn Thr Asn Thr Asn Leu Pro Arg
   35 40 45
<210> 449
   <211> 25
   <212> PRT
   <213> Arabidopsis thaiiana
<400> 449
   Met Leu Leu Phe Arg Ile Asn Leu His Thr Ile Arg Phe Asn Ser Phe
   1 5 10 15
   Lys Ser Asn Lys Leu Lys Gln Arg Leu
   20 25
<210> 450
   <211> 67
   <212> PRT
   <213> Arabidopsis thaliana
<400> 450
   Met Asn Leu Ile Val Val Tyr Ala Ala Pro Ser Met Ser Arg Arg Ser
   1               5                   10                  15
   Gly Leu Trp Gly Glu Leu Lys Glu Val Val Ser Gly Leu Val Gly Leu
               20                  25                  30
   Leu Ile Ile Gly Gly Asp Phe Ile Thr Ile Leu Arg Val Asp Glu Arg
           35                  40                  45
   Met Gly Gly Asn Gly Arg Leu Ser Pro Asp Phe Leu Ala Phe Gly Asp
       50                  55                  60
   Trp Ile Asn
   65
<210> 451
   <211> 14
   <212> PRT
   <213> Arabidopsis thaliana
<400> 451
   Met Arg Glu Trp Val Glu Met Gly Gly Phe His Gln Ile Phe
   1               5                   10
<210> 452
   <211> 28
   <212> PRT
   <213> Arabidopsis thaliana
<400> 452
   Met Gly Arg Val Glu Gly Ser Cys Ile Trp Phe Gly Arg Ala Val Asp
   1               5                   10                  15
   Asn Trp Arg Arg Leu Tyr His His Leu Glu Ser Gly
               20                  25
<210> 453
   <211> 31
   <212> PRT
   <213> Arabidopsis thaliana
<400> 453
   Met Leu Leu Ala Phe Phe Thr Val Val Glu Thr Ser Met Ile Cys Phe
   1               5                   10                  15
   Val Asp Gln Ile Leu Leu Pro His Ser Leu Tyr Asp Gln Arg Lys
               20                  25                  30
<210> 454
   <211> 10
   <212> PRT
   <213> Arabidopsis thaliana
<400> 454
   Met Ile Lys Glu Ser Glu Ile Leu Tyr Pro
   1 5 10
<210> 455
   <211> 16
   <212> PRT
   <213> Arabidopsis thaliana
<400> 455
   Met Asp Asn Arg Met Arg Leu Ile Met Gly Thr Lys Phe His Phe Leu
   1 5 10 15
<210> 456
   <211> 13
   <212> PRT
   <213> Arabidopsis thaliana
<400> 456
   Met Asn Ile Thr Gly Thr Glu Trp Ile Thr Glu Cys Asp
   1 5 10
<210> 457
   <211> 13
   <212> PRT
   <213> Arabidopsis thaliana
<400> 457
   Met Ser Glu Ala Lys Glu Phe Gly Gln Arg Asn Lys Ser
   1 5 10
<210> 458
   <211> 49
   <212> PRT
   <213> Arabidopsis thaliana
<400> 458
   Met Arg Ala Leu Thr Ile Arg Leu Glu Thr Asn Phe Val Ser Ser Leu
   1               5                   10                  15
   Ser Phe Leu Ala Arg Phe Ser Ser Ile Trp Met Arg Arg Asp Trp Thr
               20                  25                  30
   Cys Val Trp Thr Arg Thr Lys Ser Cys Ile Tyr Phe Ile Ala Phe Ile
           35                  40                  45
   Val
<210> 459
   <211> 10
   <212> PRT
   <213> Arabidopsis thaliana
<400> 459
   Met Tyr Ile Leu Lys Tyr Gly His Phe Ser
   1               5                   10
<210> 460
   <211> 27
   <212> PRT
   <213> Arabidopsis thaliana
<400> 460
   Met Thr Ile Leu Ser Leu Asp Arg Ser Arg Arg Ala Gly Gly Ser Ile
   1               5                   10                  15
   Arg Ile Val Tyr Gly Phe Ala Lys Tyr Gly Ser
               20                  25
<210> 461
   <211> 14
   <212> PRT
   <213> Arabidopsis thaliana
<400> 461
   Met Asp Ile Phe Tyr Pro Ile Tyr Lys Arg Tyr Ile Ser Asp
   1               5                   10
<210> 462
   <211> 23
   <212> PRT
   <213> Arabidopsis thaliana
<400> 462
   Met Asp Ile Ser Leu Asp Thr Ile Tyr Leu Val Met Leu Lys Asn Leu
   1 5 10 15
   Gly Tyr Asn Val Thr Ile Val
   20
<210> 463
   <211> 24
   <212> PRT
   <213> Arabidopsis thaliana
<400> 463
   Met Asn Val Leu Ile Cys Phe Leu Phe Arg Cys Val Cys Ser Phe Leu
   1 5 10 15
   Val Ile Tyr Ile Leu Gln Phe Ser
   20
<210> 464
   <211> 21
   <212> PRT
   <213> Arabidopsis thaliana
<400> 464
   Met His Gly Thr Phe Arg Phe Ala Arg Ser Cys Leu Tyr Pro Leu Gln
   1 5 10 15
   Leu Asp Ser Thr Ile
   20
<210> 465
   <211> 51
   <212> PRT
   <213> Arabidopsis thaliana
<400> 465
   Met Gly His Phe Val Leu Leu Asp Arg Ala Cys Thr His Ser Asn Leu
   1 5 10 15
   Thr Val Leu Phe Asn Ser Arg Val Phe Ala Phe Cys Phe Phe Ser Ser
   20 25 30
   Glu Arg Ala Arg Gly Ser Ile Phe Asn Val Tyr Tyr Val Leu Lys Leu
   35 40 45
   Tyr Phe Ile
   50
<210> 466
   <211> 34
   <212> PRT
   <213> Arabidopsis thaliana
<400> 466
   Met Gly Ser Gly Ala Ser Gly Pro Val Arg Ser Ser Gln Ser Ser Gln
   1 5 10 15
   Ala Gly Gly Arg Phe Asn Asp Ala Asp Pro Ile Ala Ile Asp Tyr Gly
   20 25 30
   Lys Tyr
<210> 467
   <211> 46
   <212> PRT
   <213> Arabidopsis thaliana
<400> 467
   Met Lys Lys Gln Gln Lys Thr Ile Lys Thr Ser Leu Asn Leu Glu Lys
   1 5 10 15
   Leu Lys Ser Lys Arg Asn Leu Ile Phe Ser Val Ile Tyr Gly Asp Arg
   20 25 30
   Ile Ser Ile Ile Lys Thr Ser Thr Ser Leu Ala Thr Leu Arg
   35 40 45
<210> 468
   <211> 11
   <212> PRT
   <213> Arabidopsis thaliana
<400> 468
   Met Lys Thr Asn Lys Asn Thr Lys Tyr Ile Ile
   1 5 10
<210> 469
   <211> 42
   <212> PRT
   <213> Arabidopsis thaliana
<400> 469
   Met Ile Met Cys Gly Leu Thr Leu Arg Val Ala Cys Thr Arg Tyr Arg
   1               5                   10                  15
   Ala Val Ile Asp Cys Tyr His Asn Lys Arg Gly Ile Leu Arg Lys Ser
               20                  25                  30
   Gln Glu Trp Arg Lys Leu Leu Arg Met Ser
           35                  40
<210> 470
   <211> 13
   <212> PRT
   <213> Arabidopsis thaliana
<400> 470
   Met Asp Val Gln Leu Leu His Gln Gln Ser Tyr Ser Ser
   1               5                   10
<210> 471
   <211> 23
   <212> PRT
   <213> Arabidopsis thaiiana
<400> 471
   Met Asn Ala Gly Arg Gly Arg Tyr Thr Phe Leu His Thr Tyr Asp Glu
   1               5                   10                  15
   Tyr Asp Cys Trp Trp Ser Ser
               20
<210> 472
   <211> 12
   <212> PRT
   <213> Arabidopsis thaliana
<400> 472
   Met Glu Lys Val Phe Lys Cys Phe Glu Arg Val Phe
   1               5                   10
<210> 473
   <211> 24
   <212> PRT
   <213> Arabidopsis thaliana
<400> 473
   Met Thr Leu His Asn Val Leu Arg Cys Val Thr Lys Arg Ser Leu Gly
   1               5                   10                  15
   Tyr Gly Ile Phe Asp Ser Asp Ile
   20
<210> 474
   <211> 35
   <212> PRT
   <213> Arabidopsis thaliana
<400> 474
   Met Ala Ser Ser Ile Arg Thr Ser Lys Phe Trp Arg Ile Gly Val Ala
   1 5 10 15
   Phe Lys Glu Gly Asn Gly Glu Arg Ala Thr Leu Tyr Lys Ser Ser Lys
   20 25 30
   Ala Arg Leu
   35
<210> 475
   <211> 73
   <212> PRT
   <213> Arabidopsis thaliana
<400> 475
   Met Ser Lys Asn Arg Met Phe Val Leu Asn Ile Arg Asn Asp Ile Ala
   1 5 10 15
   Gln Cys Leu Lys Met Cys Tyr Lys Glu Glu Ser Trp Leu Trp His Leu
   20 25 30
   Arg Phe Gly His Leu Asn Phe Gly Gly Leu Glu Leu Leu Ser Arg Lys
   35 40 45
   Glu Met Val Arg Gly Leu Pro Cys Ile Asn His Pro Lys Gln Gly Cys
   50 55 60
   Glu Val Val Phe Lys Phe Lys Ala Lys
   65 70
<210> 476
   <211> 20
   <212> PRT
   <213> Arabidopsis thaliana
<400> 476 Gly Val Tyr Glu
<210> 477
   <211> 12
   <212> PRT
   <213> Arabidopsis thaliana
<400> 477
   Met Lys Val Val Phe Leu Lys Leu Lys Arg Met Val
   1 5 10
<210> 478
   <211> 25
   <212> PRT
   <213> Arabidopsis thaliana
<400> 478
   Met Lys Pro Phe Ser Thr Ile Ser His Thr Ser Cys Ala Pro Arg Ile
   1 5 10 15
   Ala Phe Ile Arg Arg Leu Gln Leu Ser
   20 25
<210> 479
   <211> 52
   <212> PRT
   <213> Arabidopsis thaliana
<400> 479
   Met Leu Leu Trp Pro Thr Tyr Leu Gln Arg Pro Ser Arg Thr Tyr Leu
   1 5 10 15
   His Leu Ile Leu Asp Arg Leu Asp Arg Glu Leu Ser Cys Leu Ser Phe
   20 25 30
   Leu Ser Leu Ser Asn His Pro Phe Glu Phe Glu Lys Asp Tyr Leu His
   35 40 45
   Ser Pro Val Gln
   50
<210> 480
   <211> 10
   <212> PRT
   <213> Arabidopsis thaliana
<400> 480
   Met Glu Gly Asn Ile Val Thr Arg His Leu
   1 5 10
<210> 481
   <211> 12
   <212> PRT
   <213> Arabidopsis thaliana
<400> 481
   Met Glu Lys Val Leu Lys Cys Phe Glu Arg Val Phe
   1 5 10
<210> 482
   <211> 22
   <212> PRT
   <213> Arabidopsis thaliana
<400> 482
   Met Cys Lys Val Pro Thr Lys Thr Ile Ile Ile Lys Val Ser Phe Phe
   1 5 10 15
   Leu Tyr Lys Lys Ile Lys
   20
<210> 483
   <211> 14
   <212> PRT
   <213> Arabidopsis thaliana
<400> 483
   Met Met Met Val Leu Val Gly Thr Leu His Ile Thr Tyr Leu
   1 5 10
<210> 484
   <211> 24
   <212> PRT
   <213> Arabidopsis thaliana
<400> 484
   Met Pro Ala Pro Ile Ile Pro Ile Asp Leu Cys Leu Leu Leu Ile Val
   1 5 10 15
   Phe Gly Phe Phe Ser Phe Leu Ile
   20
<210> 485
   <211> 76
   <212> PRT
   <213> Arabidopsis thaliana
<400> 485
   Met Lys Leu Lys Thr Thr Leu Leu Tyr Phe Leu Ile Gln Lys Glu Arg
   1               5                   10                  15
   Asn Phe Asp Asp Asp Gly Phe Gly Gly His Phe Thr His Tyr Leu Ser
               20                  25                  30
   Leu Val Cys Leu Val Gly Ile Tyr Leu Tyr Asn Lys Cys Ala Phe Ile
           35                  40                  45
   His Leu Tyr Ser Gly Arg Asp Cys Trp Leu Leu Lys Gln Ile Leu Thr
       50                  55                  60
   Leu Val Thr Ser His Ala Cys Thr His Asn Thr Tyr
   65                  70                  75
<210> 486
   <211> 33
   <212> PRT
   <213> Arabidopsis thaliana
<400> 486
   Met Ile Ser Ser Phe Leu Ile Gly Leu Glu Lys Met Ala Arg Ser Leu
   1               5                   10                  15
   Pro Leu Cys Thr Ser Arg Tyr Arg Ser His Phe Phe Pro Ser Lys Arg
               20                  25                  30
   Asn
<210> 487
   <211> 11
   <212> PRT
   <213> Arabidopsis thaliana
<400> 487
   Met Arg Ser Ile Ala Gly Gly Ala Gln Arg Lys
   1               5                   10
<210> 488
   <211> 14
   <212> PRT
   <213> Arabidopsis thaliana
<400> 488
   Met Asn Thr Ala Pro Asp Thr Leu Pro Gln Arg Val Thr Val
   1               5                   10
<210> 489
   <211> 12
   <212> PRT
   <213> Arabidopsis thaliana
<400> 489
   Met Leu Ser Pro Leu Thr Leu Ser Leu Thr Gln Gln
   1 5 10
<210> 490
   <211> 55
   <212> PRT
   <213> Arabidopsis thaliana
<400> 490
   Met Ser Ser Gly Ser Thr Cys Leu Leu Phe Gly Leu Pro Gly Gly Val
   1 5 10 15
   Ser Leu Val Gly Gly Gly Val Thr Glu Thr Thr Pro Ser Thr Gly Gly
   20 25 30
   Ser Ser Lys Ile Ile Ile Val Ile Val Val Leu Gly Thr Gly Leu Met
   35 40 45
   Asp Ser Ala Tyr Val Lys Arg
   50 55
<210> 491
   <211> 11
   <212> PRT
   <213> Arabidopsis thaliana
<400> 491
   Met Asn Arg Glu Pro Ile Val Asp Leu Leu Gly
   1 5 10
<210> 492
   <211> 10
   <212> PRT
   <213> Arabidopsis thaliana
<400> 492
   Met Thr Thr Val Asn Arg Asp Leu Asp Arg
   1 5 10
<210> 493
   <211> 23
   <212> PRT
   <213> Arabidopsis thaliana
<400> 493
   Met Ile Ser Ile Leu Gln Ser Asn Lys Asn Val Ser Phe Thr Phe Tyr
   1               5                   10                  15
   Leu His Leu Arg Phe Ile Ser
               20
<210> 494
   <211> 13
   <212> PRT
   <213> Arabidopsis thaliana
<400> 494
   Met Ile Leu Phe Leu Phe Ile Phe Leu Val Ala Ser His
   1               5                   10
<210> 495
   <211> 35
   <212> PRT
   <213> Arabidopsis thaliana
<400> 495
   Met Phe Pro Leu Pro Phe Thr Ser Ile Phe Asp Ser Ser Pro Lys Ala
   1               5                   10                  15
   Thr Phe Pro Leu Thr Asp Ser Ser Ser Ser Ala Asn Ile Leu Phe Leu
               20                  25                  30
   Pro His Met
           35
<210> 496
   <211> 30
   <212> PRT
   <213> Arabidopsis thaliana
<400> 496
   Met Leu Leu Asn Val Lys Leu Leu Ala Thr Lys His Leu Arg Arg Arg
   1               5                   10                  15
   Pro Thr Thr Leu Lys Lys Lys Phe Lys Lys Lys Thr Cys Tyr
               20                  25                  30
<210> 497
   <211> 32
   <212> PRT
   <213> Arabidopsis thaliana
<400> 497
   Met Asn Lys Lys Arg Ile Ile Ser Gly Thr Ser Ile Val Val Gln Val
   1               5                   10                  15
   Ile Thr Cys Ala Gly Glu Lys Val Cys Ser Arg Ser Leu Met Asn Arg
               20                  25                  30
<211> 34
   <212> PRT
   <213> Arabidopsis thaliana
<400> 498
<210> 499
   <211> 39
   <212> PRT
   <213> Arabidopsis thaliana
<400> 499
   Met Asn His His Leu Gln Leu Arg Gly Glu Val Ile Leu Val Glu Val
   1               5                   10                  15
   Val Val Leu Gln Gly Glu Glu Asp Ala Val Val Val Val Gln Cys Pro
               20                  25                  30
   Leu His Met Phe Arg Val Gln
           35
<210> 500
   <211> 28
   <212> PRT
   <213> Arabidopsis thaliana
<400> 500
   Met Gly Asp Leu Thr Glu Ser Pro Arg Val Gly Ser Leu Phe Ser Phe
   1               5                   10                  15
   Phe Asp Phe Cys Pro Leu Phe Phe Val Lys Asn Val
               20                  25
<210> 501
   <211> 16
   <212> PRT
   <213> Arabidopsis thaliana
<400> 501
   Met Arg Ser Leu Ala Ser Leu Ser Lys Ser Asn Arg Val Ile His Ser
   1               5                   10                  15
<210> 502
   <211> 32
   <212> PRT
   <213> Arabidopsis thaliana
<400> 502
   Met Lys Glu Ile Asn Gln Thr Lys Met Ser Phe Ser Ser Ile Tyr Thr
   1               5                   10                  15
   Phe Leu Tyr Trp Gly Val Met Pro Leu Phe Ala Ser Ser Leu Asp Phe
               20                  25                  30
<210> 503
   <211> 16
   <212> PRT
   <213> Arabidopsis thaliana
<400> 503
   Met Pro Cys Ser Val Arg Asn Val Arg Ser Ile Phe Leu Pro Val Phe
   1               5                   10                  15
<210> 504
   <211> 18
   <212> PRT
   <213> Arabidopsis thaliana
<400> 504
   Met Ser Asp Gln Ser Phe Phe Gln Phe Phe Lys Trp Ile Tyr Gln Val
   1               5                   10                  15
   Phe Ile
<210> 505
   <211> 12
   <212> PRT
   <213> Arabidopsis thaliana
<400> 505
   Met Gln Cys Met Pro Cys Gly His His His Leu Ser
   1               5                   10
<210> 506
   <211> 14
   <212> PRT
   <213> Arabidopsis thaliana
<400> 506
   Met Trp Lys Ile Arg Arg Leu Trp Lys Thr Lys Arg Ala Ile
   1 5 10
<210> 507
   <211> 21
   <212> PRT
   <213> Arabidopsis thaliana
<400> 507
   Met Asn Leu Val Tyr Ser Phe Gly Ser Ser Val Leu Lys Asp Ser Ala
   1 5 10 15
   Tyr Val Leu Glu Met
   20
<210> 508
   <211> 17
   <212> PRT
   <213> Arabidopsis thaliana
<400> 508
   Met Met Ser Thr Arg His Ala Leu His Ser Ser Arg Thr Asp Leu Leu
   1 5 10 15
   Thr
<210> 509
   <211> 42
   <212> PRT
   <213> Arabidopsis thaliana
<400> 509
   Met Phe Tyr Ile Ala Leu Leu Val Phe Gln Ser Leu Arg Ile Phe His
   1 5 10 15
   Met Ser Glu Thr Cys Leu Leu Leu Pro Met Val Arg Ile Arg Pro Ala
   20 25 30
   Phe Ala Leu Glu Pro Ser Ala Ile Gly Pro
   35 40
<210> 510
   <211> 10
   <212> PRT
   <213> Arabidopsis thaliana
<400> 510
   Met His Cys Ile Gln Val Ala Gln Ile Cys
   1 5 10
<210> 511
   <211> 12
   <212> PRT
   <213> Arabidopsis thaliana
<400> 511
   Met Glu Thr Ile Ser Thr Phe Phe Thr Asp Leu Gln
   1 5 10
<210> 512
   <211> 18
   <212> PRT
   <213> Arabidopsis thaliana
<400> 512
   Met Val Thr Lys Lys Lys Phe Leu His Glu Ile Tyr His Trp Tyr Trp
   1 5 10 15
   Glu Glu
<210> 513
   <211> 40
   <212> PRT
   <213> Arabidopsis thaliana
<400> 513
   Met Glu Asp Lys Leu Tyr Phe Asp Leu Phe Ile Phe Asp Pro Leu Ala
   1 5 10 15
   Gln Met Phe Val Leu Ser Arg Phe Asp Glu Leu Pro Phe Val Ile Thr
   20 25 30
   Trp Ile Met Ile Asp Leu Glu Met
   35 40
<210> 514
   <211> 44
   <212> PRT
   <213> Arabidopsis thaliana
<400> 514
   Met Ser Ser Ile Leu Asn Ser Lys Arg Pro Lys Phe Tyr Cys Lys Ser
   1 5 10 15
   Val Lys Asn Val Glu Ile Val Ser Ile Phe Thr Pro Pro Asn Thr Asn
   20 25 30
   Gly Lys Phe Arg Glu Glu Ile Ser Phe Leu Ser Pro
   35 40
<210> 515
   <211> 91
   <212> PRT
   <213> Arabidopsis thaliana
<400> 515
   Met Leu Arg Ser Phe Pro Phe Ser Leu Leu Pro Ile Pro Met Val Asn
   1 5 10 15
   Phe Val Lys Lys Phe Leu Phe Cys His His Asn Thr Ser Lys Pro Ser
   20 25 30
   Pro Arg Leu Phe Leu Cys Phe Gly His Ile Tyr Ile Ser Lys Ser Ile
   35 40 45
   Met Ile His Val Met Thr Asn Gly Asn Ser Ser Asn Arg Asp Ser Thr
   50 55 60
   Asn Ile Cys Ala Arg Gly Ser Asn Ile Asn Lys Ser Lys Tyr Ser Leu
   65 70 75 80
   Ser Ser Ile Ser Lys Ala Cys Glu Arg Ile Pro
   85 90
<210> 516
   <211> 132
   <212> PRT
   <213> Arabidopsis thaliana
<400> 516
   Met Val Met Ile Phe Ser Ser Lys Met Cys Leu Val Asp His His His
   1 5 10 15
   Gln Ser Thr Asp Leu Leu Leu Val Ile His Val Leu His Leu Leu Leu
   20 25 30
   Leu Leu Thr Met Met Leu Cys Ser Arg Asn Pro Asn Pro Asn Gln Arg
   35 40 45
   Arg Leu Cys Arg Phe Met Thr Ser Leu Cys Met Met Met Arg Met Cys
   50 55 60
   Leu Ser Leu Phe Leu Ser Ser Lys Ser Leu Pro Leu Leu Leu Ser Gln
   65 70 75 80
   Leu Gly Leu Arg Met Phe Ser Leu Leu Ser His Leu Leu Leu Arg Ser
   85 90 95
   Thr Gly Asn Arg Thr Val Leu Leu Ser Met Ile Ser Trp Glu Thr Ile
   100 105 110
   Trp Gly Arg Lys Gly Gln Ile Val Thr Glu Arg Arg Arg Val Val Pro
   115 120 125
   Phe Leu Met Ile
   130
<210> 517
   <211> 18
   <212> PRT
   <213> Arabidopsis thaliana
<400> 517
   Met Glu Asn Val Asn His Gln Lys Lys Ile Arg Thr Leu Val Val Val
   1 5 10 15
   Val His
<210> 518
   <211> 50
   <212> PRT
   <213> Arabidopsis thaliana
<400> 518
   Met Glu Leu Pro Phe Ser Ser Arg Ser Leu Ser Ala Pro Phe Phe Pro
   1 5 10 15
   Lys Leu Phe Pro Met Arg Ser Ser Lys Gly Glu Leu Phe Cys Phe Leu
   20 25 30
   Cys Phe Val Gly Glu Asp Glu Ile Glu Glu Lys Thr Phe Ser Asn Leu
   35 40 45
   Ala Asp
   50
<210> 519
   <211> 39
   <212> PRT
   <213> Arabidopsis thaliana
<400> 519
   Met Cys Glu Arg Thr Arg Thr Lys Gln Lys Lys His Leu Pro Thr Ser
   1 5 10 15
   Trp His Asp Gly Glu Ile Phe Gly Ser Ser Phe Leu Gly Asn Asn Glu
   20 25 30
   Asp Leu Asp Ser Pro Cys Ile
   35
<210> 520
   <211> 74
   <212> PRT
   <213> Arabidopsis thaliana
<400> 520
   Met Thr Glu Arg Phe Leu Ala Ala Ala Ser Ser Gly Ile Thr Lys Ile
   1 5 10 15
   Ser Thr Val Pro Ala Ser Glu Glu Lys Leu Thr Ile Arg Met Lys Ser
   20 25 30
   Leu Ser Leu Glu Met Val Ile Arg Val Phe Leu Phe Pro Gly Arg Lys
   35 40 45
   Tyr Leu Arg Val Ile Ala Ser Lys Pro Asn Pro Lys Phe Ser Ala Ser
   50 55 60
   Ser Asp Leu Lys Ser Val Ile Glu Val Phe
   65 70
<210> 521
   <211> 25
   <212> PRT
   <213> Arabidopsis thaliana
<400> 521
   Met Ser Ile Ala Lys Ala Ser Pro Pro Glu Tyr Asp Asn Asp Pro Ser
   1               5                   10                  15
   Leu Ile Val Thr Asn Ser Pro Pro Asp
               20                  25
<210> 522
   <211> 21
   <212> PRT
   <213> Arabidopsis thaliana
<400> 522
   Met Asp Arg Cys Arg Ile Leu Ala Ala Met Leu Leu Gln Leu Thr Leu
   1               5                   10                  15
   Ile Arg Ile Leu Gln
               20
<210> 523
   <211> 25
   <212> PRT
   <213> Arabidopsis thaliana
<400> 523
   Met Ser Val Arg Arg Gly Val Cys Tyr Tyr Gln Arg Trp Ile Val Val
   1               5                   10                  15
   Val Phe Trp Arg Arg Cys Phe Cys Asn
               20                  25
<210> 524
   <211> 116
   <212> PRT
   <213> Arabidopsis thaliana
<400> 524
   Met Thr Thr Asn Lys Val Ile Ala Ile Cys Gln Ser Gly Gly Glu Phe
   1               5                   10                  15
   Val Thr Ile Lys Asp Gly Ser Leu Ser Tyr Ser Gly Gly Asp Ala Phe
               20                  25                  30
   Ala Ile Asp Ile Asp Gln Asn Thr Ser Met Thr Asp Phe Lys Ser Glu
           35                  40                  45
   Leu Ala Glu Asn Phe Gly Phe Gly Leu Glu Ala Met Thr Leu Lys Tyr
       50                  55                  60
   Phe Leu Pro Gly Asn Lys Lys Thr Leu Ile Thr Ile Ser Lys Asp Lys
   65                  70                  75                  80
   Asp Phe Ile Arg Met Val Asn Phe Ser Ser Asp Ala Gly Thr Val Glu
                   85                  90                  95
   Ile Phe Val Ile Pro Glu Glu Ala Ala Ala Lys Asn Leu Ser Val Met
               100                 105                 110
   Pro Ala Ser Arg
           115
<210> 525
   <211> 20
   <212> PRT
   <213> Arabidopsis thaliana
<400> 525
   Met Val Ser Lys Asp Gln Gln Met Leu Phe Lys Glu Val Glu Asn Tyr
   1               5                   10                  15
   Ile Cys Leu Thr
   20
<210> 526
   <211> 13
   <212> PRT
   <213> Arabidopsis thaliana
<400> 526
   Met Arg Asp Val Asn Arg Leu Gln Leu Trp Gln Thr Pro
   1               5                   10
<210> 527
   <211> 14
   <212> PRT
   <213> Arabidopsis thaliana
<400> 527
   Met Phe Glu Leu Met Ser Ser Ile Tyr Ser Ser Pro Pro Pro
   1 5 10
<210> 528
   <211> 20
   <212> PRT
   <213> Arabidopsis thaliana
<400> 528
   Met Leu Ile Asp Cys Asn Ser Gly Arg Leu His Asn His Ser Gly Thr
   1 5 10 15
   Trp Pro Ser Ile
   20
<210> 529
   <211> 20
   <212> PRT
   <213> Arabidopsis thaliana
<400> 529
   Met Asp Pro Gly Ile Cys Leu Asn Leu Cys Gln Ala Tyr Ile Val Leu
   1 5 10 15
   His Leu Leu Glu
   20
<210> 530
   <211> 10
   <212> PRT
   <213> Arabidopsis thaliana
<400> 530
   Met Leu Gly Asp Asp Met Tyr Leu Asp Arg
   1 5 10
<210> 531
   <211> 35
   <212> PRT
   <213> Arabidopsis thaliana
<400> 531
   Met Thr Cys Thr Leu Ile Gly Lys Asn Leu Arg Thr Ser His Gln Val
   1 5 10 15
   Leu Met His Ile Asp Arg Ala Ile Leu Arg Gln Cys Ser Gly Thr Leu
   20 25 30
   Leu Asp Leu
   35
<210> 532
   <211> 11
   <212> PRT
   <213> Arabidopsis thaliana
<400> 532
   Met Ala Asn Leu Arg Ser Lys Ala Lys Pro Ile
   1 5 10
<210> 533
   <211> 96
   <212> PRT
   <213> Arabidopsis thaliana
<400> 533
   Met His Phe Thr Pro Ala Leu Lys Leu Ala Thr Pro Ser Leu Val Pro
   1 5 10 15
   Ser Ser Ser Ile Gly Phe Thr Lys Thr Ala Thr Ala Ser Arg Ala Ile
   20 25 30
   Val Ala Glu Ile Ser Pro Glu Gly Thr Arg Gly Thr Thr Met Glu Arg
   35 40 45
   Lys Val Ser Lys Ala Glu Thr Ala Thr Gly Ser Ser Val Glu Glu Ser
   50 55 60
   Thr Glu Asp Ile Ala Ala Ala Lys Lys Ala Glu Gly Asp Gly Gly Arg
   65 70 75 80
   Asp Ser Glu Val Arg Ile Glu Arg Ile Ala Thr Ala Val Ala Thr Arg
   85 90 95
<210> 534
   <211> 15
   <212> PRT
   <213> Arabidopsis thaliana
<400> 534
   Met Gly Asn Tyr Leu Gly Thr Phe Ile Asn Gly Glu Phe Lys Lys
   1 5 10 15
<210> 535
   <211> 80
   <212> PRT
   <213> Arabidopsis thaliana
<400> 535
   Met Lys Leu Pro Ser Lys Met Ala Thr Leu Ile Ser Pro Asn Ser Leu
   1               5                   10                  15
   Trp Thr Val Thr Ala Ser Pro Gln Pro Leu Ser Thr Val Ile Ser Ser
               20                  25                  30
   Pro Pro Pro Ser Leu Cys Ala Leu Phe Ser Pro Gln Ser Leu Phe His
           35                  40                  45
   His Leu Leu Gln Leu Ser Ser Gln Leu Leu Tyr Leu Leu Ser Ile Leu
       50                  55                  60
   Gln Leu Lys Ile Pro Trp Pro Phe Leu Leu Cys Leu Leu Ser Phe Pro
   65                  70                  75                  80
<210> 536
   <211> 23
   <212> PRT
   <213> Arabidopsis thaliana
<400> 536
   Met Ile Gly Asn Leu Phe Lys Leu Val Leu Leu Tyr Phe Leu Asn Ser
   1               5                   10                  15
   Pro Leu Ile Asn Val Pro Arg
               20
<210> 537
   <211> 18
   <212> PRT
   <213> Arabidopsis thaliana
<400> 537
   Met Asn Ile Met Lys Pro Phe Arg Arg Cys Arg His Ser Ser Cys Cys
   1               5                   10                  15
   Lys Leu
<210> 538
   <211> 13
   <212> PRT
   <213> Arabidopsis thaliana
<400> 538
   Met Pro Pro Leu Glu Leu Leu Gln Val Val Ser Ala Ile
   1 5 10
<210> 539
   <211> 25
   <212> PRT
   <213> Arabidopsis thaliana
<400> 539
   Met Trp Leu Asp Cys Thr Tyr Asn Leu Gln Gln Leu Glu Trp Arg His
   1 5 10 15
   Leu Leu Lys Gly Phe Met Met Phe Met
   20 25
<210> 540
   <211> 16
   <212> PRT
   <213> Arabidopsis thaliana
<400> 540
   Met Glu Asp Asn Ile Leu Gly Ile Cys Val Ser Asn Glu Thr Leu Phe
   1 5 10 15
<210> 541
   <211> 19
   <212> PRT
   <213> Arabidopsis thaliana
<400> 541
   Met Ala Pro Glu Phe Ser Met Asp Pro Ser Arg Leu Leu Val Thr Gly
   1 5 10 15
   Phe Thr Ser
<210> 542
   <211> 46
   <212> PRT
   <213> Arabidopsis thaliana
<400> 542
   Met Leu Ser Ser Met Val Val Pro Val Asn Gly Phe Ser Leu Asn Leu
   1 5 10 15
   Ser Lys Pro Asn Leu Val Val Phe Asn Gly Ser Arg Val Phe Tyr Gly
   20 25 30
   Ser Phe Ser Leu Thr Cys Tyr Arg Phe Tyr Phe Leu Ile Ile
   35 40 45
<210> 543
   <211> 35
   <212> PRT
   <213> Arabidopsis thaliana
<400> 543
   Met Leu Phe Ile Tyr Lys Phe Tyr Trp His Met Gln Ile Tyr Val Glu
   1 5 10 15
   Val Lys Arg Lys Thr Lys Gln Tyr Ala Ile Val Glu Met Ser Phe Leu
   20 25 30
   Gly Asn Gly
   35
<210> 544
   <211> 27
   <212> PRT
   <213> Arabidopsis thaliana
<400> 544
   Met Lys Cys Tyr Leu Tyr Ile Asn Ser Ile Gly Thr Cys Lys Tyr Thr
   1 5 10 15
   Leu Lys Ser Lys Glu Lys Gln Asn Ser Thr Gln
   20 25
<210> 545
   <211> 22
   <212> PRT
   <213> Arabidopsis thaliana
<400> 545
   Met Ala Ser Ile Lys Asn Ile Gly Ala Lys Arg Lys Ile Glu Val Lys
   1 5 10 15
   Ile Phe Cys Leu Met Thr
   20
<210> 546
   <211> 10
   <212> PRT
   <213> Arabidopsis thaliana
<400> 546
   Met Lys Ser Glu Cys Val Tyr Met Met Ser
   1 5 10
<210> 547
   <211> 11
   <212> PRT
   <213> Arabidopsis thaliana
<400> 547
   Met Val Ile Thr Tyr Thr Phe Phe Val Pro Leu
   1               5                   10
<210> 548
   <211> 48
   <212> PRT
   <213> Arabidopsis thaliana
<400> 548
   Met Ala Pro Val Thr Val Ile Lys Thr Ser Cys Ile Arg Ile Leu Ile
   1               5                   10                  15
   Ser Phe Leu Thr Glu Glu Val Thr Ser Phe Ser Phe Phe Ala Ser Ile
               20                  25                  30
   Ala Cys Arg Arg His Ile Gln His Phe Ile Ser Ser Cys Ser Ser Cys
           35                  40                  45
<210> 549
   <211> 10
   <212> PRT
   <213> Arabidopsis thaliana
<400> 549
   Met Tyr Thr His Ser Asp Phe Ile Ser Asn
   1               5                   10
<210> 550
   <211> 57
   <212> PRT
   <213> Arabidopsis thaliana
<400> 550
   Met Ser Leu Glu Asp Pro Leu Lys Ser Pro Asn Lys Gly Glu Lys Ala
   1               5                   10                  15
   Ile Ser Arg Pro Phe Ala Ile Ala Arg Arg Ala Ala Arg Leu Tyr Thr
               20                  25                  30
   Ala Trp Leu Arg Tyr Arg Ser Tyr Val Val Ile Gly Leu Lys Ser Gly
           35                  40                  45
   Ile Pro Ala Ile Gln Ala Tyr Phe Phe
       50                  55
<210> 551
   <211> 12
   <212> PRT
   <213> Arabidopsis thaliana
<400> 551
   Met Ala Gly Ile Pro Leu Leu Arg Pro Ile Thr Thr
   1 5 10
<210> 552
   <211> 18
   <212> PRT
   <213> Arabidopsis thaliana
<400> 552
   Met Thr Ser Leu Ala Phe Ser Arg Ser Arg Ile Glu Thr Pro Gln Val
   1 5 10 15
   Ser Arg
<210> 553
   <211> 18
   <212> PRT
   <213> Arabidopsis thaliana
<400> 553
   Met Phe Leu Thr Ser Ala Leu Gly Thr Leu Val Met Ile Phe Asp Val
   1 5 10 15
   Lys Cys
<210> 554
   <211> 41
   <212> PRT
   <213> Arabidopsis thaliana
<400> 554
   Met Lys Gln Ser Cys Phe Lys Tyr Tyr Leu Met Phe Ser Gly Leu Ile
   1 5 10 15
   Cys Arg Arg Gly Ala Tyr Trp Leu Asn Arg Ser Lys Ser Thr Asp Val
   20 25 30
   Met Thr Lys Leu Trp Cys Tyr Thr Ile
   35 40
<210> 555
   <211> 10
   <212> PRT
   <213> Arabidopsis thaliana
<400> 555
   Met Ser Ser Arg Asn Thr Phe Ile Ser Val
   1 5 10
<210> 556
   <211> 25
   <212> PRT
   <213> Arabidopsis thaliana
<400> 556
   Met Lys Val Phe Leu Glu Asp Met Pro Gln Pro Ser Cys Cys Lys Lys
   1 5 10 15
   Glu Lys Gln Ile Leu Lys Ser Ala Arg
   20 25
<210> 557
   <211> 34
   <212> PRT
   <213> Arabidopsis thaliana
<400> 557
   Met Tyr Cys Lys Gln Gln Glu Glu Lys Lys Thr Thr Leu Arg Glu Met
   1 5 10 15
   Asn Trp Arg Val Asp Val Lys Ser Ser Gln Ala Val Lys Lys Leu Arg
   20 25 30
   Ile Asp
<210> 558
   <211> 103
   <212> PRT
   <213> Arabidopsis thaliana
<400> 558
   Met Thr Ser Ser Ile Lys Ser Lys Met Gly Arg Val Thr Ser Trp Lys
   1 5 10 15
   Lys Ser Ala Thr Ser Thr Thr Thr Arg Val Ala Ile Lys Asp Thr Thr
   20 25 30
   Ser Ser Lys Pro Thr Ile Pro Thr Thr Pro Thr Ala Ala Pro Thr Leu
   35 40 45
   Leu Thr Leu Arg Ile Arg Cys Ile Leu His Ser Asn Asn Lys Val Arg
   50 55 60
   Thr Asn Leu Leu Phe Pro Thr Thr Lys Val Ser Phe Leu Asn Asn Asn
   65 70 75 80
   Ser Arg Glu Ile Thr Ser Asn His His His Leu Gly Cys Thr Ser Ala
   85 90 95
   Lys Pro Arg Ser Tyr Cys Ser
   100
<210> 559
   <211> 11
   <212> PRT
   <213> Arabidopsis thaliana
<400> 559
   Met His Asp Ser Val Ser Arg Ile Leu Val Val
   1 5 10
<210> 560
   <211> 24
   <212> PRT
   <213> Arabidopsis thaliana
<400> 560
   Met Ser Ser Leu Cys Ile Leu Gly Phe Gln Arg Arg Val Asn Leu Arg
   1 5 10 15
   Thr Phe Leu Asn Leu Arg Arg Ala
   20
<210> 561
   <211> 17
   <212> PRT
   <213> Arabidopsis thaliana
<400> 561
   Met Ile Gly Glu Asn Gln Ile Thr Ser Leu Asp Thr Leu Glu His Ile
   1 5 10 15
   Phe
<210> 562
   <211> 13
   <212> PRT
   <213> Arabidopsis thaliana
<400> 562
   Met Leu Tyr Leu Lys Asn Ile Ser Leu Ser Tyr Leu Thr
   1               5                   10
<210> 563
   <211> 32
   <212> PRT
   <213> Arabidopsis thaliana
<400> 563
   Met Ser Ser Val Leu Gln Phe His Phe Tyr Leu Pro Lys Leu Val Arg
   1               5                   10                  15
   Glu Phe Phe Gln Asn Phe Ala Gly Leu Trp Leu Leu Ser Phe Arg Arg
               20                  25                  30
<210> 564
   <211> 22
   <212> PRT
   <213> Arabidopsis thaliana
<400> 564
   Met Cys Ser Arg Val Ser Arg Glu Val Ile Trp Phe Ser Pro Ile Ile
   1               5                   10                  15
   Tyr Cys Tyr Gln Gly Ser
               20
<210> 565
   <211> 33
   <212> PRT
   <213> Arabidopsis thaliana
<400> 565
   Met Glu Cys Ile Pro Gln Asn Ser His Gly Val Pro Leu Ser Leu Leu
   1               5                   10                  15
   Thr Thr Gln Ala Leu His Leu Thr Ile Leu Trp Leu Val Thr Gly Gln
               20                  25                  30
   Thr
<210> 566
   <211> 29
   <212> PRT
   <213> Arabidopsis thaliana
<400> 566
   Met Gly Ser Thr Ser Leu Leu Leu Ala Lys Gly Leu Gly Ile Ser Thr
   1               5                   10                  15
   Arg Thr Arg Ile Leu Glu Thr Ser Thr Leu Leu Thr Gln
               20                  25
<210> 567
   <211> 26
   <212> PRT
   <213> Arabidopsis thaliana
<400> 567
   Met Lys Met Lys Arg Met Ala Phe Cys Ala Lys Asn Ala Ser Val Leu
   1               5                   10                  15
   His His Gln Leu His Thr Gly Val Ile Arg
               20                  25
<210> 568
   <211> 18
   <212> PRT
   <213> Arabidopsis thaliana
<400> 568
   Met Arg Leu Pro Arg Ile Val Arg Leu Lys Glu Ser Phe Cys Trp His
   1               5                   10                  15
   Cys Glu
<210> 569
   <211> 13
   <212> PRT
   <213> Arabidopsis thaliana
<400> 569
   Met Leu Val Ser Cys Ile Thr Asn Ser Thr Leu Val Leu
   1               5                   10
<210> 570
   <211> 12
   <212> PRT
   <213> Arabidopsis thaliana
<400> 570
   Met Gly Leu His Gly Asn Phe Gly Glu Tyr Thr Pro
   1 5 10
<210> 571
   <211> 14
   <212> PRT
   <213> Arabidopsis thaliana
<400> 571
   Met Ala His Arg Leu Cys Ser Ser Leu Leu Gly Gln Gln Gly
   1 5 10
<210> 572
   <211> 25
   <212> PRT
   <213> Arabidopsis thaliana
<400> 572
   Met Gly Ile Leu Gly Asn Thr Leu His Glu Ile Ala Gln Asn Ser Glu
   1 5 10 15
   Ile Glu Gly Glu Leu Leu Leu Ala Leu
   20 25
<210> 573
   <211> 22
   <212> PRT
   <213> Arabidopsis thaliana
<400> 573
   Met Glu Glu Ala Phe Ser Pro Leu His Leu Phe Pro Arg Ile Cys Tyr
   1 5 10 15
   Thr Cys Leu Gln Gln Arg
   20
<210> 574
   <211> 18
   <212> PRT
   <213> Arabidopsis thaliana
<400> 574
   Met Ile Leu Leu Ser Leu Thr Leu Leu Gln Thr Cys Ile Ala Asn Ala
   1 5 10 15
   Gly Lys
<210> 575
   <211> 22
   <212> PRT
   <213> Arabidopsis thaliana
<400> 575
   Met Glu His Thr Asn Ile Ser Phe Gly Lys Thr Arg Val Phe Arg Gly
   1 5 10 15
   Ser Leu Cys Asp Phe Pro
   20
<210> 576
   <211> 30
   <212> DNA
   <213> Arabidopsis thaliana
<400> 576
   atgctatgtg tgcttcaagg tttaagggag 30
<210> 577
   <211> 51
   <212> DNA
   <213> Arabidopsis thaliana
<400> 577
   atgataatta ttaataatga taattattta ttattgtttt ataataataa t 51
<210> 578
   <211> 39
   <212> DNA
   <213> Arabidopsis thaliana
<400> 578
   atgataatta tttattattg ttttataata ataattaat 39
<210> 579
   <211> 33
   <212> DNA
   <213> Arabidopsis thaliana
<400> 579
   atgctcatgt accggatgag aagcggagca aac 33
<210> 580
   <211> 54
   <212> DNA
   <213> Arabidopsis thaliana
<400> 580
   atgggcttaa acgaggatag tgtgtttcgt agcataaagc cttttaaaag ccca 54
<210> 581
   <211> 117
   <212> DNA
   <213> Arabidopsis thaliana
<400> 581
   atgaatgtat tagcaagagc tccacggctc cgtcatcagc tccagaatct gacacaagat    60
   cggagaaaaa ctcaaatgca gatgaagggt caacgagtca gaacgacgtc gctgcag      117
<210> 582
   <211> 63
   <212> DNA
   <213> Arabidopsis thaliana
<400> 582
   atggcgagag aagaaaaaga acaatctgtg tatgatattt acacgtttgc gtctctgcta    60
   ttg                                                                   63

## Description

### FIELD OF THE INVENTION

The present invention relates to methods and materials able to confer an increased tolerance to oxidative stress in host cells and organisms and an increased cell survival in the presence of agents that induce mitochondrial dysfunction and/or oxidative stress. In particular, the present invention provides peptides and nucleic acids encoding said peptides to improve the tolerance of a cell, preferably an eukaryotic cell, to oxidative stress, to confer oxidative stress tolerance to an organism when transfected herein or treated therewith, or to prevent, ameliorate, treat or alleviate oxidative stress related disorders.

### BACKGROUND OF THE INVENTION

Oxidative stress or the induction of reactive oxygen species (ROS), such as e.g. superoxide, hydrogen peroxide and hydroxyl (.OH) free radicals, has been implicated in a variety of phenomena in many biological systems as diverse as the initiation of a fungal infection in plants to the onset or causative factor of apoptosis and various diseases, including cardiovascular disease, atherosclerosis, Parkinson's disease and Alzheimer disease [1-4]. ROS can be derived from endogenous sources via the metabolism of oxygen containing species, such as during aerobic respiration, or from exogenous sources such as toxins and environmental pollutants and stress conditions. ROS are associated with oxidative damage at the cellular level: they react with biological molecules which in its turn can lead to the destruction of cells and cellular components (e.g. mitochondria), causing cells to lose their structure and/or function, thus affecting cell metabolism and catabolism. The dominant role of the mitochondria in the cellular or aerobic respiration process makes them particularly vulnerable to ROS. Oxidative stress and the concomitant ROS generation may thus lead to mitochondrial dysfunction and subsequently trigger apoptosis pathways.

### Oxidative stress related disorders

Oxidative stress is implicated in a variety of disease states, including Alzheimer's disease, Parkinson's disease, inflammatory diseases, neurodegenerative diseases, heart disease, HIV disease, chronic fatigue syndrome, hepatitis, cancer, autoimmune diseases cancer, and aging. For instance, a direct link between exposure to paraquat and the development of Parkinson's disease has been reported [5,6].

More in particular, as also described above, oxidative stress is implicated in mitochondrial dysfunction. Mitochondrial dysfunction has been established to contribute to the pathology of numerous diseases and is suspected in many more. In humans, many muscular and neurological disorders, various forms of cancer, diabetes, obesity, other disorders and ageing are associated with mitochondrial dysfunction (as discussed e.g. in Wallace, 2005, Annu Rev Genet 39, 359-407, Modica-Napolitano, 2004, Mitochondrion 4, 755-62 or Orth, 2001, Am J Med Genet 106, 27-36). A role for loss of mitochondrial function in normal aging has long been suspected. Most hypotheses focus on free radical damage to mitochondrial DNA. Mitochondrial dysfunction **also** plays a central role in the pathogenesis of several inborn errors of metabolism (e.g. Wilson's disease (WD) and inborn errors in respiratory chain complexes) but also in the frequent non-alcoholic fatty liver disease (NAFLD) or non-alcoholic steatohepatitis, which is the hepatic manifestation of the metabolic syndrome, and in other associated disorders as diabetes and obesity. In this respect, there is growing evidence that mitochondrial dysfunction, particularly respiratory chain deficiency, plays an role in the pathophysiology of NAFLD, which is linked to the generation of ROS by the damaged respiratory chain [10].

Wilson's disease (WD) is a rare (1/30-100.000) autosomal recessive inborn error of metabolism caused by mutations in ATP7b, an ATPase that is localized to the trans-Golgi and the mitochondria and essential for copper excretion from hepatocytes. Copper toxicity in WD is thought to arise from a direct effect of copper on mitochondria, ultimately amounting to tissue damage in liver and brain. The known mechanisms by which excess copper directly causes mitochondrial dysfunction and damage, are via (1) a deficiency in the mitochondrial respiratory chain, at the level of the copper-dependent complex IV (cytochrome C oxidase) and generation of ROS; (2) a cross-linking of mitochondrial membranous proteins, resulting in contraction of the mitochondrial membrane; and (3) an increase in sphingomyelinase activity, thereby changing the ceramide content of the membranes (mitochondrial membrane and cell membrane), leading to a pro-apoptotic phenotype. WD or mitochondrial copper toxicity in this context can therefore be considered as a model for inherited (1/5000 in the population) and acquired mitochondrial dysfunction disorders in general (e.g. non-alcoholic fatty liver disease and the metabolic syndrome). There hence remains a need for treatments of mitochondrial dysfunction related disorders, particularly by targeting the generation and accumulation of ROS.

### Oxidative stress in plants

Plants have developed a sophisticated regulatory system which involves both production and scavenging of ROS in cells. During normal growth and development, this pathway monitors the level of ROS produced by metabolism and controls the expression and activity of ROS scavenging pathways, which include enzymatic and non-enzymatic components (antioxidants). In general, plant development and yield depend on the ability of the plant to manage oxidative stress, whether it is via signaling or scavenging pathways. Consequently, improvements in a plant's ability to withstand oxidative stress, or to obtain a higher degree of cross-tolerance once oxidative stress has been experienced, has significant value in agriculture.

### Improving tolerance against oxidative stress

It is clear that an increased tolerance to oxidative stress in host cells and organisms is advantageous for many biological systems. Interestingly, overexpression of a peptide that was initially purified from the secretions of various oxidatively stressed cells from human neural cell lines increases their tolerance for oxidative stress [7]. Similarly, *Arabidopsis thaliana* peptides LEA5 and AtMtATP6 both lead to increased oxidative stress tolerance when overexpressed in yeast [8, 9]. The late abundance protein LEA5 (At4g02380) is a 97 amino acids peptide that was isolated by screening an *A. thaliana* cDNA library for clones which enabled growth of the yeast *Δyap1* mutant on toxic concentrations of H₂O₂. The 55 amino acids peptide AtMtATP6 (At3g46430) is a component of the mitochondrial F₁F₀-ATPase an enzyme that is involved in oxidative respiration. In *A. thaliana* suspension cultured cells, AtMtATP6 expression was induced upon osmotic, cold and oxidative stress, the latter caused by H₂O₂. Expression of AtMtATP6 improved the tolerance of wild-type yeast to various abiotic stresses like H₂O₂ and paraquat.

Based on these findings, the identification and characterization of novel Oxidative Stress Induced Peptides (OSIPs) could reveal novel pathways involved in governing tolerance/resistance of organisms against oxidative stress and can be used in novel strategies to protect an organism against (oxidative) stress and/or to improve the stress tolerance of said organism. Such knowledge on anti-oxidative stress peptides can have broad implications for understanding and treatment of diseases that are characterized by the induction of ROS, in particular mitochondrial dysfunction disorders, or can be used for improving stress tolerance in crops.

### SUMMARY OF THE INVENTION

The present invention relates to the identification of 176 oxidative stress induced peptides (OSIPs) encoding regions (Table 2), which correspond to 575 OSIP peptides in the different reading frames (Table 1, SEQ ID No. 1 to SEQ ID No. 575), which may be useful as agents to increase oxidative stress tolerance in cells or organisms and to increase cell survival in the presence of substances that induce mitochondrial dysfunction and/or oxidative stress. Preferred OSIP peptides that confer oxidative stress tolerance and resistance to a cell or organism comprise peptides with amino acid sequence selected from SEQ ID No. 1 to SEQ ID No. 7. Thus, the present invention relates to compounds of a peptidic nature possessing pharmacological or biotherapeutic activity, and to the nucleic acids encoding said peptides, which can be used for the treatment and the prophylaxis of a wide range of oxidative stress-induced pathologies in mammals, including humans and/or as oxidative stress tolerance increasing agents in a wide variety of cells and organisms, including yeast, fungi and plants.

A first embodiment of the present invention provides an isolated peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6 or SEQ ID No. 7, more preferably to amino acid sequence SEQ ID No. 1, wherein the isolated peptide increases the oxidative stress tolerance of a cell or organism under oxidative stress conditions, or stated differently, wherein the isolated peptide increases viability of a cell or organism under oxidative stress conditions and/or in the presence of substances that induce mitochondrial dysfunction.

Another embodiment of the present invention relates to an isolated nucleic acid encoding for a peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6 or SEQ ID No. 7, more preferably to amino acid sequence SEQ ID No. 1, wherein the, isolated peptide increases the oxidative stress tolerance of a cell or organism under oxidative stress conditions. Preferably, said nucleic acid comprises a nucleotide sequence with at least 70% or 80%, more preferably at least 90%, more preferably at least 95%, 97%, 99% or 100% sequence identity to a nucleotide sequence selected from SEQ ID No. 576, SEQ ID No. 577, SEQ ID No. 578, SEQ ID No. 579, SEQ ID No. 580, SEQ ID No. 581 or SEQ ID No. 582, preferably to nucleotide sequence SEQ ID No. 576.

Another embodiment of the invention provides a genetic construct comprising the following operably linked DNA elements: (a) a nucleic acid encoding for a peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6 or SEQ ID No. 7, more preferably to amino acid sequence SEQ ID No. 1, or a nucleic acid comprising a nucleotide sequence with at least 70% or 80%, more preferably at least 90%, more preferably at least 95%, 97%, 99% or 100% sequence identity to a nucleotide sequence selected from SEQ ID No. 576, SEQ ID No. 577, SEQ ID No. 578, SEQ ID No. 579, SEQ ID No. 580, SEQ ID No. 581 or SEQ ID No. 582, respectively, preferably to nucleotide sequence SE SEQ ID No. 576; (b) one or more control sequences capable of driving expression of said nucleic acid, and (c) a 3' end region comprising a transcription termination sequence.

In another embodiment, the present invention provides a method for increasing the oxidative stress tolerance of a cell or organism, preferably an eukaryotic cell or organism, or modulating the oxidative stress level in a cell or organism, preferably an eukaryotic cell or organism, comprising contacting or transfecting said cell or organism with (i) a peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6 or SEQ ID No. 7, more preferably to amino acid sequence SEQ ID No. 1; (ii) a peptidomimetic of (i); (iii) a nucleic acid encoding for (i); or (iv) a nucleic acid comprising a nucleotide sequence with at least 70% or 80%, more preferably at least 90%, more preferably at least 95%, 97%, 99% or 100% sequence identity to a nucleotide sequence selected from SEQ ID No. 576, SEQ ID No. 577, SEQ ID No. 578, SEQ ID No. 579, SEQ ID No. 580, SEQ ID No. 581 or SEQ ID No. 582, respectively, preferably to nucleotide sequence SEQ ID No. 576.

Preferably, said method includes contacting the cell with a peptide of the present invention, a peptidomimetic thereof or a nucleic acid encoding therefo, under conditions effective to increase cell survival in the presence of agents that induce mitochondrial dysfunction and/or oxidative stress (relative to the same cell under the same conditions without the oxidative stress tolerance inducing agent). The cell can be in a cell culture, a tissue, an organ, or an organism. Hence, the method can be carried out in vitro. The cell can be a plant cell, a microbial cell, a fungal cell, a yeast cell, a mammalian cell, or a human cell.

In yet another preferred embodiment, said method for increasing the survival in the presence of oxidative stress-inducing agents or agents that induce mitochondrial dysfunction and/or for increasing the oxidative stress tolerance of a cell or non-human organism, preferably an eukaryotic cell or eukaryotic, non-human organism, comprising introducing and expressing into said cell or non-human organism (i) a nucleic acid encoding for a peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6, or SEQ ID No. 7, more preferably to amino acid sequence SEQ ID No. 1, or (ii) a nucleic acid comprising a nucleotide sequence with at least 70% or 80%, more preferably at least 90%, more preferably at least 95%, 97%, 99% or 100% sequence identity to a nucleotide sequence selected from SEQ ID No. 576, SEQ ID No. 577, SEQ ID No. 578, SEQ ID No. 579, SEQ ID No. 580, SEQ ID No. 581 or SEQ ID No. 582, preferably to nucleotide sequence SEQ ID No. 576. The cell can be a plant cell, a microbial cell, a fungal cell, a yeast cell, a mammalian cell, or a human cell. The cell can be in a cell culture, a tissue, an organ, or any non-human organism. Preferably, said nucleic acid is incorporated in the genetic construct according to another embodiment of the present invention. Preferably, said nucleic acid capable of encoding an oxidative stress tolerance increasing peptide is operably linked to a promoter that drives expression of a coding sequence in said cell. Preferably, said nucleic acid and/or chimeric genetic construct is stably incorporated in the genome of said cell.

Yet another embodiment of the present invention relates to a transgenic cell or non-human organism having an increased survival in oxidative stress conditions and/or having an increased tolerance to oxidative stress relative to the corresponding wild-type cell or non-human organism obtainable by the above method according to another embodiment of the present invention. Thus, the present invention also provides a transgenic cell or non-human organism having increased tolerance to oxidative stress relative to the corresponding wild-type cell or non-human organism wherein said transgenic cell or non-human organism is transfected with and expresses the nucleic acid or the genetic construct according to other embodiments of the present invention. Preferably, said transgenic cell or non-human organism is a plant cell or plant or part thereof, or a microbial eukaryotic cell or organism, such as a fungi/fungal cell or yeast. Thus, the present invention also provides transgenic plants and plant cells having increased levels of tolerance to oxidative stress, nucleic acids according to the present invention to generate transgenic plants and plant cells having increased levels of tolerance to oxidative stress, and methods for making plants and plant cells having increased levels of tolerance to oxidative stress. In another embodiment, the present invention also relates to harvestable parts, including seeds, of said transgenic plant. Yet another embodiment of the present invention relates to a peptide of the present invention comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6, or SEQ ID No. 7, more preferably to amino acid sequence SEQ ID No. 1, for use in the treatment or prevention of a mammal or a human having an oxidative stress related disorder. Preferably, said oxidative stress related disorder is a mitochondrial dysfunction related disorder.

Yet another embodiment of the present invention relates to a pharmaceutical composition comprising (i) a peptide comprising an amino acid sequence with 70% or 80%, more preferably at least 90%, more preferably at least 95%, 97%, 99% or 100% sequence identity to an amino acid sequence selected from SEQ ID No. 1 to SEQ ID No. 575; preferably selected from SEQ ID No. 1, SEQ ID No. 2, SEQ ID No. 3, SEQ ID No. 4, SEQ ID No. 5, SEQ ID No. 6, or SEQ ID No. 7, more preferably to amino acid sequence SEQ ID No. 1, or a peptidomimetic thereof, or a nucleic acid encoding therefor, and (ii) one or more pharmaceutically acceptable compounds, carriers and/or adjuvants.

Thus, the present invention also relates to the use of an oxidative stress tolerance inducing peptide of the present invention for modulating or controlling the oxidative stress level and/or mitochondrial dysfunction in a patient or for the prevention or treatment of oxidative stress related disorders, such as mitochondrial dysfunction related disordes, in an organism. In a particular embodiment, the present invention also relates to the use of said peptides or nucleic acids according to the present invention for the manufacture of a medicament for the prevention and/or treatment of (oxidative) stress related disorders, in particular mitochondrial dysfunction disorders, and to the use of said peptides or nucleic acids for the screening of materials for their therapeutic activity. Said method for modulating or controlling the oxidative stress level and/or mitochondrial dysfunction in a patient or for the prevention or treatment of oxidative stress related disorders in an organism includes administering to the patient or organism a therapeutically effective amount of a peptide of the present invention, a peptidomimetic thereof or a composition comprising said peptide or peptidomimetic, under conditions effective to decrease or prevent an increase in the level of at least one ROS species in the patient, and/or effective to decrease or prevent an increase in the level of mitochondrial dysfunction in the patient (relative to the same conditions without the oxidative stress tolerance inducing agent).

### DETAILED DESCRIPTION

### Legends of the figures

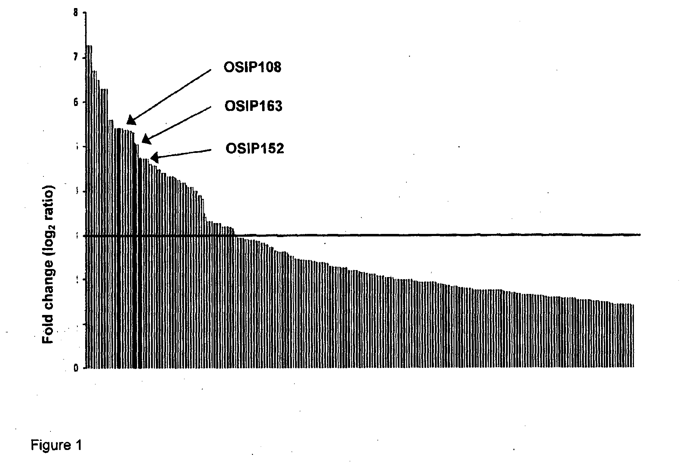

Figure 1 presents the different open reading frames, deduced from the 176 selected OSIP encoding regions, ordered based on their induction level.

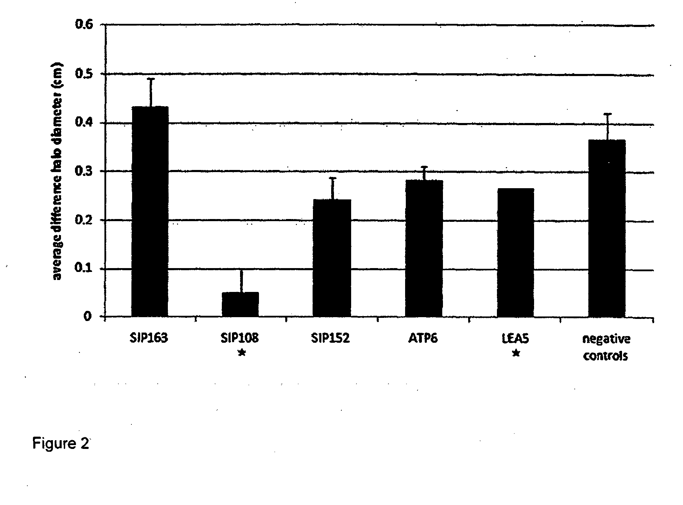

Figure 2 represents the results from the halo-test to assay the oxidative stress tolerance of the yeast in the presence & absence of OSIPs.

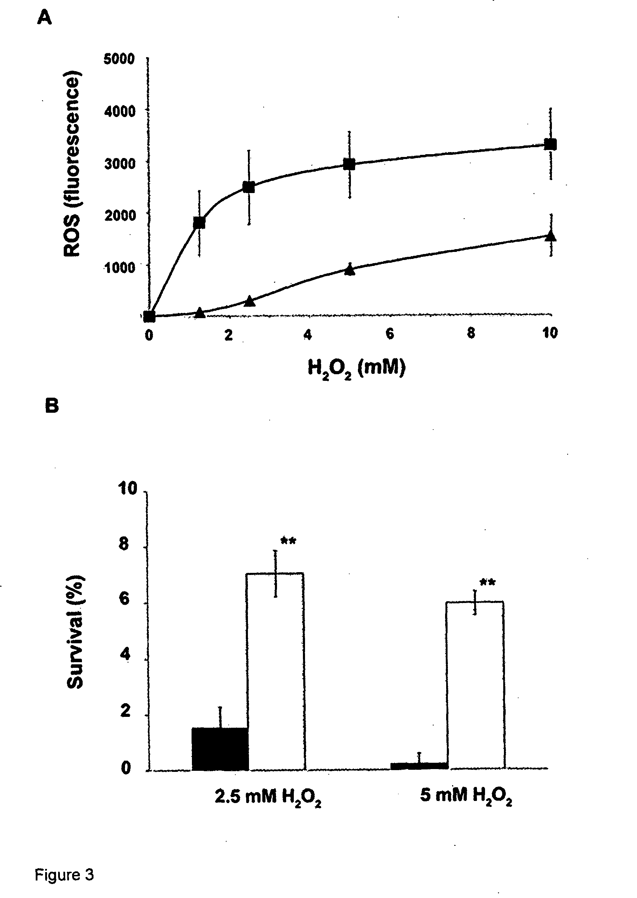

Figure 3 shows the effect of exogenously added OSIP108 on (A) peroxide-induced ROS levels in yeast (square: in the absence of OSIP108, triangle: in the presence of OSIP108) (B) the survival rate of peroxide-treated yeast cells (black bar: in the absence of OSIP108, white bar: in the presence of OSIP108).

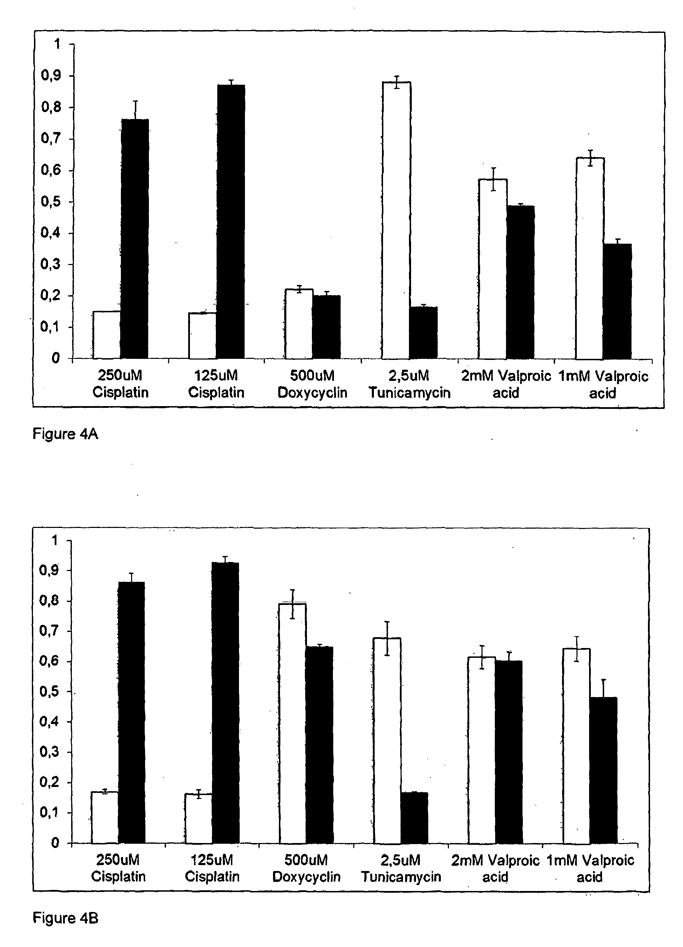

Figure 4 shows the effect of OSIP108 on yeast growth inhibition induced by apoptosis-inducing compounds. Yeast growth was monitored upon treatment with different apoptosis-inducing compounds in the presence (black bars) or absence (white bars) of 200µM OSIP108. Percentage growth was calculated as the ratio of the OD600 of the treated culture over an unstressed yeast culture after 10h (A) or 48h (B) of incubation.

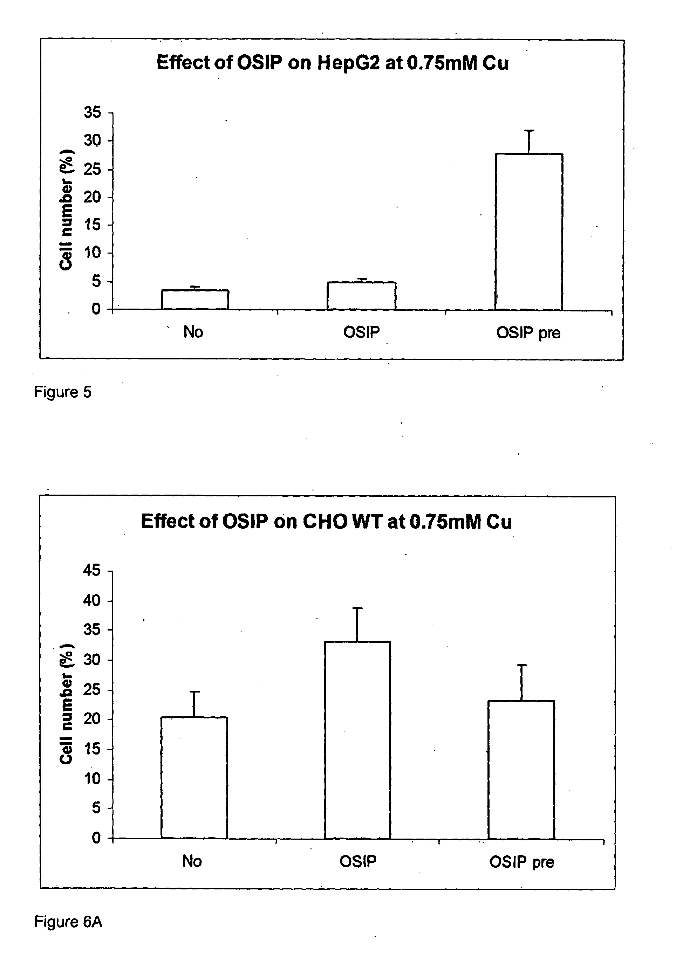

Figure 5 shows that OSIP108 significantly enhances copper resistance of human hepatoma cell line HepG2. The mean and SE of three experiments is shown. No: viability of cells without addition of peptide; OSIP: OSIP108 was added simultaneously added to cells together with Cu; OSIP pre, cells were preincubated with OSIP108 for 12 h prior to addition of Cu.

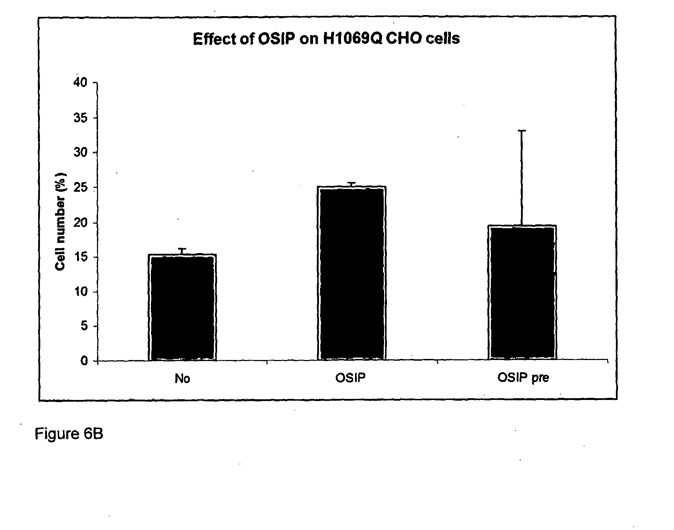

Figure 6 shows that OSIP108 significantly enhances copper resistance of cells expressing wild-type hATP7B (A) and cells expressing the hATP7B H1069Q mutant frequently found in patients having WD (B). No: viability of cells without addition of peptide; OSIP: OSIP108 was added simultaneously added to cells together with Cu; and OSIP pre: cells were preincubated with the peptide for 12 h prior to addition of Cu.

### Description

Tiling array technology was used for the identification of novel - previously unannotated - peptide-encoding genes, particularly for the identification of specific peptide-encoding genes that were upregulated in *A. thaliana* during oxidative stress induced by the herbicide paraquat. This way, 176 potential 'Oxidative Stress Induced Peptides' (OSIPs) encoding regions were identified (listed in Table 2) which correspond to 575 OSIPs in the different open reading frames of said OSIP encoding regions (Table 1). The ability of said peptides to increase the oxidative stress resistance and/or survival in oxidative stress conditions of a host cell or organism, preferably an eukaryotic cell or organism, was evaluated in several biological systems, including overexpression in yeast and exogenous application of an OSIP peptide to yeast and other cell cultures and plants.

The scope of the applicability of the present invention will become apparent from the detailed description and drawings provided below.

Reference throughout this specification to "one embodiment" or "an embodiment" means that a particular feature, structure or characteristic described in connection with the embodiment is included in at least one embodiment of the present invention. Thus, appearances, of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. Where an indefinite or definite article is used when referring to a singular noun e.g. "a" or "an", "the", this includes a plural of that noun unless something else is specifically stated.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects.

The phrase "oxidative stress conditions" as used herein, refers to conditions that results in oxidative stress and elevate the ROS level beyond the normal level, resulting in e.g. destruction of cells and cellular components (e.g. mitochondria), causing cells to lose their structure and/or function, and/or cell death. Particular oxidative stress conditions are those that result in or are related with mitochondrial dysfunction.

As used herein the phrase "oxidative stress" refers to an undesirable imbalance where in general oxidants outnumber antioxidants. This situation can particularly arise if the rate of ROS production overwhelms existing antioxidant defenses. In such circumstances, a series of cellular responses (e.g. mitochondrial dysfunction and the subsequent impaired respiratory chain and cellular respiration) can occur that can lead to an even greater increase in ROS production. Excessive ROS production and its otherwise ineffective regulation can be detrimental to cells and tissues, inducing cellular damage that ultimately can lead to cell death (apoptosis). Oxidative stress-associated damage also can cause undesirable changes to the structural and functional integrities of cells that can lead to the propagation of cells instead of apoptosis. Additionally, oxidatively-damaged cellular macromolecules can trigger immune responses that can lead to disease. See generally, D. G. Lindsay et al. (2002) Mol. Aspects of Med. 23:1-38. In the case of plants, oxidative stress occurs e.g. in situations of ozone stress, in cases of necrosis as a result of pathogen infection or wounding, in cases of senescence and due to application of certain herbicides (like atrazine or paraquat).

"Increased stress tolerance" as used herein comprises, for any given stress, but particularly for oxidative stress, increasing tolerance in a(n) (eukaryotic) cell, tissue, organ or organism to oxidative stress conditions, whether said (eukaryotic) cell, tissue, organ or organism already have some degree of tolerance to the particular stress, such as oxidative stress, or whether said (eukaryotic) cell, tissue, organ or organism is being provided with tolerance to that stress, particularly oxidative stress, anew. Particularly, said increased stress tolerance is in the meaning of increased oxidative stress tolerance, and/or increased viability and cell survival under oxidative stress conditions, but it is understood that due to cross-tolerance said tolerance to oxidative of a cell or organism may go hand in hand with an increased tolerance of a cell or organism to other stress conditions as well, particularly abiotic or environmental stress conditions including metal toxicity, temperature stress (i.e. stress induced by sub-optimal or supra-optimal growth temperatures for a particular cell or organism), osmotic stress (i.e. any stress associated with or induced by loss of water, reduced turgor or reduced water content of a cell, tissue, organ or organism), drought stress (i.e. any stress which is induced by or associated with the deprivation of water or reduced supply of water to a cell, tissue, organ or organism) or salt stress (i.e. any stress which is associated with or induced by elevated concentrations of salt or ions in general and which result in a perturbation in the osmotic potential of the intracellular or extracellular environment of a cell).

The terms "tolerance" and "resistance" as used herein encompass protection against stress ranging from a delay to substantially a complete inhibition of alteration in cellular metabolism, reduced cell growth and/or cell death caused by stress conditions, particularly oxidative stress conditions.

By "isolated" it is meant material that is substantially or essentially free from components that normally accompany it in its native state used or that is essentially free of other cellular materials or culture medium when produced by recombinant techniques, or substantially free of chemical precursors when chemically synthesized. For example, an "isolated poly- or oligonucleotide", as used herein, refers to a poly- or oligonucleotide, which has been purified from the sequences which flank it in a naturally-occurring state.

The terms "oligonucleotide", "polynucleotide" or "nucleic acid" as used herein refers to a polymer composed of a multiplicity of nucleotide units (deoxyribonucleotides or ribonucleotides, or related structural variants or synthetic analogues thereof) linked via phosphodiester bonds (or related structural variants or synthetic analogues thereof). An oligonucleotide is typically rather short in length, generally from about 10 to 30 nucleotides, but these terms can refer to nucleic acid molecules of any length, although the term "polynucleotide" is typically used for large oligonucleotides and typically refers to nucleic acid polymers greater than 30 nucleotides in length. The term "polynucleotide" or "nucleic acid" as used herein designates mRNA, RNA, cRNA, cDNA or DNA.

The term "recombinant nucleic acid" as used herein refers to a nucleic acid polymer formed in vitro by the manipulation of nucleic acid into a form not normally found in nature. For example, the recombinant nucleic acid may be in the form of an expression vector. Generally, such expression vectors include transcriptional and translational regulatory nucleic acid operably linked to the nucleotide sequence.

The terms "peptide" or "polypeptide" refer to a polymer of amino acid residues and to variants and synthetic analogues of the same, encompassing native peptides (including synthetically synthesized or recombinant peptides) and peptidomimetics (typically, synthetically synthesized peptides). Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers. A peptide of the present invention may also be produced by recombinant expression in prokaryotic and eukaryotic engineered cells other than plant cells, such as bacteria, fungi, or animal cells. Suitable expression systems are known to those skilled in the art. By "recombinant (poly)peptide" is meant a (poly)peptide made using recombinant techniques, i.e., through the expression of a recombinant or synthetic polynucleotide. When the polypeptide is recombinantly produced, it is also preferably substantially free of culture medium, i.e., culture medium represents less than about 20%, more preferably less than about 10%, and most preferably less than about 5% of the volume of the peptide preparation. The term "peptide" also refers to modified peptides wherein the modifications render the peptides even more stable e.g. while in a body. Such modifications include, but are not limited to N-terminus modification, C-terminus modification, peptide bond modification, including, but not limited to, CH₂-NH, CH₂-S, CH₂-S=0, 0=C-NH, CH₂-0, CH₂-CH₂, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992).

"Homologues" of a peptide include peptides, oligopeptides, polypeptides or proteins having amino acid substitutions, deletions and/or insertions relative to the unmodified peptide in question and having similar biological and functional activity as the unmodified protein from which they are derived. To produce such homologues, amino acids of the protein may be replaced by other amino acids having similar properties ("conservative substitution") (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company). The homologues useful in the present invention have at least 70% sequence identity or similarity (functional identity) to the unmodified peptide, preferably at least 80% or 90%, more preferably at least 95%, 97%, 99% sequence identity or similarity to an unmodified peptide of the invention. Furthermore, homologues of a peptide according to the present invention are capable of increasing the oxidative stress tolerance or viability of a cell or organism under oxidative stress conditions.

The term "sequence identity" as used herein refers to the extent that sequences are identical on a nucleotide-by-nucleotide basis or an amino acid-by-amino acid basis over a window of comparison. Sequence identity is generally determined by aligning the residues of the two sequences to optimize the number of identical amino acids or nucleotides along the lengths of their sequences; gaps in either or both sequences are permitted in making the alignment in order to optimize the number of identical residues, although the amino acids or nucleotides in each sequence must nonetheless remain in their proper order. Thus, a "percentage of sequence identity" is calculated by comparing two optimally aligned sequences over the window of comparison, determining the number of positions at which the identical nucleic acid base (e.g., A, T, C, G, I, U) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Preferably, sequence identity between two amino acid or two nucleotide sequences is determined by comparing said sequences using the Blastp or Blastn program, respectively, available at http://blast.ncbi.nlm.nih.gov/Blast.cgi. Preferably, the default values for all BLAST 2 search parameters are used, including in the case of Blastp: matrix = BLOSUM62; open gap penalty = 11, extension gap penalty = 1, gap x-dropoff = 50, expect = 10, wordsize = 3, and filter on; and in the case of Blastn: [i.e. "Expect threshold" = 10; "word size" = 11; "Match/mismatch scores" = (2,-3); "Gap costs" = (existence: 5 - extension: 2); filter for low complexity regions & mask for lookup table only). "Similarity" refers to the percentage number of amino acids that are identical or constitute conservative substitutions.

The term "expression cassette" refers to any recombinant expression system for the purpose of expressing a nucleic acid sequence of the invention *in vitro* or *in vivo,* constitutively or inducibly, in any cell, including, in addition to plant cells, prokaryotic, yeast, fungal, insect or mammalian cells. The term includes linear and circular expression systems. The term includes all vectors. The cassettes can remain episomal or integrate into the host cell genome. The expression cassettes can have the ability to self-replicate or not (i.e. drive only transient expression in a cell). The term includes recombinant expression cassettes that contain only the minimum elements needed for transcription of the recombinant nucleic acid.

A "nucleotide sequence encoding a peptide" (i.e. a gene, coding sequence, open reading frame or ORF) is a nucleotide sequence that can be transcribed into mRNA and/or translated into a polypeptide when present in an expressible format, i.e. when the coding sequence or ORF is placed under the control of appropriate control sequences or regulatory sequences. A coding sequence or ORF is bounded by a 5' translation start codon and a 3' translation stop codon. A coding sequence or ORF can include, but is not limited to RNA, mRNA, cDNA, recombinant nucleotide sequences, synthetically manufactured nucleotide sequences or genomic DNA. The coding sequence or ORF can be interrupted by intervening nucleic acids.

### Peptide for increasing the oxidative stress tolerance of a host cell or organism

A first object of the present invention relates to an isolated peptide (also herein referred to as "peptide of the present invention") comprising an amino acid sequence which is at least 70%, more preferably at least 80%, more preferably at least 90%, more preferably at least 95%, 97%, 99% or 100% identical to an amino acid sequence selected from the amino acid sequences listed in Table 1, i.e an amino acid sequence selected from SEQ ID No. 1 to SEQ ID No. 575, wherein said isolated peptide increases the tolerance of a host cell or organism to oxidative stress conditions, or wherein said isolated peptide increases the cell viability under oxidative stress conditions or in the presence of agents inducing mitochondrial dysfunction.

In a preferred embodiment of the present invention, said isolated peptide that increases the tolerance of a host cell or organism to oxidative stress conditions comprise an amino acid sequence which is at least 70% or 80%, more preferably at least 90%, more preferably at least 95%, 97%, 99% or 100% identical to amino acid sequence SEQ ID No. 1; SEQ ID No. 2; SEQ ID No. 3; SEQ ID No. 4; SEQ ID No. 5; SEQ ID No. 6 or SEQ ID No. 7 (Table 3). Most preferably, said isolated peptide that increases the tolerance of a host cell or organism to oxidative stress conditions comprise an amino acid sequence which is at least 70% or 80%, more preferably at least 90%, more preferably at least 95%, 97%, 99% or 100% identical to amino acid sequence SEQ ID No. 1.

**Table 3. List of validated peptides that increase oxidative stress tolerance**

| **CODE** | **AMINO ACID CODE** | **SEQ ID NO** |
|---|---|---|
| (O)SIP108 | MLCVLQGLRE | SEQ ID No. 1 |
| (O)SIP14_1 | MIIINNDNYLLLFYNNN | SEQ ID No. 2 |
| (O)SIP14_2 | MIIIYYCFIIIIN | SEQ ID No. 3 |
| (O)SIP11 | MLMYRMRSGAN | SEQ ID No. 4 |
| (O)SIP163 | MGLNEDSVFRSIKPFKSP | SEQ ID No. 5 |
| (O)SIP152 | MNVLARAPRLRHQLQNLTQDRRKTQMQMKGQRVRTTSLQ | SEQ ID No. 6 |
| (O)SIP37 | MAREEKEQSVYDIYTFASLLL | SEQ ID No. 7 |

A particular embodiment relates to a derivative of a peptide of the present invention, such as but not limited to, an allelic variant, a homologue or a mutation of a peptide of the present invention. Preferably, a derivative, variant, mutation or homologue of a peptide of the present invention has at least the same or better functional activity than the unmodified peptide, such as the capability of increasing the oxidative stress tolerance of a host cell or organism, and/or the capacity to increase the cell viability and cell survival under oxidative stress conditions and/or in the presence of an agent inducing mitochondrial dysfunction. The functional activity can be tested with for example the methods as described in Example 2, 3 and 4, e.g. evaluating the peroxide MIC values or halo formation following overexpression of the modified peptide in yeast, or measuring the ROS accumulation following exogeneous application of the modified peptide to a yeast culture.

The term "derivative(s) of a peptide" refers to peptides or polypeptides which, compared to the amino acid of a naturally-occurring form of the peptide as presented in SEQ ID No. 1 to SEQ ID No. 575, preferably as presented in SEQ ID No. 1, SEQ ID No. 2, SEQ ID No. 3, SEQ ID No. 4, SEQ ID No. 5, SEQ ID No. 6 or SEQ ID No. 7, most preferably as presented in SEQ ID No. 1 may comprise: (i) substitutions, deletions or additions of naturally and non-naturally occurring amino acid residues; (ii) amino acid residues that are substituted by corresponding naturally or non-naturally altered amino acids; (iii) naturally occurring altered, (such as glycosylated, acylated, myristoylated or phosphorylated amino acids) or non-naturally occurring amino acid residues (such as biotinylated amino acids, or amino acids modified after CNBr treatment); (iv) peptides carrying post-translational modifications. A derivative may also comprise one or more non-amino acid substituents compared to the amino acid from which it is derived, for example a reporter molecule or other ligand, covalently or non-covalently bound to the amino acid such as, for example, a reporter molecule which is bound to facilitate its detection. Preferably, amino acid substitutions comprise conservative amino acid substitutions. One or more amino acid residues may be introduced into a predetermined site in said peptide of the present invention. Insertions can comprise amino-terminal and/or carboxy-terminal fusions as well as intra-sequence insertions of single or multiple amino acids. Examples of amino- or carboxy-terminal fusion proteins or peptides include the binding domain or activation domain of a transcriptional activator as used in the yeast two-hybrid system, phage coat proteins, (histidine)₆-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

In a particular embodiment the invention relates to a method for recombinant production of a peptide of the invention, comprising introducing an expression cassette comprising a nucleic acid encoding an oxidative stress tolerance inducing peptide of the invention, introducing said expression cassette in a suitable host cell, culturing the resulting recombinant host under suitable conditions and isolating the oxidative stress tolerance inducing peptide produced. A preferred method involves the synthesis of nucleic acid sequences by PCR and its insertion into said expression vector and subsequently transfecting or transforming a suitable host cell with the expression vector. A large number of suitable methods exist in the art to produce peptides in appropriate hosts. If the host is a unicellular organism such as a prokaryote or a mammalian or insect cell, the person skilled in the art can revert to a variety of culture conditions. To increase the yield and the solubility of the expression product, the medium can be buffered or supplemented with suitable additives known to enhance or facilitate both. In general, the skilled person is also aware that the culture and temperature conditions may have to be adapted to the needs of the host and the requirements of the peptide expressed. In case an inducible promoter controls the nucleic acid of the invention in the vector present in the host cell, expression of the polypeptide can be induced by addition of an appropriate inducing agent. Conveniently, the produced protein is harvested from the culture medium, lysates of the cultured cells or from isolated (biological) membranes by established techniques. Suitable expression protocols and strategies are known to the skilled person and can be retrieved e.g. from Sambrook, 2001. In a particular embodiment when recombinantly producing the peptides of the invention in a host cell, the expression vector may encode a fusion peptide or fusion polypeptide e.g. wherein the peptide of the invention is coupled to a signal peptide to direct expression to a specific compartment or site or to a tag which facilitates purification of the fusion peptide or polypeptide. Suitable tags are well known in the art and comprise e.g. a hexahistidine tag and a GST (glutathione S-transferase) tag. The fusion peptide or fusion polypeptide expressed may be further processed in order to cleave the compensating peptide or polypeptide or the signal peptide or tag fused to the peptide of the invention. This can take place at any stage of the purification process after culturing the host cell. Suitable methods to cleave off the undesired part are either chemical methods using e.g. cyanogen bromide or N-chloro succinimide, or, preferably, enzymatic methods, using e.g. proteases suitable for cleavage specific for a certain amino acid sequence and include Factor Xa or TEV protease.

In another particular embodiment a peptide of the present invention can be produced synthetically. Chemical synthesis of peptides is well known in the art. Solid phase synthesis is commonly used and various commercial synthesizers are available, for example automated synthesizers by Applied Biosystems Inc.; Beckman; MultiSyntech; GenScript, Jena etc. Solution phase synthetic methods, as known by the person skilled in the art, may also be used, although they are less convenient. The peptides are commonly analysed by matrix-associated laser desorption time-of-flight mass spectrometry. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins may be introduced into the molecule during chemical synthesis. In addition, small molecules and label moieties may be attached during the synthesis process. Preferably, introduction of the functional groups and conjugation to other molecules minimally affect the structure and function of the subject peptide.

In specific embodiments the N-and C-terminus of an oxidative stress tolerance inducing peptide of the present invention may be derivatized using conventional chemical synthesis methods. The peptides of the invention may contain an acyl group, such as an acetyl group. Methods for acylating, and specifically for acetylating the free amino group at the N-terminus are well known in the art. For the C-terminus, the carboxyl group may be modified by esterification with alcohols or amidated to form -CONH₂ or CONHR. Methods of esterification and amidation are well known in the art.

Furthermore, an oxidative stress tolerance inducing peptide of the present invention may also be produced semi-synthetically, for example by a combination of recombinant and synthetic production. In the case that fragments of the peptides are produced synthetically, the remaining part of the peptide would have to be produced otherwise, e.g. recombinantly as described further below, and then be linked to the fragment to form the peptide of the invention.

Furthermore, the invention encompasses peptidomimetics of the peptide as defined above. A peptidomimetic is a small protein- or peptide-like chain designed to mimic a peptide. Peptidomimetics typically arise from modifications of an existing peptide in order to alter the properties of the peptide. For example, they may arise from modifications to change the stability of the peptide. These modifications involve changes to the peptide that will not occur naturally (such as altered backbones and the incorporation of non-natural amino acids), including the replacement of amino acids or peptide bonds by functional analogues. Such functional analogues include all known amino acids other than the 20 gene-encoded amino acids, such as for example selenocysteine. The use of peptidomimetics as compared to other mimetics has some particular advantages, including (i) their conformationally restrained structure allows to minimize binding to non-target compounds and to enhance the activity at the desired targets; (ii) through the addition of hydrophobic residues and/or replacement of amide bonds the transport of peptidomimetics through cellular membranes can be improved; (iii) peptidomimetics (e.g. cyclic peptides) are less susceptible to degradation by peptidases and other enzymes.

Yet another alternative method for producing the peptide of the invention is *in vitro* translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant peptides upon the addition of cloning vectors, DNA fragments, or RNA sequences containing coding regions and appropriate promoter elements.

Methods of isolation of the peptide produced are well-known in the art and comprise, without limitation, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation (see, for example, Sambrook, 2001).

### Nucleic acid encoding for a peptide that increases the oxidative stress tolerance of a host cell or organism

Another embodiment of the present invention provides a nucleic acid that can be used to confer oxidative stress tolerance or resistance to a host cell or organism, and/or to confer increased cell viability under oxidative stress conditions or in the present of agents that induce mitochondrial dysfunction. The present invention therefore also provides an isolated nucleic acid encoding a peptide of the present invention as defined above, the complement thereof or a part thereof.

In a particular embodiment the invention provides a nucleotide sequence (also referred to as "nucleic acid of the (present) invention") capable of encoding an oxidative stress tolerance increasing peptide comprising an amino acid sequence selected from SEQ ID No. 1 to SEQ ID No. 575, or a nucleotide sequence encoding a homologue having at least 70% or 80%, more preferably at least 90%, more preferably at least 95%, 97%, 99% or 100% identity with an amino acid sequence selected from SEQ ID No. 1 to SEQ ID No. 575. Preferably, said nucleotide sequence according to the present invention is capable of encoding an oxidative stress tolerance increasing peptide comprising an amino acid sequence selected from SEQ ID No. 1 (e.g. nucleotide sequence SEQ ID No. 576); SEQ ID No. 2 (e.g. nucleotide sequence SEQ ID No. 577); SEQ ID No. 3 (e.g. nucleotide sequence SEQ ID No. 578); SEQ ID No. 4 (e.g. nucleotide sequence SEQ ID No. 579); SEQ ID No. 5 (e.g. nucleotide sequence SEQ ID No. 580); SEQ ID No. 6 (e.g. nucleotide sequence SEQ ID No. 581) and/or SEQ ID No. 7 (e.g. nucleotide sequence SEQ ID No. 582), or a nucleotide sequence encoding a homologue having at least 70% or 80%, more preferably at least 90%, more preferably at least 95%, 97%, 99% or 100% identity with an amino acid sequence selected from SEQ ID No. 1; SEQ ID No. 2; SEQ ID No. 3; SEQ ID No. 4; SEQ ID No. 5; SEQ ID No. 6 and SEQ ID No. 7. Most preferably, said nucleotide sequence according to the present invention is capable of encoding an oxidative stress tolerance increasing peptide comprising amino acid sequence SEQ ID No. 1, or a nucleotide sequence encoding a homologue having at least 70% or 80%, more preferably at least 90%, more preferably at least 95%, 97%, 99% or 100% identity with amino acid sequence SEQ ID No. 1.

Preferably, said nucleotide sequence according to the present invention has at least 70% or 80%, more preferably at least 90%, more preferably at least 95%, 97%, 99% or 100% identity with a nucleotide sequence selected from SEQ ID No. 576, SEQ ID No. 577, SEQ ID No. 578, SEQ ID No. 579, SEQ ID No. 580, SEQ ID No. 581 and SEQ ID No. 582 (Table 4), or a complement thereof. More preferably, said nucleotide sequence of the present invention has at least 70% or 80%, more preferably at least 90%, more preferably at least 95%, 97%, 99% or 100% identity with a nucleotide sequence selected from SEQ ID No. 576, or a complement thereof.

Advantageously, the nucleic acids according to the invention may be produced using recombinant or synthetic means, such as, for example, PCR cloning mechanisms. Generally, such techniques as defined herein are well known in the art, for example as described in Sambrook et al. (Molecular Cloning: a Laboratory Manual, 2001). Polynucleotides may also be synthesized by well-known techniques as described in the technical literature [see e.g. 15, 16]. Double stranded DNA fragments may then be obtained either by synthesizing the complementary strand and annealing the strands together under appropriate conditions, or by adding the complementary strand using DNA polymerase with an appropriate primer sequence.

**Table 4. List of nucleic acids encoding for validated oxidative stress tolerance increasing peptides**

| **CODE** | **AMINO ACID CODE** | **SEQ ID NO** |
|---|---|---|
| (O)SIP108 | atgctatgtgtgcttcaaggtttaagggag | SEQ ID No. 576 |
| (O)SIP14_1 | atgataattattaataatgataattatttattattgttttataataataat | SEQ ID No. 577 |
| (O)SIP14_2 | atgataattatttattattgttttataataataattaat | SEQ ID No. 578 |
| (O)SIP11 | atgctcatgtaccggatgagaagcggagcaaac | SEQ ID No. 579 |
| (O)SIP163 | atgggcttaaacgaggatagtgtgtttcgtagcataaagccttttaaaagccca | SEQ ID No. 580 |
| (0)SIP152 | | SEQ ID No. 581 |
| (O)SIP37 | | SEQ ID No. 582 |

### Use of the oxidative stress tolerance inducing agent of the invention

The present invention further relates to the use of a peptide or peptidomimetic of the invention or a nucleic acid encoding for said peptide of the present invention, or a homologue or derivative for increasing the oxidative stress tolerance of a host cell and/or for increasing the viability or survivability of a cell under oxidative stress conditions or in the presence of an agent inducing mitochondrial dysfunction. In an embodiment the present invention relates to the use of a peptide or peptidomimetic of the invention or a nucleic acid encoding for said peptide of the present invention, or a homologue or derivative thereof for the prevention or treatment of oxidative stress related disorders (including but not limited to mitochondrial dysfunction disorders) or oxidative stress related cellular (mitochondrial) damage in an organism, such as a microbial organism or a plant or mammal, such as a human or to improve the oxidative stress tolerance or resistance of a host cell or an organism, such as a microbial organism or a plant or mammal. Alternatively, the present invention relates to a peptide or peptidomimetic of the present invention or derivative thereof or a nucleic acid encoding therefor for use as an oxidative stress tolerance inducing agent. An "oxidative stress tolerance inducing agent" is in the meaning that said peptide, peptidomimetic or nucleic acid of the present invention (or a derivative thereof) is able to (i) increase, enhance or improve the oxidative stress tolerance of a host cell or an organism, preferably an eukaryotic cell or organism (mammal, plant or microbial organism); and/or (ii) increase the viability of a host cell in oxidative stress conditions or in the presence of an agent inducing mitochondrial dysfunction; and/or (iii) protect a host cell or organism against oxidative stress; (iv) and/or be used for treating, ameliorating and/or preventing of oxidative stress related disorders, particularly mitochondrial dysfunction disorders, in an organism, such as a mammal, human or plant. It is understood that the terms "tolerance" and "resistance" are used interchangeably herein. Said oxidative stress tolerance inducing agent may effectively decrease or prevent an increase in the ROS levels in the cell. However, not all individual ROS species monitored would necessarily have to demonstrate a decrease or lack of an increase in its level. Particularly, said oxidative stress tolerance inducing agent may effectively increase or prevent a decrease in viability or survivability of a cell in oxidative stress conditions and/or in the presence of an agent inducing mitochondrial dysfunction. It is understood that due to cross-tolerance said oxidative stress tolerance inducing agent may increase the tolerance of a cell or organism to other abiotic or environmental stress conditions as well.

A particular embodiment of the present invention relates to a method for increasing or inducing the oxidative stress tolerance of a cell or non-human organism, preferably an eukaryotic cell, such as a fungal cell, yeast or a plant cell, comprising introducing into said cell a chimeric genetic construct comprising a nucleic acid capable of encoding an oxidative stress tolerance increasing peptide of the present invention. Preferably, said nucleic acid capable of encoding an oxidative stress tolerance increasing peptide is operably linked to a promoter that drives expression of a coding sequence in said cell. Preferably, said chimeric genetic construct is stably incorporated in the genome of said cell.

Another particular embodiment of the present invention relates to a method for inducing or increasing the oxidative stress tolerance or resistance of a cell or increasing the viability of a cell under oxidative stress conditions, preferably an eukaryotic cell, by contacting said cell with an oxidative stress tolerance inducing agent according to the present invention, preferably contacting said cell with a peptide or peptidomimetic of the invention or a derivative thereof. The cell can be a microbial cell, such as yeast or a fungal cell, a plant cell, a mammal or human cell, in a cell culture, a tissue, an organ, or an organism. Hence, this method can be carried out *in vivo* or *in vitro.*

Yet another embodiment of the present invention relates to a peptide of the present invention or a nucleic acid encoding therefor for use in preventing or treating an oxidative stress-related disorder, preferably a mitochondrial dysfunction disorder. Thus, the present invention provides a method of treating an oxidative stress-related disorder in a subject in need thereof, the method comprising administering to the subject a therapeutically effective amount of the pharmaceutical composition of the present invention, thereby treating the oxidative stress-related disorder.

In another particular embodiment of the present invention a method for affecting or increasing the lifespan of one or more (eukaryotic) cells is provided, comprising the use of a peptide of the present invention, a peptidomimetic thereof or a nucleic acid encoding therefor. The eukaryotic cell can be a cultured cell, such as a tissue cell culture, or the cell can be one of many cells in, for example, a tissue, or an organ, or a biological system, such as a mammal or a human. Thus, the lifespan of a tissue or an organ, for example, a tissue or an organ that is intended for transplantation, can be extended (relative to a similar cell, tissue, organ or biological system that does not receive a peptide or composition of the invention) using the method of the invention. Lifespan can include the number of times a cell or cell population can divide (replicative lifespan), or the length of time a cell or organism survives before dying (chronological lifespan).

### Increasing oxidative stress tolerance of eukaryotic cells & organisms

A particular embodiment of the present invention encompasses a genetic modification of a host cell or non-human organism, preferably an eukaryotic microbial cell or organism, a plant or a plant cell. The term "genetic modification" refers to a change by human intervention in the genetic content of a cell compared to a wild type cell and includes techniques like genetic engineering, breeding or mutagenesis. The change in genetic content comprises modifications of the genome and includes addition, deletion and substitution of genetic material in the chromosomes of said cell as well as in episomes. The term also encompasses the addition of extrachromosomal information to said cell. Preferably, the genetic modification results in modulated, preferably increased, expression of a nucleic acid. Genetic modification typically includes a selection step, during which the cells or organisms, preferably eukaryotic microbial cells or organisms, or plants or plant cells, with the desired characteristics are selected.

Thus, a particular embodiment of the present invention relates to a method for increasing the oxidative stress tolerance of a cell, preferably an eukaryotic cell, including but not limited to a fungal cell, yeast or a plant cell, comprising introducing into said cell a chimeric genetic construct comprising a nucleic acid capable of encoding an oxidative stress tolerance increasing peptide of the present invention.

The present invention thus also relates to a recombinant genetic construct or chimeric gene comprising a nucleic acid according to the invention operably linked to one or more regulatory elements. The genetic constructs facilitate the introduction and/or expression and/or maintenance of a nucleotide sequence as defined above into said cell or non-human organism, such as an eukaryotic microbial cell, a plant cell, tissue or organ. Preferably, the chimeric genetic construct comprises (i) an isolated nucleic acid encoding a peptide comprising a sequence having at least 70%, preferably 80% or 90%, more preferably 95%, 96%, 97%, 98%, 99% or 100% sequence identity or similarity to the amino acid sequence as given in SEQ ID No. 1, SEQ ID No. 2, SEQ ID No. 3, SEQ ID No. 4, SEQ ID No. 5, SEQ ID No. 6 or SEQ ID No. 7, preferably as given in SEQ ID No. 1, or a complement thereof; (ii) a regulatory element operably linked to the nucleic acid of (i), and optionally (iii) a 3' end region comprising a transcription termination sequence. Preferably, said regulatory element of (ii) is a regulatory element functional in said cell, e.g. a plant- or yeast-expressible promoter in the case of a plant or yeast cell, respectively. Preferably, said transcription termination sequence of (iii) is functional in said eukaryotic cell.

The term "operably linked" as used herein refers to a functional linkage between the regulatory element (or the termination sequence) and the gene or nucleic acid of interest, such that the regulatory element is able to initiate transcription of the gene or nucleic acid of interest.

For expression in a host cell, the nucleic acid molecule must be linked operably to or comprise a suitable promoter which expresses the gene at the right point in time and with the required spatial expression pattern. As used herein, the term "plant-expressible promoter" refers to a promoter that is capable of driving transcription in a plant cell. This not only includes any promoter of plant origin, but also any promoter of non-plant origin which is capable of directing transcription in a plant cell. The promoter may also be an artificial or synthetic promoter. The term "promoter", such as a "plant-expressible promoter", includes, but is not restricted to, constitutive, inducible, organ-, tissue- or cell-specific and/or developmentally regulated promoters. The terms "regulatory element", "control sequence", "promoter" are all used herein interchangeably and, taken in a broad context, refer to regulatory nucleic acids capable of effecting expression of the sequences to which they are ligated. Similarly, the term "yeast-expressible promoter" refers to a promoter that is capable of driving transcription in a yeast cell and encompasses natural yeast promoters as well as other promoter sequences capable of driving expression in yeast cells. Suitable promoters for expression in yeast are known in the art, see for example Current Protocols in Molecular Biology, Unit 13 (Ausubel et al., 1994) and the Guide to Yeast Genetics and Molecular Biology (Guthrie and Fink, 1991).

A "constitutive promoter" refers to a promoter that is transcriptionally active during most, but not necessarily all, phases of growth and development and under most environmental conditions, in at least one cell, tissue or organ, preferably in the majority of tissues or cells of an organism. A developmentally-regulated promoter is active during certain developmental stages or in parts of the plant or organism that undergo developmental changes. An inducible promoter has induced or increased transcription initiation in response to a chemical (for a review see Gatz 1997, Annu. Rev. Plant Physiol. Plant Mol. Biol., 48: 89-108), environmental or physical stimulus, or may be "stress-inducible", i.e. activated when a plant or cell is exposed to various stress conditions, or a "pathogen-inducible" i.e. activated when a plant is exposed to exposure to various pathogens. An organ-specific or tissue-specific promoter is one that is capable of preferentially initiating transcription in certain organs or tissues, such as the leaves, roots, seed tissue etc in case of a plant. In any case, the particular promoter selected to drive the expression of the oxidative stress tolerance inducing peptides of the invention in transgenic plants should be capable of causing sufficient expression of these peptides to result in the production of an effective amount of the peptides in plant tissues to confer an increased resistance to stress conditions in said plant tissues. Examples of constitutive promoters capable of driving such expression are the 35S, rice actin, maize ubiquitin, and elF-4A promoters or the promoter of the nopaline synthase gene ("PNOS") of the Ti-plasmid or the promoter of the octopine synthase gene.

Optionally, one or more terminator sequences may also be used in the construct introduced into said cell, preferably an eukaryotic cell, such as a plant, fungal, yeast or mammal cell. The term "terminator" encompasses a control sequence which is a DNA sequence, at the end of a transcriptional unit, which signals 3' processing and polyadenylation of a primary transcript and termination of transcription. The terminator can be derived from the natural gene, from a variety of other (plant) genes, or from T-DNA. The terminator to be added may be derived from, for example, the nopaline synthase or octopine synthase genes, or alternatively from another eukaryotic (plant) gene.

Additional regulatory elements may include transcriptional as well as translational enhancers. Those skilled in the art will be aware of terminator and enhancer sequences which may be suitable for use in performing the invention. Methods for increasing expression of genes or gene products are well documented in the art and include, for example, overexpression driven by appropriate promoters (as described herein), the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the peptide of interest. An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold [17, 18]. Such intron enhancement of gene expression is typically greatest when placed near the 5' end of the transcription unit. Use of the maize introns Adh1 -S intron 1, 2, and 6, the Bronze-1 intron are known in the art. For general information see: The Maize Handbook, Chapter 1 16, Freeling and Walbot, Eds., Springer, N.Y. (1994).

Furthermore, the recombinant nucleic acid can be constructed and employed to target the gene product of the nucleic acid of the invention to a specific intracellular compartment within said cell or to direct said peptide to the extracellular environment. This can generally be obtained by operably joining a DNA sequence encoding a transit or signal peptide to the recombinant nucleic acid.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a genetic construct of the invention or a derivative thereof. Suitable markers may be selected from markers that confer antibiotic resistance. Cells containing the recombinant DNA will thus be able to survive in the presence of antibiotic concentrations that kill untransformed cells. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers and different markers will be preferred, depending on the organism and the selection method.

Since the marker genes, particularly genes for resistance to antibiotics, are no longer required or are generally undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or comprises both vectors. In case of (plant) transformation with *Agrobacteria,* the transformants usually receive only a part of the vector, i.e. the sequence flanked by the T-DNA, which usually represents the expression cassette. The marker genes can subsequently be removed from the transformed organism (plant) by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. A further advantageous method relies on what is known as recombination systems, e.g. the Cre/Iox system whereby elimination of the marker gene by crossing is no longer necessary. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the (plant) genome of the nucleic acid sequences according to the invention is also contemplated.

According to another embodiment, the present invention relates to the use of the nucleic acid encoding a peptide of the present invention or the use of such a peptide as selectable marker gene in plants or other organisms, with selection taking place by treating with a suitable oxidative stress condition, such as in the presence of paraquat.

Thus, a particular embodiment of the present invention relates to a method for increasing the oxidative stress tolerance of an cell or organism, preferably an eukaryotic cell or non-human organism comprising introducing into said cell or (non-human) organism a chimeric genetic construct comprising a nucleic acid capable of encoding an oxidative stress tolerance increasing peptide comprising an amino acid sequence having at least 70%, preferably 80% or 90%, more preferably 95%, 96%, 97%, 98%, 99% or 100% sequence identity or similarity to the amino acid sequence as given in SEQ ID No. 1, SEQ ID No. 2, SEQ ID No. 3, SEQ ID No. 4, SEQ ID No. 5, SEQ ID No. 6 or SEQ ID No. 7, preferably as given in SEQ ID No. 1. Preferably, said eukaryotic cell or organism is a fungal cell or yeast, or a plant cell or plant, or a mammal cell. Advantageously, a transgenic microbial eukaryotic cell or organism, plant cell or plant obtained by the methods of the present invention is tolerant or resistant to oxidative stress conditions and/or has increased survivability in oxidative stress conditions or in the presence of agents that induce mitochondrial dysfunction.

The term "transformation" or "introduction" as referred to herein means the transfer of an exogenous or foreign polynucleotide or gene into a cell, irrespective of the method used for transfer. The polynucleotide may be transiently or stably introduced into said cell and may be maintained non-integrated, for example, as a plasmid, or alternatively, may be integrated into the host genome. Transformation of a cell, particularly a plant or fungal cell is now a fairly routine technique.

In the particular case of a plant cell, the resulting transformed plant cell may then be used to regenerate a transformed plant in a manner known to persons skilled in the art.

Advantageously, any of several transformation methods may be used to introduce the gene or polynucleotide of the present invention into a suitable ancestor cell. Transformation methods include the use of liposomes, electroporation, chemicals that increase free DNA uptake, injection of the DNA directly into the (plant) cell, particle gun bombardment, transformation using viruses or pollen and microprojection. Methods may be selected from the calcium/polyethylene glycol method for protoplasts (11); electroporation of protoplasts (12); microinjection into (plant) material (13); DNA or RNA-coated particle bombardment (14) infection with (non-integrative) viruses and the like.

Transgenic plants, including transgenic crop plants, are preferably produced via *Agrobacterium*-mediated transformation (e.g. as described in WO 94/00977, WO97/48814; WO98/54961). Said methods are further described by way of example in B. Jenes et al., Techniques for Gene Transfer, in: Transgenic Plants, Vol. 1 , Engineering and Utilization, eds. S.D. Kung and R. Wu, Academic Press (1993) 128-143 and in Potrykus Annu. Rev. Plant Physiol. Plant Molec. Biol. 42 (1991) 205-225. The nucleic acid or the chimeric genetic construct of the present invention is preferably cloned into a vector, which is suitable for transforming *Agrobacterium tumefaciens,* for example pBin19. Agrobacteria transformed by such a vector can then be used in known manner for the transformation of plants, such as described in for example, Hofgen and Willmitzer in Nucl. Acid Res. (1988) 16, 9877 or is known inter alia from F.F. White, Vectors for Gene Transfer in Higher Plants; in Transgenic Plants, Vol. 1 , Engineering and Utilization, eds. S.D. Kung and R. Wu, Academic Press, 1993, pp. 15-38.

In the particular case of plants, generally after transformation, the plant cells or cell groupings are selected for the presence of one or more markers which are encoded by suitable plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants.

Alternatively, following DNA transfer and regeneration, putatively transformed host cells, including but not limited to plants or plant cells, may be evaluated, for instance using Southern analysis, for the presence of the gene of interest, copy number and/or genomic organisation. Alternatively or additionally, expression levels of the newly introduced DNA in said host cell, including but not limited to plant cells, may be undertaken using Northern and/or Western analysis, both techniques being well known to persons having ordinary skill in the art.

A whole organism may be regenerated from a single transformed or transfected cell, using methods known in the art. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem).

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest. Plants that are particularly useful in the methods of the invention include in particular monocotyledonous and dicotyledonous plants including fodder or forage legumes, ornamental plants, food crops, trees or shrubs, such as rice, maize, wheat, barley, soybean, sunflower, canola, alfalfa, millet, barley, rapeseed, cotton, amaranth, artichoke, asparagus, broccoli, Brussels sprouts, cabbage, carrot, cauliflower, celery, collard greens, flax, kale, lentil, oilseed rape, okra, onion, potato, sugar beet, sugar cane, tomato, squash, or tea.

Furthermore, the characteristic of the transgenic cells or non-human organisms (plants) of the present invention to display tolerance to oxidative stress conditions may be combined with other approaches to confer abiotic or environmental stress tolerance, in particular oxidative stress tolerance, to said cell or organism. Thus, the approach of the present invention to confer tolerance to oxidative stress conditions to a host cell or (non-human) organism can be combined with known approaches, including the introduction of other stress tolerance genes. Combination of these approaches may have additive and/or synergistic effects in enhancing tolerance or resistance to oxidative and/or environmental stress.

Another embodiment of the invention provides host cells comprising a nucleic acid encoding a peptide of the present invention (as defined above) that increases the tolerance of a host cell or organism to oxidative stress conditions and/or increases the viability of said cell in oxidative stress conditions or in the presence of an agent inducing mitochondrial dysfunction. Preferred host cells are eukaryotic cells, including plant cells, fungal cells, yeast cells or mammal and human cells. The peptides of the present invention may also be produced by recombinant expression in prokaryotic and eukaryotic engineered cells other than plant cells, such as bacteria, fungi, or animal cells. Suitable expression systems are known to those skilled in the art.

The invention thus also extends to transgenic plants or eukaryotic microbial organisms, such as fungi or yeast, resistant to oxidative stress conditions and/or the presence of an agent resulting in mitochondrial dysfunction, wherein said plant or eukaryotic microbial organism has elevated levels of a peptide of the present invention compared to the corresponding wild type plant or eukaryotic microbial organism. Another embodiment of the present invention thus also provides a plant or an eukaryotic microbial organism obtainable by a method according to the present invention, wherein said plant or microbial organism has increased stress tolerance and has an altered level of a peptide of the present invention or a homologue thereof and/or has an altered expression of a nucleic acid encoding a peptide of the present invention or a homologue thereof.

Furthermore, a transgenic (non-human) organism for the purposes of the invention is thus understood as meaning that the nucleic acids used in the method of the invention (e.g. the chimeric genetic constructs) are not present in, or originating from, the genome of said organism, or are present in the genome of said organism but not at their natural locus in the genome of said organism, it being possible for the nucleic acids to be expressed homologously or heterologously. However, as mentioned, transgenic also means that, while the nucleic acids according to the invention or used in the inventive method are at their natural position in the genome of said organism, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, heterologous expression of the nucleic acids takes place.

In particular, the present invention provides plants or (non-human) organisms with increased tolerance to oxidative and/or abiotic stress, whereby said plant or (non-human) organism have increased expression of a nucleic acid encoding an oxidative stress tolerance increasing peptide comprising an amino acid sequence having at least 70%, preferably 80% or 90%, more preferably 95%, 96%, 97%, 98%, 99% or 100% sequence identity or similarity to the amino acid sequence as given in SEQ ID No. 1, SEQ ID No. 2, SEQ ID No. 3, SEQ ID No. 4, SEQ ID No. 5, SEQ ID No. 6 or SEQ ID No. 7, preferably as given in SEQ ID No. 1.

The present invention extends to any plant cell, plant or plant part or fungal or yeast cell obtained by any of the methods described herein, and to all plant parts, including harvestable parts of a plant, seeds and propagules thereof. The present invention also encompasses a plant or a part thereof comprising a plant cell transformed with a nucleic acid of the invention. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention.

The methods of the present invention to create an eukaryotic (non-human) organism, preferably a plant, with enhanced tolerance to (oxidative) stress can also be combined with other genes or traits of interest, known in the art, for example: herbicide tolerance; insect resistance; virus resistance; improving the preserving of fruits; improvement of starch composition and/or production; altering lipid composition; the production of (bio)polymers; alteration of the flower colour, e.g., by manipulating the anthocyanin and flavonoid biosynthetic pathway; resistance to bacteria, insects and fungi; inducing maintaining male and/or female sterility; other abiotic stress resistance, (e.g. temperature stress).

As discussed above, a nucleic acid according to the invention will have the capacity to increase tolerance to oxidative stress in a host cell or organism transfected therewith, preferably an eukaryotic host cell or organism, such as a plant or fungal cell or organism. The oxidative stress tolerance inducing effect may also be obtained by applying the peptide of the invention (as defined above) directly to said host cell or organism.

Thus, in a different embodiment, the present invention relates to a protective composition comprising a peptide or peptidomimetic of the invention, a nucleic acid of the invention, a genetic construct or vector of the invention or the host cell of the invention. In a preferred embodiment, said composition comprises an effective amount of an isolated peptide comprising an amino acid sequence which is at least 70% or 80%, more preferably at least 90%, more preferably at least 95%, 97%, 99% or 100% identical to amino acid sequence SEQ ID No. 1; SEQ ID No. 2; SEQ ID No. 3; SEQ ID No. 4; SEQ ID No. 5; SEQ ID No. 6 or SEQ ID No. 7, most preferably to SEQ ID No. 1. Preferably, said composition further comprises a physiologically acceptable carrier and/or diluent. In a preferred embodiment, the composition is a plant-protective composition. The term "plant-protective composition" relates to compositions used in the prevention or treatment of diseases related to plants, particularly in the prevention or treatment of oxidative stress conditions. Formulations of plant-protective compositions comprise wettable powders (WPs), emulsifiable concentrates (ECs), or emulsifiable microemulsion concentrates. Microemulsion is a colloidal system which, in a first approach differs from a true emulsion in the dimension of its particles which are smaller by an order of magnitude than those of a true emulsion. According to the general definition, this system contains surface active agents and two immiscible liquids, one of them is usually water, though, in principle, it is also possible to prepare a water-free microemulsion by using another solvent. The surfactant may be the mixture of even 6 to 8 tensides and additionally, it may contain alcohols or amines of medium chain length as auxiliary surfactants (cosurfactants).

A "physiologically acceptable carrier" is a nontoxic solid, semisolid or liquid filler, diluent, encapsulating material or formulation auxiliary of any type. By "agronomically acceptable carrier" is meant a solid or liquid filler, diluent or encapsulating substance that can be safely used in topical or systemic administration of a peptide of the invention to a plant, plant seed or plant cell. Examples of suitable carriers are well known in the art and comprise water or organic solvents as liquids.

### Oxidative stress related disorders

As mentioned, a peptide or nucleic acid of the present invention may be used to treat oxidative-stress related disorders both for veterinary and for human use. Oxidative stress may be responsible for initiating or otherwise causing disease. Alternatively, or additionally, the progression of the disease can be affected by any resultant oxidative stress. Particularly, oxidative stress and free radical damage is related to mitochondrial dysfunction.

Hence the phrase "oxidative stress related disorder" as used herein, refers to a disease or medical condition (including syndromes) wherein the onset or progression thereof is promoted by oxidative stress, in particular wherein the healthy function of one or more organelles, non-organelle subcellular structures, cell, cell types, tissues, tissue types, organs, or organ systems, particularly the mitochondria, is impaired by the action of oxidizing agents, particularly ROS. The action of oxidizing agents need not be the only route by which impairment of healthy function occurs in the course of a disease for the disease to be an oxidative stress disease.

Since oxidative stress is believed to be responsible for the pathogenesis of many neurological, heart, malignant and age-associated diseases, the present invention contemplates all such diseases including for example: a central nervous system (CNS) neurodegenerative disease, including but not limited to Parkinson's disease, Alzheimer's disease, multiple sclerosis, amyotrophic lateral sclerosis, or Huntington's disease; stroke; atherosclerosis; myocardial ischemia; myocardial reperfusion; autoimmune diseases; cancer; cardiovascular disease or diabetes or a complication of diabetes; circulatory impairment; retinopathy; blindness; kidney disease; pancreas disease; neuropathy; gum disease; cataracts; skin disease; skin damage by flame, heat, radiation (including ultraviolet light radiation). In one embodiment, the oxidative stress disease is senescence. "Senescence," as used herein, refers to one or more of a decrease in the overall health of a mammal, a decrease in the overall fitness of a mammal, or a decrease in the overall quality of life of a mammal, wherein such decrease is generally attributed to the aging process. Ameliorating senescence may lead to maintenance of a particular level of systemic well-being to a later point in the mammal's life, or may lead to at least a partial increase in the expected lifespan of the organism, such as a mammal.

Preferably, said oxidative stress related disease is a mitochondrial dysfunction related disorder or syndrome. Mitochondrial dysfunction relates to abnormalities in mitochondria and diseases and conditions associated with or involving decreased mitochondrial function. Conditions and diseases including various neurodegenerative diseases, including but not limited to Parkinson's disease, Alzheimer's disease and mitochondrial encephalopathies, as well as normal aging, complications of diabetes, and age-related macular degeneration are but a few examples of such diseases related to mitochondrial dysfunction. Other mitochondrial dysfunction diseases and syndrome include, the non-alcoholic fatty liver disease (NAFLD), the metabolic syndrome and disorders related to (inborn) errors in (mitochondrial) respiratory chain complexes, such as Wilson's Disease.

Thus, yet another preferred embodiment of the present invention relates to a peptide or peptidomimetic of the present invention for use in preventing, treating, ameliorating or diagnosing an oxidative stress-related disorder, particularly a mitochondrial dysfunction related disorder. Thus, the present invention provides a method of treating an oxidative stress-related disorder, preferably a mitochondrial dysfunction related disorder, in a subject in need thereof, the method comprising administering to the subject a therapeutically effective amount of said peptide or peptidomimetic of the present invention, thereby treating or ameliorating the oxidative stress-related or mitochondrial dysfunction related disorder. Alternatively, the present invention also relates to the use of a peptide or nucleic acid of the present invention for the manufacture of a medicament for the prevention and/or treatment of oxidative stress related disorders and to the use of said peptide or nucleic acid for the screening of materials for their therapeutic activity.

By "ameliorating" a disease or disorder is meant improving the condition of an organism suffering or at risk of suffering from the disease or disorder. Ameliorating can comprise one or more of the following: a reduction in the severity of a symptom of the disease, a reduction in the extent of a symptom of the disease, a reduction in the number of symptoms of the disease, a reduction in the number of disease agents, a reduction in the spread of a symptom of the disease, a delay in the onset of a symptom of the disease, a delay in disease onset, or a reduction in the time between onset of the disease and remission of the disease, among others apparent to the skilled artisan having the benefit of the present invention. To the extent that the foregoing examples of ameliorating a disease are defined in relative terms, the proper comparison is to the disease or symptoms thereof when no composition or material is administered to ameliorate it and no method is performed to ameliorate it. The terms "preventing" (herein meaning "to stop a disease from onsetting") and "treating" (herein meaning "to improve the condition of an organism, such as a mammal, suffering from a disease") are both within the scope of "ameliorating," as used herein.

A peptide or peptidomimetic of the present invention may be provided per se or as part of a pharmaceutical composition, where it may be formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. Formulations optionally contain excipients such as those set forth in the "Handbook of Pharmaceutical Excipients" (1986). As used herein a "pharmaceutical composition" refers to a preparation of one or more of the active ingredients described herein, i.e. a peptide or peptidomimetic of the present invention, with other chemical components such as pharmaceutically acceptable carriers and excipients. The purpose of a pharmaceutical composition is to facilitate administration of a compound to an organism. Thus, the present invention also relates to a composition comprising a) a peptide comprising an amino acid sequence which is at least 70%, more preferably at least 80%, more preferably at least 90%, more preferably at least 95%, 97%, 99% or 100% identical to an amino acid sequence selected from the amino acid sequences SEQ ID No. 1 to SEQ ID No. 575, preferably to an amino acid sequence selected from SEQ ID No:1, SEQ ID No. 2, SEQ ID No. 3, SEQ ID No. 4, SEQ ID No. 5, SEQ ID No. 6 or SEQ ID No. 7, more preferably to amino acid sequence SEQ ID No. 1., or a nucleic acid encoding for said peptide and b) one or more pharmaceutically acceptable compounds, carriers and/or adjuvants. The compositions of this invention can suitably be used as concentrates, emulsions, solutions, granulates, dusts, sprays, aerosols, suspensions, ointments, creams, tablets, pellets or powders.

The phrase "pharmaceutically acceptable carrier" as used herein refers to any material, substance, or diluent that does not cause significant irritation to an organism and does not abrogate the biological activity and properties of the administered compound with which the active ingredient is formulated in order to facilitate its application or dissemination to the locus to be treated, for instance by dissolving, dispersing or diffusing the said composition, and/or to facilitate its storage, transport or handling without impairing its effectiveness. An adjuvant is included under these phrases. The term "excipient" as used herein refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols. The term "active ingredient" refers to the oxidative stress tolerance inducing peptide(s) of the present invention accountable for the biological effect.

Techniques for formulation and administration of drugs may be found in "Remington's Pharmaceutical Sciences," Mack Publishing Co., Easton, PA, latest edition (herein incorporated by reference). Suitable pharmaceutical carriers for use in the said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives.

The oxidative stress tolerance inducing agents of the present invention and pharmaceutical compositions thereof may be administered by any route appropriate to the condition to be treated. Suitable routes of administration may, for example, include oral, rectal, nasal, topical (including ocular, buccal and sublingual), vaginal and parenteral (including subcutaneous, intramuscular, intravenous, intradermal, intrathecal and epidural). The preferred route of administration may vary with for example the condition of the recipient.

Alternately, one may administer the pharmaceutical composition in a local rather than systemic manner, for example, via injection of the pharmaceutical composition directly into a tissue region of a patient..

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

The formulations may be optionally applied as a topical ointment or cream containing the active ingredient(s) (e.g. sun screen), such as e.g. when treating (prophylactically or therapeutically) oxidative damage to the skin of a patient. When formulated in an ointment, the active ingredients may be employed with either a paraffinic or a water-miscible ointment base. Alternatively, the active ingredients may be formulated in a cream with an oil-in-water cream base. If desired, the aqueous phase of the cream base may include, for example, at least 30% w/w of a polyhydric alcohol, i.e. an alcohol having two or more hydroxyll groups such as propylene glycol, butane 1,3-diol, mannitol, sorbitol, glycerol and polyethylene glycol (including PEG400) and mixtures thereof. The topical formulations may desirably include a compound which enhances absorption or penetration of the active ingredient through the skin or other affected areas. Examples of such dermal penetration enhancers include dimethylsulfoxide and related analogs. The oily phase of said emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Optionally, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous sterile solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions. The parenteral solutions may further contain anti-oxidants, buffers, bacteriostats and solutes which render the formulation isotonic with the blood of the intended recipient.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients, i.e. the oxidative stress tolerance inducing peptides of the present invention, effective to prevent, alleviate or ameliorate symptoms of a disorder (e.g., Parkinson's Disease) or prolong the survival of the subject being treated.

Determination of a therapeutically effective amount is well within the capability of those skilled in the art. For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.I).

Dosage amount and interval may be adjusted individually to tissue or blood levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

**Table 1. List of oxidative stress inhibiting peptides deduced from the different open reading frames of the OSIP encoding regions (based on tiling array data)**

| **PEPTIDE CODE** | **AMINO ACID CODE** | **SEQ ID NO** |
|---|---|---|
| >SIP108_4\|PQ4\|-\|3 | MLCVLQGLRE- | SEQ ID No. 1 |
| >SIP14_1\|PQ4\|+\|2 | MIIINNDNYLLLFYNNN- | SEQ ID No. 2 |
| >SIP14_2\|PQ4\|+\|3 | MIIIYYCFIIIIN- | SEQ ID No. 3 |
| >SIP11_0\|PQ4\|+\|3 | MLMYRMRSGAN- | SEQ ID No. 4 |
| >SIP163_3\|PQ4\|-\|3 | MGLNEDSVFRSIKPFKSP- | SEQ ID No. 5 |
| >SIP152_2\|PQ4\|+\|3 | | SEQ ID No. 6 |
| >SIP37_1\|PQ4\|-\|1 | MAREEKEQSVYDIYTFASLLL- | SEQ ID No. 7 |
| >SIP74_2\|PQ4\|+\|3 | MHCLLSLLEMVPSNLLSALLNLN- | SEQ ID No. 8 |
| >SIP74_3\|PQ4\|-\|1 | MSVEICRRYYLPPPNNCTYTTPQTSI- | SEQ ID No. 9 |
| >SIP74_4\|PQ4\|-\|2 | MICYKNLARQNIN- | SEQ ID No. 10 |
| >SIP74_5\|PQ4\|-\|3 | MSSYQSIYEC- | SEQ ID No. 11 |
| >SIP133_1\|PQ4\|+\|3 | MIHIHINWRLIRIG- | SEQ ID No. 12 |
| >SIP133_2\|PQ4\|-\|1 | MHIYALANSNQSPINVYMYHSHRP- | SEQ ID No. 13 |
| >SIP133_3\|PQ4\|-\|2 | MCICIIHIGPRLILTILQYVF- | SEQ ID No. 14 |
| >SIP89_0\|PQ4\|+\|1 | | SEQ ID No. 15 |
| >SIP89_1\|PQ4\|+\|1 | MKLVQADPFSDCF- | SEQ ID No. 16 |
| >SIP89_2\|PQ4\|+\|2 | MEAPSRVIIIDACHSRSWSHQRERRRNHHR- | SEQ ID No. 17 |
| >SIP89_3\|PQ4\|+\|2 | MLVNLARENKIKT- | SEQ ID No. 18 |
| >SIP89_4\|PQ4\|+\|2 | MKKAYGKIDMHSFYIDLGPHSFNHS- | SEQ ID No. 19 |
| >SIP89_5\|PQ4\|+\|3 | MLVILVLGVINGREEETIIVEPYGFQNTQGRDR- | SEQ ID No. 20 |
| >SIP89_6\|PQ4\|+\|3 | MGRLICTHFTLI- | SEQ ID No. 21 |
| >SIP89_7\|PQ4\|+\|3 | MLRSHETCTSRSI- | SEQ ID No. 22 |
| >SIP89_8\|PQ4\|-\|1 | MSAYQSSHKLSSSMS- | SEQ ID No. 23 |
| >SIP5_0\|PQ4\|+\|1 | MNKTQSHLLFSLSTCLNSYVNTKLPSILVNSKLSINT- | SEQ ID No. 24 |
| >SIP5_3\|PQ4\|+\|2 | MNHLOISLIS- | SEQ ID No. 25 |
| >SIP5_4\|PQ4\|+\|3 | MPKLICEHQTPIHSRQFKALYQYITSITLPMR- | SEQ ID No. 26 |
| >SIP5_5\|PQ4\|-\|1 | MGVWCSHMSLGMLIS- | SEQ ID No. 27 |
| >SIP5_6\|PQ4\|-\|2 | MGRGKVWVVEQSRRKI- | SEQ ID No. 28 |
| >SIP5_7\|PQ4\|-\|3 | MDGSLVFTYEFRHVDKLKSKWD- | SEQ ID No. 29 |
| >SIP32_0\|PQ4\|+\|1 | | SEQ ID No. 30 |
| >SIP32_2\|PQ4\|+\|2 | | SEQ ID No. 31 |
| >SIP32_4\|PQ4\|+\|3 | MVGRRYNGLHSLSR- | SEQ ID No. 32 |
| >SIP32_5\|PQ4\|+\|3 | MDRQANPKSAVLNQAQGRSTI- | SEQ ID No. 33 |
| >SIP32_6\|PQ4\|-\|2 | MSEVVHYRGPVGSISRFENSLYKRCPNC- | SEQ ID No. 34 |
| >SIP32_7\|PQ4\|-\|3 | MQRELKEFKLKIEFEKKETKL- | SEQ ID No. 35 |
| >SIP108_0\|PQ4\|+\|1 | MDNRDKSCFANKHAVAGKLIGFRTPAYSLKP- | SEQ ID No. 36 |
| >SIP108_2\|PQ4\|-\|2 | MCASRFKGVGGCSKTDQFTCNCMFICKT- | SEQ ID No. 37 |
| >SIP108_3\|PQ4\|-\|3 | MVTKETQAEGEKVCVIIIL- | SEQ ID No. 38 |
| >SIP75_1\|PQ4\|+\|2 | MLCCWDFDRSLPLILL- | SEQ ID No. 39 |
| >SIP84_0\|PQ4\|+\|1 | | SEQ ID No. 40 |
| >SIP84_3\|PQ4\|+\|3 | | SEQ ID No. 41 |
| >SIP84_5\|PQ4\|-\|1 | MWSPSWRSTLPQRSMLQRVRLVR- | SEQ ID No. 42 |
| >SIP84_6\|PQ4\|-\|2 | | SEQ ID No. 43 |
| >SIP84_7\|PQ4\|-\|3 | | SEQ ID No. 44 |
| >SIP129_0\|PQ4\|+\|1 | | SEQ ID No. 45 |
| >SIP129_2\|PQ4\|-\|2 | MLYAQALILCFI- | SEQ ID No. 46 |
| >SIP129_3\|PQ4\|-\|3 | | SEQ ID No. 47 |
| >SIP129_4\|PQ4\|-\|3 | MKYWDVDFDINERSFWL- | SEQ ID No. 48 |
| >SIP148_0\|PQ4\|+\|1 | MLYKNSNDSM- | SEQ ID No. 49 |
| >SIP148_2\|PQ4\|+\|3 | | SEQ ID No. 50 |
| >SIP148_4\|PQ4\|-\|2 | MTHPIRGICEQILDFTRSIKL- | SEQ ID No. 51 |
| >SIP163_0\|PQ4\|+\|1 | MPQLCLDLMKSLKL- | SEQ ID No. 52 |
| >SIP163_1\|PQ4\|+\|1 | | SEQ ID No. 53 |
| >SIP163_2\|PQ4\|-\|2 | MIIPSSLKTRKLKVESKS- | SEQ ID No. 54 |
| >SIP75_2\|PQ4\|+\|2 | MSRRMILTQYW- | SEQ ID No. 55 |
| >SIP14_0\|PQ4\|+\|1 | | SEQ ID No. 56 |
| >SIP147_1\|PQ4\|+\|2 | MKPYMINNFS- | SEQ ID No. 57 |
| >SIP147_3\|PQ4\|-\|2 | MSSPPIPSPLIPSPPIPPPPPRFYVPPSKSRRGKGP- | SEQ ID No. 58 |
| >SIP147_4\|PQ4\|-\|3 | MVANYLMKQYLQYCYQVKRCRLHQFHLH- | SEQ ID No. 59 |
| >SIP147_5\|PQ4\|-\|3 | MYLLQNPAEEKAHKQIE- | SEQ ID No. 60 |
| >S1P152_1\|PQ4\|+\|2 | MQENKARVTTEKRSVCRTPPINTENRFDCLMIC- | SEQ ID No. 61 |
| >SIP75_3\|PQ4\|+\|3 | MHLRKRRRLLSQKRTKW- | SEQ ID No. 62 |
| >SIP152_3\|PQ4\|-\|1 | MARHSLLSPPYDSPLSLYIFSF- | SEQ ID No. 63 |
| >SIP152_4\|PQ4\|-\|2 | MTVLFHCTYSLFDDK- | SEQ ID No. 64 |
| >SIP152_5\|PQ4\|-\|3 | MLSRSSNNQICSPC- | SEQ ID No. 65 |
| >SIP152_6\|PQ4\|-\|3 | MKKICWASQLS- | SEQ ID No. 66 |
| >SIP87_1\|PQ4\|+\|2 | MDPAMEVDMVKGMEADTVVEIITVTHPKSFLLLIVS- | SEQ ID No. 67 |
| >SIP87_2\|PQ4\|+\|3 | MIIMCSMFCRYIYIHACI- | SEQ ID No. 68 |
| >SIP87_3\|PQ4\|-\|1 | MYIYVTTKHRTHYYHKFRIMKQ- | SEQ ID No. 69 |
| >SIP8_1\|PQ4\|+\|1 | MFCKFAIFHFYA- | SEQ ID No. 70 |
| >SIP8_2\|PQ4\|+\|2 | MEETLYTQHQK- | SEQ ID No. 71 |
| >SIP8_3\|PQ4\|+\|2 | MPENQHKSGE- | SEQ ID No. 72 |
| >SIP8_6\|PQ4\|-\|3 | MEDCELAEHDLVIETAVSSAGKLNPFLMFSYFWCCV- | SEQ ID No. 73 |
| >SIP8_7\|PQ4\|-\|3 | MRFLYNTLPGFQIDFFSSV- | SEQ ID No. 74 |
| >SIP58_2\|PQ4\|+\|2 | MCCQPLPLAIPLRTAVKPSRIKERQSKGVRSAT- | SEQ ID No. 75 |
| >SIP58_3\|PQ4\|+\|2 | MMFLQVNRSMLKKADSTIQRQSLANTETLAT- | SEQ ID No. 76 |
| >SIP58_4\|PQ4\|-\|1 | | SEQ ID No. 77 |
| >SIP58_5\|PQ4\|-\|1 | MGLLGEWGNTLELGTILFLIISYELLLEFT- | SEQ ID No. 78 |
| >SIP58_6\|PQ4\|-\|2 | MSSSWNLLSFQS- | SEQ ID No. 79 |
| >SIP145_1\|PQ4\|+\|2 | MNELQRLYLKT- | SEQ ID No. 80 |
| >SIP145_2\|PQ4\|+\|2 | MIYESQDTTDKLIPEHMSNRLTMTGSKDIK- | SEQ ID No. 81 |
| >SIP145_4\|PQ4+\|3 | MIYESQDTTDKLISKHMSNRLKQT- | SEQ ID No. 82 |
| >SIP145_5\|PQ4\|-\|2 | MCLDINLSWSWDS- | SEQ ID No. 83 |
| >SIP145_6\|PQ4\|-\|2 | MIVSKYLQPI- | SEQ ID No. 84 |
| >SIP145_7\|PQ4\|-\|3 | MSLDPVIVSLLLMCSGISLSVVSWDS- | SEQ ID No. 85 |
| >SIP43_1\|PQ4\|+\|3 | | SEQ ID No. 86 |
| >SIP43_2\|PQ4\|-\|1 | | SEQ ID No. 87 |
| >SIP43_3\|PQ4\|-\|2 | | SEQ ID No. 88 |
| >SIP43_4\|PQ4\|-\|2 | MNFHMILMITRPCNAGMNGLWD- | SEQ ID No. 89 |
| >SIP43_5\|PQ4\|-\|3 | | SEQ ID No. 90 |
| >SIP65_2\|PQ4\|+\|3 | MRDQFNLQISAW- | SEQ ID No. 91 |
| >SIP65_3\|PQ4\|+\|3 | MNQIQLlYFNFLQPQCTIYLlFLFTKVFIYNTKNI- | SEQ ID No. 92 |
| >SIP65_5\|PQ4\|-\|1 | MGHHRILRKTHTSYIFRVIYKNFGEQKN- | SEQ ID No. 93 |
| >SIP6_3\|PQ4\|-\|2 | MIHHLYLARNPRISDTTSLITPILKC- | SEQ ID No. 94 |
| >SIP144_1\|PQ4\|-\|3 | MQNQIEKRCQFY- | SEQ ID No. 95 |
| >SIP144_2\|PQ4\|-\|3 | MNNELQQLYLKTKSKSHKQDIISFFLIQ- | SEQ ID No. 96 |
| >SIP78_0\|PQ4\|-\|1 | | SEQ ID No. 97 |
| >SIP78_1\|PQ4\|-\|2 | MLFSYTNLLNVCICPLERR- | SEQ ID No. 98 |
| >SIP78_2\|PQ4\|-\|3 | MCAYVPSNAGDEYCDRNASDCENRGSV- | SEQ ID No. 99 |
| >SIP149_1\|PQ4\|+\|1 | MSCSDENLLTLDGVFNCVL- | SEQ ID No. 100 |
| >SIP149_2\|PQ4\|+\|2 | MTISYIFFLLKTAI- | SEQ ID No. 101 |
| >SIP149_3\|PQ4\|+\|3 | | SEQ ID No. 102 |
| >SIP149_5\|PQ4\|-\|1 | MCNRIFPDICP- | SEQ ID No. 103 |
| >SIP149_6\|PQ4\|-\|1 | MMMIFQDPLSSHHYIAVLRRKKIYDIVIVEEILVRS- | SEQ ID No. 104 |
| >SIP149_7\|PQ4\|-\|2 | MILSLSRRYLFDREREKY- | SEQ ID No. 105 |
| >SIP155_0\|PQ4\|+\|1 | | SEQ ID No. 106 |
| >SIP155_2\|PQ4\|-\|3 | MWSISPRKKKQYCCHHKCLVLFSLPLFA- | SEQ ID No. 107 |
| >SIP162_0\|PQ4\|+1\| | MTWSNVIQLSLELKQPATSGT- | SEQ ID No. 108 |
| >SIP162_1\|PQ4\|+\|1 | MPLPVQALSLQVWD- | SEQ ID No. 109 |
| >SIP162_2PQ4\|+\|1 | MWCKHSSLLFLR- | SEQ ID No. 110 |
| >SIP162_4\|PQ4\|-\|2 | MDQSQTWRDKACTGKGIVD- | SEQ ID No. 111 |
| >SIP162_5\|PQ4\|-\|2 | | SEQ ID No. 112 |
| >SIP162_6\|PQ4\|-\|3 | MIFTLETGGRSVYTTSG- | SEQ ID No. 113 |
| >SIP134_0\|PQ4\|+\|1 | MGYGRSLIWLAEDTFGEISRRCVREVNDRYKQSFACLA | SEQ ID No. 114 |
| | SDM- | |
| >SIP134_1\|PQ4\|+\|2 | MTDINKVSPVWLL1CRKLRTRKIVSSFS- | SEQ ID No. 115 |
| >SIP134_3\|PQ4\|-\|2 | | SEQ ID No. 116 |
| >S1P134_4\|PQ4\|-\|3 | MKFKYMNTKLKNE- | SEQ ID No. 117 |
| >SIP134_5\|PQ4\|-\|3 | | SEQ ID No. 118 |
| >SIP26_0\|PQ4\|+\|3 | MQQRVNCGHHLDLL- | SEQ ID No. 119 |
| >SIP26_2\|PQ4\|-\|3 | MVNHLQQIQMVTAIDSLLHRLYQGRQRLN- | SEQ ID No. 120 |
| >SIP0_0\|PQ4\|+\|1 | MKERSKTQLN- | SEQ ID No. 121 |
| >SIP0_1\|PQ4\|+\|3 | MGYRNRSYLVKSLPF- | SEQ ID No. 122 |
| >SIP99_0\|PQ4\|+\|1 | MRSRRIKKTRRIKRTRRIN- | SEQ ID No. 123 |
| >SIP99_1\|PQ41+\|2 | MFFE4RLGFHLLAGLVWLVNPSRF- | SEQ ID No. 124 |
| >SIP102_1\|PQ4\|+\|1 | MLSNDANRETGKSLYLKG- | SEQ ID No. 125 |
| >SIP102_3\|PQ4\|+\|2 | MMQTVKLESLYISRDKTPELALLWL- | SEQ ID No. 126 |
| >SIP120_0\|PQ4\|-\|2 | | SEQ ID No. 127 |
| >SIP31_0\|PQ4\|+\|1 | MSFLAENVESRFLAKKC- | SEQ ID No. 128 |
| >SIP31_1\|PQ4\|+\|1 | MLNCFLAGKY- | SEQ ID No. 129 |
| >SIP31_2\|PQ4\|+\|1 | MNVVFGGKMLNCVLAGKY- | SEQ ID No. 130 |
| >SIP31_4\|PQ4\|+\|2 | MLIWFGEKMLNCVLAGK- | SEQ ID No. 131 |
| >SIP31_5\|PQ4\|+\|3 | MFNWFGGKC- | SEQ ID No. 132 |
| >SIP31_6\|PQ4\|+\|3 | MLNCVLARKY- | SEQ ID No. 133 |
| >SIP31_7\|PQ4\|+\|3 | MLNCVLAGKC- | SEQ ID No. 134 |
| >SIP103_2\|PQ4\|-\|1 | MSLIIQICFSLSERHLWAYIHF- | SEQ ID No. 135 |
| >SIP103_4\|PQ4\|-\|3 | MALNNYRLQIPLKHLPIPRHKLRNELNNTNLLLLE- | SEQ ID No. 136 |
| >SIP44_0\|PQ4\|+\|2 | MMIIHLQVTQHSEKDRLRKNHTYKLFCKNIF- | SEQ ID No. 137 |
| >SIP44_3[PQ4\|-\|1 | MYDFFSAYLFHYW- | SEQ ID No. 138 |
| >SIP44_4\|PQ4\|-\|2 | MICRLRRLGREWSLVKIDWLKLRMFLRAISNYFCRC- | SEQ ID No. 139 |
| >SIP44_5\|PQ4\|-\|2 | MIFSQPIFFTMLCNL- | SEQ ID No. 140 |
| >SIP79_1\|PQ4\|+\|2 | MVSCSNVRCGLSGCFGRKRT- | SEQ ID No. 141 |
| >SIP79_3\|PQ4\|-\|1 | MSRLSLCVFVLLCAFAAKAAAQSAPNV- | SEQ ID No. 142 |
| >SIP79_5\|PQ4\|-\|3 | MCVCVAMCVCGQSSRSIRT- | SEQ ID No. 143 |
| >SIP106_0\|PQ4\|+\|2 | MMIASQAVLHSQLQATWLQQSQLKQN- | SEQ ID No. 144 |
| >SIP106_2\|PQ4\|-\|1 | MGLDGVTNVDFDELGVDVSKLLLSLKWFWLLD- | SEQ ID No. 145 |
| >SIP106_3\|PQ4\|-\|2 | MWILMNWVLTCRNCC- | SEQ ID No. 146 |
| >SIP106_4\|PQ4\|-\|3 | MNGSRWSNECGF- | SEQ ID No. 147 |
| >SIP106_5\|PQ4\|-\|3 | MVLVARLRLRAQARSCWC- | SEQ ID No. 148 |
| >SIP22_1\|PQ4\|+\|3 | MHGTDVTFRKDARSGCDL- | SEQ ID No. 149 |
| >SIP22_2\|PQ4\|-\|1 | | SEQ ID No. 150 |
| >SIP22_3\|PQ4\|-\|2 | | SEQ ID No. 151 |
| >SIP72_0\|PQ4\|+\|1 | MAELLQQVRY- | SEQ ID No. 152 |
| >SIP72_2\|PQ4\|+\|2 | MTLTCISQLLSS- | SEQ ID No. 153 |
| >SIP72_3\|PQ4\|-\|2 | MSVLVADLLEQFRHHLVELQISMLSYKLLMRFSLRILL- | SEQ ID No. 154 |
| >SIP36_1\|PQ4\|+\|2 | MI LKCWSSRFLRVSPYQNAHSLSLG- | SEQ ID No. 155 |
| >SIP36_4\|PQ4\|-\|2 | MRILIWTHSQEPGTPAL- | SEQ ID No. 156 |
| >SIP153_2\|PQ4\|-\|3 | | SEQ ID No. 157 |
| >SIP17_1\|PQ4\|-\|2 | MSTKVNGGGDE- | SEQ ID No. 158 |
| >SIP17_2\|PQ4\|-\|2 | MDMMVVNTEAVVFTDLVILNTEEAVVLRI- | SEQ ID No. 159 |
| >SIP17_3\|PQ4\|-\|3 | MNREEAVVLWI- | SEQ ID No. 160 |
| >SIP92_0\|PQ4\|-\|1 | MGIRYLNGPKRTRPTSCKPNFPSDK- | SEQ ID No. 161 |
| >SIP92_1\|PQ4\|-\|2 | | SEQ ID No. 162 |
| >SIP92_2\|PQ4\|-\|3 | MKWMVAAWRR- | SEQ ID No. 163 |
| >SIP45_0\|PQ4\|-\|2 | MVFLRAISNYL- | SEQ ID No. 164 |
| >SIP45_1\|PQ4\|-\|3 | MICRPRRQGRD- | SEQ ID No. 165 |
| >SIP109_1\|PQ4\|-\|1 | MNMLLKSQRYMHYP- | SEQ ID No. 166 |
| >SIP109_2\|PQ4\|-\|3 | MILCEYIYEHALKVTKIYALPVDAPVTVGFVF- | SEQ ID No. 167 |
| >SIP76_0\|PQ4\|+\|3 | MFLSVLEFGS- | SEQ ID No. 168 |
| >SIP76_2\|PQ4\|-\|1 | MYLWGMQEAMMNDIVPSMMQ- | SEQ ID No. 169 |
| >SIP76_3\|PQ4\|-\|2 | MERCTCGACKKR- | SEQ ID No. 170 |
| >SIP76_4\|PQ4\|-\|3 | MCSHWKREWRDVLVGHARSDDE- | SEQ ID No. 171 |
| >SIP165_0\|PQ4\|+\|1 | MNHLNRPKVLTQGPLSKIFNSSNRYSLL- | SEQ ID No. 172 |
| >SIP165_2\|PQ4\|-\|1 | MSGYSYDCTGLFPVLL- | SEQ ID No. 173 |
| >SIP165_3\|PQ4\|-\|2 | MIARVCSQSYCEEELPINIQRHVCWK1/FAVQAA- | SEQ ID No. 174 |
| >SIP19_1\|PQ4\|+\|1 | MSTSSTWNHMQHAIKHN- | SEQ ID No. 175 |
| >SIP19_2\|PQ4\|+\|2 | MRKYNNIESITSSNQ- | SEQ ID No. 176 |
| >SIP19_3\|PQ4\|+\|3 | MEPHATRNQT- | SEQ ID No. 177 |
| >SIP19_4\|PQ4\|-\|2 | MFDCVLHVVPCTRRTHGNQLDCLFY- | SEQ ID No. 178 |
| >SIP19_6\|PQ4\|-\|3 | | SEQ ID No. 179 |
| >SIP104_1\|PQ4\|-\|1 | MQQRLMMNLLDQWELVA- | SEQ ID No. 180 |
| >SIP104_2\|PQ4\|-\|2 | | SEQ ID No. 181 |
| >SIP104_3\|PQ4\|-\|2 | MLSFGFRQDLFDTTRLSKNGKK- | SEQ ID No. 182 |
| >SIP88_1\|PQ4\|+\|2 | | SEQ ID No. 183 |
| >SIP88_2\|PQ4\|-\|2 | MEKKVTKHKK- | SEQ ID No. 184 |
| >SIP27_0\|PQ4\|+\|1 | MRKALDITRKPLSWSTGHTK- | SEQ ID No. 185 |
| >SIP27_1\|PQ4\|+\|3 | MEKAEHLSSSAHEKSS- | SEQ ID No. 186 |
| >SIP27_3\|PQ4\|-\|3 | MIQKSQTRLVIKLYFV- | SEQ ID No. 187 |
| >SIP130_0\|PQ4\|+\|1 | MNPTIDPTTI- | SEQ ID No. 188 |
| >SIP130_1\|PQ4\|-\|1 | MDFGSYGFNLPVLVMSRSYGGGGG- | SEQ ID No. 189 |
| >SIP52_0\|PQ4\|+\|1 | MHSQYYSPNHGY- | SEQ ID No. 190 |
| >SIP52_1\|PQ4\|+\|2 | MATNKIRLGTKNQTE- | SEQ ID No. 191 |
| >SIP52_3\|PQ4\|-\|1 | | SEQ ID No. 192 |
| >SIP52_4\|PQ4\|-\|1 | MYSDEMLLLLGGL- | SEQ ID No. 193 |
| >SIP174_0\|PQ4\|+\|1 | MADLSPNLLKQPKGLALSRF- | SEQ ID No. 194 |
| >SIP174_2\|PQ4\|+\|2 | MVLASSLLGARPFVG- | SEQ ID No. 195 |
| >SIP174_3\|PQ4\|-\|1 | MSRNLTHRPKRRENTRT- | SEQ ID No. 196 |
| >SIP174_4\|PQ4\|-\|1 | MDELQGGRRQAPCFQRSGGPNLI- | SEQ ID No. 197 |
| >SIP174_5\|PQ4\|-\|3 | MLSEKWRSESYLNCENN- | SEQ ID No. 198 |
| >SIP47_0\|PQ4\|+\|3 | | SEQ ID No. 199 |
| >SIP47_1\|PQ4\|-1\|1 | MGTRKNTRFYQGRRRKFWSR- | SEQ ID No. 200 |
| >SIP60_1\|PQ4\|+\|3 | MEYKCHNSDFTAVEK- | SEQ ID No. 201 |
| >SIP60_2\|PQ4\|-\|1 | MLMVKMDQKDPDTISMSLFSCFLTFPLQ- | SEQ ID No. 202 |
| >SIP168_0\|PQ4\|+\|2 | MAYDVRGNNVTTHVMGPFSGPSEVWLGP- | SEQ ID No. 203 |
| >SIP168_1\|PQ4\|+\|3 | MLWAHLVAQVKSGLALKSMEGLM- | SEQ ID No. 204 |
| >SIP146_0\|PQ4\|+\|1 | MPPPPKRQAVGPRVYAIAGEEDVDEDGADPIVGKSS- | SEQ ID No. 205 |
| >SIP146_1\|PQ4\|+\|2 | MLMRMAPTRLLVSLLKNLFK- | SEQ ID No. 206 |
| >SIP146_2\|PQ4\|+\|3 | MSIACACFWCSCLS- | SEQ ID No. 207 |
| >SIP146_3\|PQ4\|-\|2 | MAYTRGPTACRFGGGGIIPLF- | SEQ ID No. 208 |
| >SIP64_0\|PQ4\|+\|2 | MMPRRPTRRLYSSLKTPLRHDA- | SEQ ID No. 209 |
| >SIP64_1\|PQ4\|-\|1 | | SEQ ID No. 210 |
| >SIP64_3\|PQ4\|-\|3 | MFLLLSCSIVLFQISIAHRL- | SEQ ID No. 211 |
| >SIP46_0\|PQ4\|+\|2 | MNVRIHPITN- | SEQ ID No. 212 |
| >SIP46_2\|PQ4\|+\|3 | MSGYIRSQTNTRAHEY- | SEQ ID No. 213 |
| >SIP117_0\|PQ4\|+\|3 | MFFLQLQLHRGLV- | SEQ ID No. 214 |
| >SIP117_1\|PQ4\|+\|3 | MKRTLKAQRMILKLLPVSKMHCLSNL- | SEQ ID No. 215 |
| >SIP158_1\|PQ4\|+\|3 | MSLVYWVALFHVHAVNNFHLPQP- | SEQ ID No. 216 |
| >SIP158_3\|PQ4\|-\|1 | | SEQ ID No. 217 |
| | | |
| >SIP158_4\|PQ4\|-\|2 | MFFTLTIESAVCFINRRRGLGSKGDHRDQG- | SEQ ID No. 218 |
| >SIP30_0\|PQ4\|+\|3 | MPCCLAPSPDWTRH- | SEQ ID No. 219 |
| >SIP30_2\|PQ4\|-\|2 | MSGPVRRWSEATRHVRRDRSGNALFM- | SEQ ID No. 220 |
| >SIP33_0\|PQ4\|+\|3 | MEASGTKVPSESRNIALLLDRL- | SEQ ID No. 221 |
| >SIP23_0\|PQ4\|+\|3 | MSNPELISHTQQKELRWQRLHLVM- | SEQ ID No. 222 |
| >SIP23_2\|PQ4\|-\|1 | MIRCFCVTTKVLSFFRFKCCITLV- | SEQ ID No. 223 |
| >SIP23_3\|PQ4\|-\|3 | MFLCDYQSFELF- | SEQ ID No. 224 |
| >SIP23_4\|PQ4\|-\|3 | MLYHFGVGSVWVFIR- | SEQ ID No. 225 |
| >SIP23_5\|PQ4\|-\|3 | MTRCSLCHRSSFC- | SEQ ID No. 226 |
| >SIP56_1\|PQ4\|-\|2 | MNPESSSAES- | SEQ ID No. 227 |
| >SIP56_3\|PQ4\|-\|3 | MRMRRGTRRRASR- | SEQ ID No. 228 |
| >SIP132_0\|PQ4\|+\|2 | MISDPSFAEALHKRRLSSAKRRW- | SEQ ID No. 229 |
| >SIP132_2\|PQ4\|-\|3 | MIVSSCAKPQRMRDQRSCVA- | SEQ ID No. 230 |
| >SIP132_3\|PQ4\|-\|3 | MGKPRIKSSTIPSHLLFLGLR- | SEQ ID No. 231 |
| >SIP21_0\|PQ4\|+\|1 | | SEQ ID No. 232 |
| >SIP21_1\|PQ4\|-\|1 | MPSCLDRKPNLLKL- | SEQ ID No. 233 |
| >SIP15_1\|PQ4\|+\|2 | MYYPNIKPENNLNFVF- | SEQ ID No. 234 |
| >SIP15_3\|PQ4\|-\|1 | MNHDLYLLKYSVLCEVNIGPVLG- | SEQ ID No. 235 |
| >SIP15_4\|PQ4\|-\|1 | MSSVLILIAFQD- | SEQ ID No. 236 |
| >SIP15_5\|PQ4\|-\|2 | MYTLVLLNVKCRRCLY- | SEQ ID No. 237 |
| >SIP15_6\|PQ4\|-\|3 | MLNWGAYINCFSRLENKVKIVFRFNVWWHIY- | SEQ ID No. 238 |
| >SIP100_0\|PQ4\|+\|1 | MYTIMRFSYRISSTSSRFR- | SEQ ID No. 239 |
| >SIP100_1\|PQ4\|-\|3 | MILHLKVHLNLELVLEIR- | SEQ ID No. 240 |
| >SIP101_2\|PQ4\|-\|1 | MFRADELLEKDEVTSFVRRAS- | SEQ ID No. 241 |
| >SIP101_3\|PQ4\|-\|2 | MTKLCFELTSFLRRTR- | SEQ ID No. 242 |
| >SIP173_0\|PQ4\|+\|1 | MYRTLSTRSS- | SEQ ID No. 243 |
| >SIP173_2\|PQ4\|+\|2 | MIEARTLSGQA- | SEQ ID No. 244 |
| >SIP173_3\|PQ4\|-\|2 | MMTWSREYDTSV- | SEQ ID No. 245 |
| >SIP42_0\|PQ4\|+\|1 | | SEQ ID No. 246 |
| >SIP42_1\|PQ4\|+\|2 | MISPLSELIASSFSLVRDSTFFSRTKS- | SEQ ID No. 247 |
| >SIP42_2\|PQ4\|+\|3 | M1RTGHASTVIFSFNPGGISSTLSS- | SEQ ID No. 248 |
| >SIP42_3\|PQ4\|-\|1 | | SEQ ID No. 249 |
| >SIP42_4\|PQ4\|-\|2 | MRFTTSSMCHNYGSV- | SEQ ID No. 250 |
| >SIP42_5\|PQ4\|-\|2 | MRIRGRIQFKGGRLVTPAILNRIVG- | SEQ ID No. 251 |
| >SIP42_6\|PQ4\|-\|3 | MAGTDHGPNDERDSGKIKGFVKSLVELWRP- | SEQ ID No. 252 |
| >SIP131_2\|PQ4\|+\|2 | | SEQ ID No. 253 |
| >SIP131_3\|PQ4\|+\|2 | MLHLPKLSIGLLQV- | SEQ ID No. 254 |
| >SIP131_4\|PQ4\|+\|3 | MQFSNMIDDYGTEVYLNDHEESRRPHSSSLV- | SEQ ID No. 255 |
| >SIP131_5\|PQ4\|-\|2 | | SEQ ID No. 256 |
| >SIP140_0\|PQ4\|+\|1 | MTGISCDIWMTSFLQRTIG- | SEQ ID No. 257 |
| >SIP140_3\|PQ4\|-\|1 | | SEQ ID No. 258 |
| >SIP140_4\|PQ4\|-\|2 | MAMFNISEAHHR- | SEQ ID No. 259 |
| >SIP140_5\|PQ4\|-\|2 | MSSRYRRRSRS- | SEQ ID No. 260 |
| >SIP61_3\|PQ4\|+\|3 | MQPFVGTRSTLHVLAETPLHP- | SEQ ID No. 261 |
| >SIP61_4\|PQ4\|-\|1 | MQGMDVTFRKGERNGCDLSEGCKERM- | SEQ ID No. 262 |
| >SIP61_5\|PQ4\|-\|3 | MQKTDVTDVTFWKDARNGCDLSEG- | SEQ ID No. 263 |
| >SIP61_6\|PQ4\|-\|3 | MLWEASIRPLYKGGRPRPLE- | SEQ ID No. 264 |
| >SIP1_1\|PQ4\|+\|3 | MTGIKRRRRLLF- | SEQ ID No. 265 |
| >SIP1_2\|PQ4\|-\|2 | MFIFDGLKFKKI- | SEQ ID No. 266 |
| >SIP116_0\|PQ4\|+\|2 | MKFMDLVDPILVHGFSAEI- | SEQ ID No. 267 |
| >SIP116_1\|PQ4\|+\|3 | MEALRPKSCSSTPSTFH- | SEQ ID No. 268 |
| >SIP159_0\|PQ4\|-\|1 | MHELILLYTKQKRPKTIKAPGIPAPTAIA- | SEQ ID No. 269 |
| >SIP159_1\|PQ4\|-\|3 | MGLSVVDLWGWDETTPFNA- | SEQ ID No. 270 |
| >SIP135_0\|PQ4\|+\|2 | MATTRLLTCARERRRILRPSKRDQRKNKRDT- | SEQ ID No. 271 |
| >SIP171_0\|PQ4\|+\|1 | MVISFAGYSPKESAR- | SEQ ID No. 272 |
| >SIP171_1\|PQ4\|+\|2 | MISPRVLGIHL- | SEQ ID No. 273 |
| >SIP171_2\|PQ4\|+\|2 | MDRSKPLRIAYYGK- | SEQ ID No. 274 |
| >SIP171_3\|PQ4\|+\|3 | MTFLIWTDRSP- | SEQ ID No. 275 |
| >SIP171_5\|PQ4\|-\|3 | MIVTRKLGIGSGSSYLP- | SEQ ID No. 276 |
| >SIP70_1\|PQ4\|+\|2 | MTAQRMSTIGMKWIAFSADLNRDLFFFPG- | SEQ ID No. 277 |
| >SIP10_0\|PQ4\|-\|1 | MLNWLTEKC- | SEQ ID No. 278 |
| >SIP10_1\|PQ4\|-\|1 | MLNCVLAGKC- | SEQ ID No. 279 |
| >SIP10_3\|PQ4\|-\|2 | MLNCVLAENVELRFGGKMLNVVLAGKC- | SEQ ID No. 280 |
| >SIP10_4\|PQ4\|-\|2 | MLIVVFGGKMLNVFFGGKMLNCVLAGKC- | SEQ ID No. 281 |
| >SIP10_5\|PQ4\|-\|3 | MLNCVFDGKMLNCVLVEKC- | SEQ ID No. 282 |
| >SIP10_6\|PQ4\|-\|3 | MLNCVLAEKC- | SEQ ID No. 283 |
| >SIP10_7\|PQ4\|-\|3 | MSFWRENVECRFDGKMLNVVFGRKMLNCFLAGKC- | SEQ ID No. 284 |
| >SIP10_8\|PQ4\|-\|3 | MLIWFGGKMLNVFFGGKM- | SEQ ID No. 285 |
| >SIP98_1\|PQ4\|-\|1 | MVVVSVRDSKEVHDGRVMVEAMISWLRV- | SEQ ID No. 286 |
| >SIP98_2\|PQ4\|-\|3 | MYWIIVGFLARFGDAVEC- | SEQ ID No. 287 |
| >SIP169_2\|PQ4\|+\|3 | MLMLLKHDLFL- | SEQ ID No. 288 |
| >SIP169_3\|PQ4\|-\|1 | MQKLLHGGLFGILS- | SEQ ID No. 289 |
| >SIP9_1\|PQ4\|-\|2 | MIFAMTKFQNGKKKKKQIPKWVELMGCDA- | SEQ ID No. 290 |
| >SIP9_3\|PQ4\|-\|3 | MEKKKKNKFQNGWN- | SEQ ID No. 291 |
| >SIP51_0\|PQ4\|-\|1 | MRASKSTKKVFYSIIELSFKGGQETLCCVRMSR- | SEQ ID No. 292 |
| >SIP51_2\|PQ4\|-\|2 | | SEQ ID No. 293 |
| >SIP151_0\|PQ4\|-\|2 | MQFPVITQCY- | SEQ ID No. 294 |
| >SIP75_4\|PQ4\|+\|3 | MSTTKQSGVGRLMLLRFFSLNCFGCYVVGILIDLCH- | SEQ ID No. 295 |
| >SIP75_5\|PQ4\|-\|1 | | SEQ ID No. 296 |
| >SIP14_4\|PQ4\|-\|1 | MSYGLTQLILLNGLDQPITHLI- | SEQ ID No. 297 |
| >SIP14_5\|PQ4\|-\|3 | MSLKLNELWVDPTHFVKWVGSTHNSFNLMG- | SEQ ID No. 298 |
| >SIP50_0\|PQ4\|+\|1 | MVDTGPVRSVLYS- | SEQ ID No. 299 |
| >SIP50_1\|PQ4\|+\|2 | MLRFLWWILVLLGRYCTVDGAAT- | SEQ ID No. 300 |
| >SIP50_4\|PQ4\|+\|3 | | SEQ ID No. 301 |
| >SIP50_5\|PQ4\|-\|2 | MSDPIACLECQHET- | SEQ ID No. 302 |
| >SIP50_6\|PQ4\|-\|3 | MKPNYDSGGEDLK- | SEQ ID No. 303 |
| >SIP20_1\|PQ4\|+\|3 | MAFKSRKIGKNKSNIKENKRKGKEKSNQWVASH- | SEQ ID No. 304 |
| >SIP20_2\|PQ4\|-\|1 | MPLGTMSFKSRGGSY- | SEQ ID No. 305 |
| >SIP62_0\|PQ4\|+\|2 | MNAGRDRYSVPLHRKSNNPS- | SEQ ID No. 306 |
| >SIP119_0\|PQ4\|-\|2 | MYLSLMILQVMQSY- | SEQ ID No. 307 |
| >SIP77_2\|PQ4\|-\|1 | MHEPVAVDGPLGLKPGLAL- | SEQ ID No. 308 |
| >SIP77_3\|PQ4\|-\|2 | MSLWRLMDHWASSLALLCRLCSIEPERPLMCI- | SEQ ID No. 309 |
| >SIP37_0\|PQ4\|+\|1 | | SEQ ID No. 310 |
| >SIP75_6\|PQ4\|-\|2 | MAEIYQNPNNITSKTVKTKET- | SEQ ID No. 311 |
| >SIP37_2\|PQ4\|-\|2 | MIFTRLRLCYCNYYARKNLYCNFGYQMPFF- | SEQ ID No. 312 |
| >SIP37_3\|PQ4\|-\|3 | MSTNQEKAKPEGDSYCFNHSEKAQL- | SEQ ID No. 313 |
| >SIP37_4\|PQ4\|-\|3 | MVCATNGERRKRTICV- | SEQ ID No. 314 |
| >SIP37_5\|PQ4\|-\|3 | MREKICTVILATKCHSSKQSDLC- | SEQ ID No. 315 |
| >SIP139_0\|PQ4\|+\|2 | MATPFFPIDPLSFSQLTHPRETDFAES- | SEQ ID No. 316 |
| >SIP139_2\|PQ4\|+\|3 | MMISMILPWLLLFFQ- | SEQ ID No. 317 |
| >SIP143_0\|PQ4\|+\|1 | MYKMYACMDVCMNACMDVWMYA- | SEQ ID No. 318 |
| >SIP143_1\|PQ4\|+\|1 | MHRYFVLGCRFSGFVKLDVEEADQVLIVLCGLT- | SEQ ID No. 319 |
| >SIP143_2\|PQ4\|+\|1 | MLLYLVYQVGTGTYQ- | SEQ ID No. 320 |
| >SIP143_4\|PQ4\|+\|2 | | SEQ ID No. 321 |
| >SIP143_5\|PQ4\|+\|2 | MWKRLIKYSSCFVG- | SEQ ID No. 322 |
| >SIP143_7\|PQ4\|+\|3 | | SEQ ID No. 323 |
| >SIP41_0\|PQ4\|+\|1 | | SEQ ID No. 324 |
| >SIP122_2\|PQ4\|+\|2 | MRSVRFLDSILSYNNFVLSN- | SEQ ID No. 325 |
| >SIP122_3\|PQ4\|-\|3 | MFGLRLLRRNLLQCF- | SEQ ID No. 326 |
| >SIP150_1\|PQ4\|-\|1 | MQVRGELFNRCLV- | SEQ ID No. 327 |
| >SIP150_2\|PQ4\|-\|2 | | SEQ ID No. 328 |
| >SIP150_4\|PQ4\|-\|3 | MSCLEKALRYCLRYQETSSNVSIL- | SEQ ID No. 329 |
| >SIP141_0\|PQ4\|+\|1 | MLKDISMRQETRPLTKQNFT- | SEQ ID No. 330 |
| >SIP141_1\|PQ4\|+\|3 | MFMFEFCSLWMCFEFECIVFE- | SEQ ID No. 331 |
| >S1P141_2\|PQ4\|+\|3 | MQITYRDDTINMRIE- | SEQ ID No. 332 |
| >SIP141_3\|PQ4\|-\|1 | | SEQ ID No. 333 |
| >SIP141_4\|PQ4\|-\|2 | | SEQ ID No. 334 |
| >SIP39_0\|PQ4\|+\|1 | MNIGTLLLMFYVSKR- | SEQ ID No. 335 |
| >SIP39_1\|PQ4\|+\|1 | MNRLVFLAKEVNNH- | SEQ ID No. 336 |
| >SIP39_2\|PQ4\|+\|2 | MLANAEVSIYKSLSRTL- | SEQ ID No. 337 |
| >SIP39_3\|PQ4\|+\|2 | MFMSSGFIDSTKRVSSI- | SEQ ID No. 338 |
| >SIP39_4\|PQ4\|-\|1 | MYHKDSIFLLLLVVL- | SEQ ID No. 339 |
| >SIP39_6\|PQ4\|-\|2 | | SEQ ID No. 340 |
| >SIP39_7\|PQ4\|-\|3 | MFKLWGFLTQG- | SEQ ID No. 341 |
| >SIP39_8\|PQ4\|-\|3 | MLIFPLECTSTRLK- | SEQ ID No. 342 |
| >SIP39_9\|PQ4\|-\|3 | | SEQ ID No. 343 |
| >SIP34_2\|PQ4\|-\|3 | MVFIESIFKDWRAEEAS- | SEQ ID No. 344 |
| >SIP90_1\|PQ4\|+\|1 | MLTQILPKHLQFKTNSLTTRfRDTP- | SEQ ID No. 345 |
| >SIP90_2\|PQ4\|-\|1 | | SEQ ID No. 346 |
| >SIP90_3\|PQ4\|-\|2 | MFFFHSLSSCDYRS- | SEQ ID No. 347 |
| >SIP90_4\|PQ4\|-\|3 | MVCFSFIHSLHVIIDHDGSNYPFCKTFIRRSYFIFNDLSV- | SEQ ID No. 348 |
| >SIP74_0\|PQ4\|+\|1 | MMTYLSFSEALVIYHPHIISS- | SEQ ID No. 349 |
| >SIP11_1\|PQ4\|+\|3 | MTKVRSISSLVMITTPKATSSIIPIRRRQLLVEI- | SEQ ID No. 350 |
| >SIP11_2\|PQ4\|+\|3 | | SEQ ID No. 351 |
| >SIP11_4\|PQ4\|-\|2 | MWKEVIIFFI- | SEQ ID No. 352 |
| >SIP11_5\|PQ4\|-\|2 | MSMSNTSKDS- | SEQ ID No. 353 |
| >SIP11_6\|PQ4\|-\|2 | MRNTGLSSAPSFLWCFSGDTFCWRSI- | SEQ ID No. 354 |
| >SIP11_7\|PQ4\|-\|3 | MAFLQVFFSMASWKSMGSHSANKQNKVRFS- | SEQ ID No. 355 |
| >SIP11_8\|P44\|-\|3 | MKIYFSLLSSSLLRFSSGT- | SEQ ID No. 356 |
| >SIP11_9\|PQ4\|-\|3 | MLLAISRIVLFFLSATPFC- | SEQ ID No. 357 |
| >SIP57_2\|PQ4\|-\|2 | MLRRMILQRNR- | SEQ ID No. 358 |
| >SIP128_1\|PQ4\|+\|2 | MSRANISQSI- | SEQ ID No. 359 |
| >SIP128_3\|PQ4\|-\|1 | MFARLMIKVS- | SEQ ID No. 360 |
| >SIP128_4\|PQ4\|-\|2 | MSLSILRKLGPHR- | SEQ ID No. 361 |
| >SIP66_0\|PQ4\|+\|1 | MNHSNAPRICSPNLITISRKKAIIFFI- | SEQ ID No. 362 |
| >SIP66_1\|PQ4\|+\|2 | MKNCWKLPSTGLP- | SEQ ID No. 363 |
| >SIP66_2\|PQ4\|+\|3 | MSLHICSSKQSCPIQ- | SEQ ID No. 364 |
| >SIP66_3\|PQ4\|+\|3 | MTREQQPLYVLFPSMLACMLV- | SEQ ID No. 365 |
| >SIP66_4\|PQ4\|-\|2 | | SEQ ID No. 366 |
| >SIP66_5\|PQ4\|-\|2 | MLVDLLLDWTGLF- | SEQ ID No. 367 |
| >SIP66_6\|PG4\|-\|3 | MEGKRTYNGCCSRVI- | SEQ ID No. 368 |
| >SIP16_0\|PQ4\|+\|3 | | SEQ ID No. 369 |
| >SIP167_0\|PQ4\|+\|1 | | SEQ ID No. 370 |
| >SIP167_1\|PQ4\|+\|3 | MNVVNKKLIWPSWSKAPD- | SEQ ID No. 371 |
| >SIP167_2\|PQ4\|-\|3 | | SEQ ID No. 372 |
| >SIP167_3\|PQ4\|-\|3 | | SEQ ID No. 373 |
| >SIP85_\|PQ4\|+\|1 | MALRIPKLYLVLPV- | SEQ ID No. 374 |
| >SIP85_5\|PQ4\|-\|2 | MRRIPEPVQITQGMQI- | SEQ ID No. 375 |
| >SIP156_1\|PQ4\|+\|2 | MDCFNANTNQRREATERNRTCKVTSGLSASQ- | SEQ ID No. 376 |
| >SIP156_2\|PQ4\|-\|1 | MCFRNPAVEI- | SEQ ID No. 377 |
| >SIP156_4\|PQ4\|-\|2 | MGNSCASETQL- | SEQ ID No. 378 |
| >SIP121_0\|PQ4\|-\|1 | | SEQ ID No. 379 |
| >SIP121_1\|PQ4\|-\|1 | MLMVLDKFCSSSLRN- | SEQ ID No. 380 |
| >SIP125_1\|PQ4\|+\|2 | MSLGHSQRQSIHILLHLYCLHWLRYPRLD- | SEQ ID No. 381 |
| >SIP125_3\|PQ4\|-\|1 | MQQDVYALSLRVS- | SEQ ID No. 382 |
| >SIP125_4\|PQ4\|-\|2 | MMNSNFPFICFRPSDFHGRHCIQSVNYIKRLDQ- | SEQ ID No. 383 |
| >SIP175_1\|PQ4\|+\|1 | MYCPITIDQIHQTRNSIPHCC- | SEQ ID No. 384 |
| >SIP175_2\|PQ4\|+\|2 | MRRQIFSFLGLTTLSSNLLVCIAP- | SEQ ID No. 385 |
| >SIP175_3\|PQ4\|-\|3 | MGQYIPKGSKKESLSRES- | SEQ ID No. 386 |
| >SIP25_2\|PQ4\|+\|3 | MKIHDQNSPISTIDSVNEQLPFLITDSNPFAQ- | SEQ ID No. 387 |
| >SIP25_3\|PQ4\|-\|1 | MGDFNSAAVMIH- | SEQ ID No. 388 |
| >SIP25_4\|PQ4\|-\|2 | MVHGFTPHTRITGQKDLSQ- | SEQ ID No. 389 |
| >SIP25_5\|PQ4\|-\|3 | MDLLLILELLGKRI- | SEQ ID No. 390 |
| >SIP25_6\|PQ4\|-\|3 | MNFHGRFQLGGGDDSLTSFQS- | SEQ ID No. 391 |
| >SIP69_0\|PQ4\|-\|1 | MKKTLIIMPGKNQNQRKKEQREI- | SEQ ID No. 392 |
| >SIP69_1\|PQ4\|-\|2 | MIDSSPFSNYAIFNNV- | SEQ ID No. 393 |
| >SIP137_0\|PQ4\|+\|1 | MPHQTQREEQDLLYL- | SEQ ID No. 394 |
| >SIP137_3\|PQ4\|+\|2 | | SEQ ID No. 395 |
| >SIP137_4\|PQ4\|-\|1 | MLHLLYFPCL- | SEQ ID No. 396 |
| >S1P137_5\|PQ4\|-\|1 | MWHLCMVUSLRSIWNYVLSFLVKAINTSF- | SEQ ID No. 397 |
| >SIP137_6\|PQ4\|-\|2 | MSFLNRYLSEKGRCCICYIFPVYKYKRSCSSL- | SEQ ID No. 398 |
| >SIP118_0\|PQ4\|+\|1 | | SEQ ID No. 399 |
| >SIP118_3\|PQ4\|-\|1 | MLFPEACFELFPTRLSIT- | SEQ ID No. 400 |
| >SIP118_4\|PQ4\|-\|2 | MGEDTNISLAFCQKRMPQSIQVKDMKMMRVRL- | SEQ ID No. 401 |
| >SIP35_1\|PQ4\|-\|2 | MKKARLQSPLCSRVNNKIAAYQSHTV- | SEQ ID No. 402 |
| >SIP80_1\|PQ4\|+\|2 | MAYFRSASRNSPTFLNR- | SEQ ID No. 403 |
| >SIP80_3\|PQ4\|-\|3 | MHRLLLIHPN- | SEQ ID No. 404 |
| >SIP80_4\|PQ4\|-\|3 | MLGSFVKRYGNTP- | SEQ ID No. 405 |
| >SIP110_0\|PQ4\|+\|1 | MNKRRPPTYNKMERLMESKYMMIWILKMKMEVFKR- | SEQ ID No. 406 |
| >SIP110_2\|PQ4\|-\|1 | MVIGYLLNTSIFILSIHIIMYLDSINLSILL- | SEQ ID No. 407 |
| >SIP28_0\|PQ4\|+\|1 | | SEQ ID No. 408 |
| >SIP28_1\|PQ4\|+\|1 | MNKKRIISGTSIVVQVITCAGLKVCSRSLMNR- | SEQ ID No. 409 |
| >SIP28_2\|PQ4\|+\|1 | MEIINLFPTFTIFRA- | SEQ ID No. 410 |
| >SIP28_3\|PQ4\|+\|1 | MTLHNVLRCVTKRSLGYGIFDSDI- | SEQ ID No. 411 |
| >SIP28_4\|PQ4\|+\|1 | MCVVQSSRNHLVKVITSFSLLMIFQEKHGYIF- | SEQ IU No. 412 |
| >SIP28_5\|PQ4\|+\|1 | MLMYRMRSGAN- | SEQ ID No. 413 |
| >SIP28_6\|PQ4\|+\|1 | MTKVRSISSLVMITTPKATSSIIPIRRRQLLVEI- | SEQ ID No. 414 |
| >SIP28_7\|PQ4\|+\|1 | | SEQ ID No. 415 |
| >SIP28_8\|PQ4\|+\|1 | MPWMKRSNQYKRMTHGS- | SEQ ID No. 416 |
| >SIP28_9\|PQ4\|+\|2 | MLQLWEVWTLCF- | SEQ ID No. 417 |
| >SIP28_10\|PQ4\|+\|2 | MASSIRTSKFWRIGVAFKEGNGERATLYKSSKSSV- | SEQ ID No. 418 |
| >SIP28_11\|PQ4\|+\|2 | | SEQ ID No. 419 |
| >SIP28_12\|PQ4\|+\|2 | MGIFFERKIRGVRNFQKV- | SEQ ID No. 420 |
| >SIP28_13\|PQ4\|+\|2 | MGVNFTSKWTQDNWREVGV- | SEQ ID No. 421 |
| >SIP28_14\|PQ4\|-\|1 | | SEQ ID No. 422 |
| >SIP28_16\|PQ4\|-\|2 | MWKEVIIFFI- | SEQ ID No. 423 |
| >SIP28_17\|PQ4\|-\|2 | MSMSNTSKDS- | SEQ ID No. 424 |
| >SIP28_18\|PQ4\|-\|2 | MRNTGLSSAPSFLWCFSGDTFCWRSI- | SEQ ID No. 425 |
| >SIP28_19\|PQ4\|-\|2 | MGFKLFENFEHLGFFFQKIYPCFS- | SEQ ID No. 426 |
| >SIP28_20\|PQ4\|-\|2 | MISILQSNENVSFTFYLHLRFIS- | SEQ ID No. 427 |
| >SIP28_21\|PQ4\|-\|2 | MILFLFIFLVASH- | SEQ ID No. 428 |
| >SIP28_22\|PQ4\|-\|2 | MSKLPTIVAFDT- | SEQ ID No. 429 |
| >SIP28_23\|PQ4\|-\|2 | | SEQ ID No. 430 |
| >SIP28_2\|PQ4\|-\|3 | MAFLQVFFSMASWKSMGSHSANKQNKVRFS- | SEQ ID No. 431 |
| >SIP28_25\|PQ4\|-\|3 | MKIYFSLLSSSLLRFSSGT- | SEQ ID No. 432 |
| >SIP28_26\|PQ4\|-\|3 | MLLAISRIVLFFLSATPFC- | SEQ ID No. 433 |
| >SIP28_27\|PQ4\|-\|3 | | SEQ ID No. 434 |
| >SIP28_28\|PQ4\|-\|3 | MSFRMLRTNILFFD1GTLVMRLLSWSLIERLLSFNLIS- | SEQ ID No. 435 |
| >SIP28_29\|PQ4\|-\|3 | MRMFPLPFTSIFDSSPKATFPLTDSSSSANILFLPHM- | SEQ ID No. 436 |
| >SIP28_30\|PQ4\|-\|3 | MISSFFFFFSS- | SEQ ID No. 437 |
| >SIP49_0\|PQ4\|-\|2 | | SEQ ID No. 438 |
| >SIP164_1\|PQ4\|-\|3 | MRTFLGTYDKCLAVSENDFSDNPTVCGAGCNG- | SEQ ID No. 439 |
| >SIP136_0\|PQ4\|-\|2 | MIKQIVISSWHKMSRHCDPFLHHTR- | SEQ ID No. 440 |
| >SIP12_2\|PQ4\|+\|2 | MTERVQWLCFTLETRLSHGCRRNNQLSLYPLVKRSM- | SEQ ID No. 441 |
| >SIP12_3\|PQ4\|+\|2 | MIEVNTLTHAITTLESVLAKRTCNWSM- | SEQ ID No. 442 |
| >SIP12_4\|PQ4\|+\|3 | MDVEETTNCHSIHL- | SEQ ID No. 443 |
| >SIP12_5\|PQ4\|+\|3 | | SEQ ID No. 444 |
| >SIP12_6\|PQ4\|-\|2 | | SEQ ID No. 445 |
| >SIP12_8\|PQ4\|-\|3 | | SEQ ID No. 446 |
| >SIP38_1\|PQ4\|+\|2 | | SEQ ID No. 447 |
| >SIP38_3\|PQ4\|-\|1 | | SEQ ID No. 448 |
| >SIP38_4\|PQ4\|-\|1 | MLLFRINLHTIRFNSFKSNKLKQRL- | SEQ ID No. 449 |
| >SIP55_1\|PQ4\|-\|1 | | SEQ ID No. 450 |
| >SIP55_2\|PQ4\|-\|2 | MREWVEMGGFHQIF- | SEQ ID No. 451 |
| >SIP55_3\|PQ4\|-\|3 | MGRVEGSCIWFGRAVDNWRRLYHHLESG- | SEQ ID No. 452 |
| >SIP73_1\|PQ4\|+\|2 | MLLAFFTVVETSMICFVDQILLPHSLYDQRK- | SEQ ID No. 453 |
| >SIP73_2\|PQ4\|+\|3 | MIKESEILYP- | SEQ ID No. 454 |
| >SIP73_3\|PQ4\|-\|2 | MDNRMRLIMGTKFHFL- | SEQ ID No. 455 |
| >SIP73_4\|PQ4\|-\|3 | MNITGTEWITECD- | SEQ ID No. 456 |
| >SIP73_5\|PQ4\|-\|3 | MSEAKEFGQRNKS- | SEQ ID No. 457 |
| >SIP105_0\|PQ4\|-\|1 | | SEQ ID No. 458 |
| >SIP13_0\|PQ4\|+\|1 | MYILKYGHFS- | SEQ ID No. 459 |
| >SIP13_1\|PQ4\|+\|1 | MTILSLDRSRRAGGSIRIVYGFAKYGS- | SEQ ID No. 460 |
| >SIP13_2\|PQ4\|+\|1 | MDIFYPIYKRYISD- | SEQ ID No. 461 |
| >SIP13_3\|PQ4\|+\|2 | MDISLDTIYLVMLKNLGYNVTIV- | SEQ ID No. 462 |
| >SIP13_6\|PQ4\|-\|2 | MNVLICFLFRCVCSFLVIYILQFS- | SEQ ID No. 463 |
| >SIP63_0\|PQ4\|+\|2 | MHGTFRFARSCLYPLQLDSTI- | SEQ ID No. 464 |
| >SIP63_1\|PQ4\|+\|3 | | SEQ ID No. 465 |
| >SIP154_0\|PQ4\|+\|3 | MGSGASGPVRSSQSSQAGGRFNDADPIAIDYGKY- | SEQ ID No. 466 |
| >SIP154_1\|PQ4\|-\|1 | | SEQ ID No. 467 |
| >SIP154_3\|PQ4\|-\|3 | MKTNKNTKYII- | SEQ ID No. 468 |
| >SIP53_1\|PQ4\|-\|2 | | SEQ ID No. 469 |
| >SIP94_2\|PQ4\|+\|3 | MDVQLLHQQSYSS- | SEQ ID No. 470 |
| >SIP94_3\|PQ4\|-\|2 | MNAGRGRYTFLHTYDEYDCWWSS- | SEQ ID No. 471 |
| >SIP2_0\|PQ4\|+\|2 | MEKVFKCFERVF- | SEQ ID No. 472 |
| >SIP2_1\|PQ4\|-\|1 | MTLHNVLRCVTKRSLGYGIFDSDI- | SEQ ID No. 473 |
| >SIP2_2\|PQ4\|-\|2 | MASSIRTSKFWRIGVAFKEGNGERATLYKSSKARL- | SEQ ID No. 474 |
| >SIP2_3\|PQ4\|-\|3 | | SEQ ID No. 475 |
| >SIP95_0\|PQ4\|+\|3 | MFPSMSQCSQRATMIIGVYE- | SEQ ID No. 476 |
| >SIP95_1\|PQ4\|+\|3 | MKWFLKLKRMV- | SEQ ID No. 477 |
| >SIP95_3\|PQ4\|-\|1 | MKPFSTISHTSCAPRIAFIRRLQLS- | SEQ ID No. 478 |
| >SIP95_4\|PQ4\|-\|2 | | SEQ ID No. 479 |
| >SIP95_5\|PQ4\|-\|3 | MEGNIVTRHL- | SEQ ID No. 480 |
| >SIP95_6\|PQ4\|-\|3 | MEKVLKCFERVF- | SEQ ID No. 481 |
| >SIP68_0\|PQ4\|+\|1 | MCKVPTKTIIIKVSFFLYKKIK- | SEQ ID No. 482 |
| >SIP68_1\|PQ4\|-\|1 | MMMVLVGTLHITYL- | SEQ ID No. 483 |
| >SIP68_2\|PQ4\|-\|1 | MPAPIIPIDLCLLLIVFGFFSFLI- | SEQ ID No. 484 |
| >SIP68_4\|PQ4\|-\|3 | | SEQ ID No. 485 |
| >SIP54_0\|PQ4\|+\|1 | MISSFLIGLEKMARSLPLCTSRYRSHFFPSKRN- | SEQ ID No. 486 |
| >SIP54_2\|PQ4\|-\|2 | MRSIAGGAQRK- | SEQ ID No. 487 |
| >SIP157_0\|PQ4\|+\|3 | MNTAPDTLPQRVTV- | SEQ ID No. 488 |
| >SIP157_1\|PQ4\|+\|3 | MLSPLTLSLTQQ- | SEQ ID No. 489 |
| >SIP157_2\|PQ4\|-\|2 | | SEQ ID No. 490 |
| >SIP161_1\|PQ4\|-\|1 | MNREPIVDLLG- | SEQ ID No. 491 |
| >SIP161_2\|PQ4\|-\|2 | MTTVNRDLDR- | SEQ ID No. 492 |
| >SIP3_0\|PQ4\|+\|1 | MISILQSNKNVSFTFYLHLRFIS- | SEQ ID No. 493 |
| >SIP3_1\|PQ4\|+\|1 | MILFLFIFLVASH- | SEQ ID No. 494 |
| >SIP3_2\|PQ4\|+\|2 | MFPLPFTSIFDSSPKATFPLTDSSSSANILFLPHM- | SEQ ID No. 495 |
| >SIP3_3\|PQ4\|-\|3 | MLLNVKLLATKHLRRRPTTLKKKFKKKTCY- | SEQ ID No. 496 |
| >SIP3_4\|PQ4\|-\|3 | MNKKRIISGTSIVVQVITCAGEKVCSRSLMNR- | SEQ ID No. 497 |
| >SIP107_0\|PQ4\|+\|3 | MMVHSTIQDNQRFCNIGSFRDLFSRSESKSMIVI- | SEQ ID No. 498 |
| >SIP107_1\|PQ4\|-\|1 | | SEQ ID No. 499 |
| >SIP96_0\|PQ4\|+\|1 | MGDLTESPRVGSLFSFFDFCPLFFVKNV- | SEQ ID No. 500 |
| >SIP96_4\|PQ4\|-\|2 | MRSLASLSKSNRVIHS- | SEQ ID No. 501 |
| >SIP71_1\|PQ4\|-\|1 | MKEINQTKMSFSSIYTFLYWGVMPLFASSLDF- | SEQ ID No. 502 |
| >SIP71_2\|PQ4\|-\|2 | MPCSVRNVRSIFLPVF- | SEQ ID No. 503 |
| >SIP71_3\|PQ4\|-\|3 | MSDQSFFQFFKWIYQVFI- | SEQ ID No. 504 |
| >SIP18_0\|PQ4\|+\|1 | MQCMPCGHHHLS- | SEQ ID No. 505 |
| >SIP18_2\|PQ4\|+\|2 | MWKIRRLWKTKRAI- | SEQ ID No. 506 |
| >SIP18_4\|PQ4\|-\|1 | MNLVYSFGSSVLKDSAYVLEM- | SEQ ID No. 507 |
| >SIP18_5\|PQ4\|-\|2 | MMSTRHALHSSRTDLLT- | SEQ ID No. 508 |
| >SIP18_6\|PQ4\|-\|2 | | SEQ ID No. 509 |
| >SIP18_8\|PQ4\|-\|3 | MHCIQVAQIC- | SEQ ID No. 510 |
| >SIP4_1\|PQ4\|+\|1 | METISTFFTDLQ- | SEQ ID No. 511 |
| >SIP4_2\|PQ4\|+\|2 | MVTKKKFLHEIYHWYWEE- | SEQ ID No. 512 |
| >SIP4_3\|PQ4\|+\|3 | MEDKLYFDLFIFDPLAQMFVLSRFDELPFVITWIMIDLEM- | SEQ ID No. 513 |
| >SIP4_4\|PQ4\|-\|1 | | SEQ ID No. 514 |
| >SIP4_5\|PQ4\|-\|2 | | SEQ ID No. 515 |
| >SIP113_0\|PQ4\|+\|1 | | SEQ ID No. 516 |
| >SIP113_2\|PQ4\|-\|1 | MENVNHQKKIRTLVVVVH- | SEQ ID No. 517 |
| >SIP113_3\|PQ4\|-\|3 | MELPFSSRSLSAPFFPKLFPMRSSKGELFCFLCFVGED EIEEKTFSNLAD- | SEQ ID No. 518 |
| >SIP83_0\|PQ4\|+\|1 | | SEQ ID No. 519 |
| >SIP83_1\|PQ4\|+\|2 | | SEQ ID No. 520 |
| >SIP83_2\|PQ4\|+\|2 | MSIAKASPPEYDNDPSLIVTNSPPD- | SEQ ID No. 521 |
| >SIP83_4\|PQ4\|-\|1 | MDRCRILAAMLLQLTLIRILQ- | SEQ ID No. 522 |
| >SIP83_6\|PQ4\|-\|2 | MSVRRGVCYYQRWIWVFWRRCFCN- | SEQ ID No. 523 |
| >SIP83_7\|PQ4\|-\|3 | | SEQ ID No. 524 |
| >SIP115_1\|PQ4\|+\|3 | MVSKDQQMLFKEVENYICLT- | SEQ ID No. 525 |
| >SIP115_2\|PQ4\|-\|1 | MRDVNRLQLWQTP- | SEQ ID No. 526 |
| >SIP115_3\|PQ4\|-\|2 | MFELMSSIYSSPPP- | SEQ ID No. 527 |
| >SIP115_4\|PQ4\|-\|2 | MLIDCNSGRLHNHSGTWPSI- | SEQ ID No. 528 |
| >SIP115_6\|PQ4\|-\|3 | MDPGICLNLCQAYIVLHLLE- | SEQ ID No. 529 |
| >SIP29_1\|PQ4\|-\|1 | MLGDDMYLDR- | SEQ ID No. 530 |
| >SIP29_2\|PQ4\|-\|2 | MTCTLIGKNLRTSHQVLMHIDRAILRQCSGTLLDL- | SEQ ID No. 531 |
| >SIP127_0\|PQ4\|+\|1 | MANLRSKAKPI- | SEQ ID No. 532 |
| >SIP127_1\|PQ4\|+\|1 | | SEQ ID No. 533 |
| >SIP127_2\|PQ4\|+\|3 | MGNYLGTFINGEFKK- | SEQ ID No. 534 |
| >SIP127_3\|PQ4\|-\|1 | | SEQ ID No. 535 |
| >SIP127_4\|PQ4\|-\|1 | MIGNLFKLVLLYFLNSPLINVPR- | SEQ ID No. 536 |
| >SIP40_0\|PQ4\|+\|1 | MNIMKPFRRCRHSSCCKL- | SEQ ID No. 537 |
| >SIP40_3\|PQ4\|+\|3 | MPPLELLQVVSAI- | SEQ ID No. 538 |
| >SIP40_4\|PQ4\|-\|3 | MWLDCTYNLQQLEWRHLLKGFMMFM- | SEQ ID No. 539 |
| >SIP114_0\|PQ4\|+\|2 | MEDNILGICVSNETLF- | SEQ ID No. 540 |
| >SIP114_1\|PQ4\|-\|1 | MAPEFSMDPSRLLVTGFTS- | SEQ ID No. 541 |
| >SIP114_2\|PQ4\|-\|3 | | SEQ ID No. 542 |
| >SIP142_0\|PQ4\|-\|2 | MLFIYKFYWHMQIYVEVKRKTKQYAIVEMSFLGNG- | SEQ ID No. 543 |
| >SIP142_1\|PQ4\|-\|3 | MKCYLYINSIGTCKYTLKSKEKQNSTQ- | SEQ ID No. 544 |
| >SIP142_2\|PQ4\|-\|3 | MASIKNIGAKRKIEVKIFCLMT- | SEQ ID No. 545 |
| >SIP82_0\|PQ4\|+\|1 | MKSECVYMMS- | SEQ ID No. 546 |
| >SIP82_2\|PQ4\|-\|1 | MVITYTFFVPL- | SEQ ID No. 547 |
| >SIP82_3\|PQ4\|-\|1 | | SEQ ID No. 548 |
| >SIP82_4\|PQ4\|-\|3 | MYTHSDFISN- | SEQ ID No. 549 |
| >SIP170_0\|PQ4\|+\|1 | | SEQ ID No. 550 |
| >SIP170_1\|PQ4\|-\|3 | MAGIPLLRPITT- | SEQ ID No. 551 |
| >SIP24_0\|PQ4\|+\|1 | MTSLAFSRSRIETPQVSR- | SEQ ID No. 552 |
| >SIP24_1\|PQ4\|+\|3 | MFLTSALGTLVMIFDVKC- | SEQ ID No. 553 |
| >SIP24_2\|PQ4\|-\|1 | | SEQ ID No. 554 |
| >SIP81_1\|PQ4\|+\|3 | MSSRNTFISV- | SEQ ID No. 555 |
| >SIP81_2\|PQ4\|-\|2 | MKVFLEDMPQPSCCKKEKQILKSAR- | SEQ ID No. 556 |
| >SIP67_0\|PQ4\|-\|1 | MYCKQQEEKKTTLREMNWRVDVKSSQAVKKLRID- | SEQ ID No. 557 |
| >SIP111_1\|PQ4\|-\|2 | | SEQ ID No. 558 |
| >SIP126_0\|PQ4\|+\|1 | MHDSVSRILVV- | SEQ ID No. 559 |
| >SIP126_1\|PQ4\|+\|1 | MSSLCILGFQRRVNLRTFLNLRRA- | SEQ ID No. 560 |
| >SIP126_3\|PQ4\|+\|3 | MIGENQITSLDTLEHIF- | SEQ ID No. 561 |
| >SIP126_4\|PQ4\|-\|1 | MLYLKNISLSYLT- | SEQ ID No. 562 |
| >SIP126_5\|PQ4\|-\|1 | MSSVLQFHFYLPKLVREFFQNFAGLWLLSFRR- | SEQ ID No. 563 |
| >SIP126_7\|PQ4\|-\|2 | MCSRVSREVIWFSPIIYCYQGS- | SEQ ID No. 564 |
| >SIP166_0\|PQ4\|+\|2 | MECIPQNSHGVPLSLLTTQALHLTILWLVTGQT- | SEQ ID No. 565 |
| >SIP166_1\|PQ4\|+\|2 | MGSTSLLLAKGLGISTRTRILETSTLLTQ- | SEQ ID No. 566 |
| >SIP166_3\|PQ4\|-\|1 | MKMKRMAFCAKNASVLHHQLHTGVIR- | SEQ ID No. 567 |
| >SIP166_4\|PQ4\|-\|1 | MRLPRIVRLKESFCWHCE- | SEQ ID No. 568 |
| >SIP166_5\|PQ4\|-\|2 | MLVSCITNSTLVL- | SEQ ID No. 569 |
| >SIP166_6\|PQ4\|-\|2 | MGLHGNFGEYTP- | SEQ ID No. 570 |
| >SIP166_8\|PQ4\|-\|3 | MAHRLCSSLLGQQG- | SEQ ID No. 571 |
| >SIP166_9\|PQ4\|-\|3 | MGILGNTLHEIAQNSEIEGELLLAL- | SEQ ID No. 572 |
| >SIP7_2\|PQ4\|+\|3 | MEEAFSPLHLFPRICYTCLQQR- | SEQ ID No. 573 |
| >SIP7_3\|PQ4\|-\|1 | MILLSLTLLQTCIANAGK- | SEQ ID No. 574 |
| >SIP7_4\|PQ4\|-\|2 | MEHTNISFGKTRVFRGSLCDFP- | SEQ ID No. 575 |

**Table 2. List of OSIP-encoding regions, identified by comparing TARs that were induced by the paraquat treatment as compared to the control treatment.**

| **seqID** | **source** | **type** | **start** | **end** | **attributes** |
|---|---|---|---|---|---|
| Chr1 | PQ4 | transcript | 274401 | 274540 | ID=TAR0; Note=intergenic; |
| Chr1 | PQ4 | transcript | 913399 | 913500 | ID=TAR1; Note=intergenic; |
| Chr1 | PQ4 | transcript | 3360572 | 3360749 | ID=TAR2; Note=EXON; Parent=AT1G10260.1; |
| Chr1 | PQ4 | transcript | 3361044 | 3361249 | ID=TAR3;Note=EXON;Parent=AT1G10260.1; |
| Chr1 | PQ4 | transcript | 3629412 | 3629690 | ID=TAR4;Note=intron; Parent=AT1 G10890.1; |
| Chr1 | PQ4 | transcript | 4627265 | 4627398 | ID=TARS; Note=intergenic; |
| Chr1 | PQ4 | transcript | 5375567 | 5375678 | ID=TAR6; Note=intergenic; |
| Chr1 | PQ4 | transcript | 5626708 | 5626818 | ID=TAR7;Note=EXON;Parent=AT1G16480.1; |
| Chr1 | PQ4 | transcript | 6072230 | 6072445 | ID=TAR8;Note=intergenic; |
| Chr1 | PQ4 | transcript | 6102943 | 6103109 | ID=TAR9;Note=intron;Parent=AT1G17745.1; |
| Chr1 | PQ4 | transcript | 6743224 | 6743473 | ID=TAR10;Note=intergenic; |
| Chr1 | PQ4 | transcript | 7719929 | 7720564 | ID=TAR11;Note=EXON;Parent=AT1G21945.1; |
| Chr1 | PQ4 | transcript | 7721562 | 7722022 | ID=TAR12;Note=EXON;Parent=AT1G21945.1; |
| Chr1 | PQ4 | transcript | 7905709 | 7905992 | ID=TAR13;Note=intron;Parent=AT1G22400.1; |
| Chr1 | PQ4 | transcript | 7985995 | 7986270 | ID=TAR14;Note=intergenic; |
| Chr1 | PQ4 | transcript | 8187998 | 8188142 | ID=TAR15;Note=intron;Parent=AT1G23090.1; |
| Chr1 | PQ4 | transcript | 12123039 | 12123323 | ID=TAR16;Note=EXON;Parent=AT1G33420.1; |
| Chr1 | PQ4 | transcript | 12691540 | 12691657 | ID=TAR17;Note=intergenic; |
| Chr1 | PQ4 | transcript | 13197381 | 13197628 | ID=TAR18;Note=intergenic; |
| Chr1 | PQ4 | transcript | 13842516 | 13842739 | ID=TAR19;Note=EXON;Parent=AT1G36630.1; |
| Chr1 | PQ4 | transcript | 14470563 | 14470779 | ID=TAR20;Note=EXON;Parent=AT1G38423.1; |
| Chr1 | PQ4 | transcript | 15165684 | 15165789 | ID=TAR21;Note=EXON;Parent=AT1G40113.1; |
| Chr1 | PQ4 | transcript | 15564772 | 15564883 | ID=TAR22;Note=EXON;Parent=AT1G41797.1; |
| Chr1 | PQ4 | transcript | 15949322 | 15949433 | ID=TAR23;Note=EXON;Parent=AT1G42490.1; |
| Chr1 | PQ4 | transcript | 17161729 | 17161949 | ID=TAR24;Note=intergenic; |
| Chr1 | PQ4 | transcript | 17467023 | 17467207 | ID=TAR25;Note=intergenic; |
| Chr1 | PQ4 | transcript | 17477421 | 17477530 | ID=TAR26;Note=EXON;Parent=AT1G47565.1; |
| Chr1 | PQ4 | transcript | 17931255 | 17931358 | ID=TAR27;Note=intergenic; |
| Chr1 | PQ4 | transcript | 18017908 | 18019918 | ID=TAR28;Note=EXON;Parent=AT1G48710.1; |
| Chr1 | PQ4 | transcript | 18835941 | 18836116 | ID=TAR29;Note=EXON;Parent=AT1G50810.1; |
| Chr1 | PQ4 | transcript | 21460729 | 21460870 | ID=TAR30;Note=intergenic; |
| Chr1 | PQ4 | transcript | 21748168 | 21748448 | ID=TAR31;Note=intergenic; |
| Chr1 | PQ4 | transcript | 22072961 | 22073361 | ID=TAR32;Note=intron;Parent=AT1G59950.1; |
| Chr1 | PQ4 | transcript | 22175661 | 22175839 | ID=TAR33;Note=EXON;Parent=AT1G60120.1; |
| Chr1 | PQ4 | transcript | 22366650 | 22366757 | ID=TAR34;Note=EXON;Parent=AT1G60750.1; |
| Chr1 | PQ4 | transcript | 22429087 | 22429209 | ID=TAR35;Note=intron;Parent=AT1G60900.1; |
| Chr1 | PQ4 | transcript | 24887782 | 24887916 | ID=TAR36;Note=EXON;Parent=AT1G66725.1; |
| Chr1 | PQ4 | transcript | 25795431 | 25795682 | ID=TAR37;Note=EXON;Parent=AT1G68690.1; |
| Chr1 | PQ4 | transcript | 26350557 | 26350809 | ID=TAR38;Note=EXON;Parent=AT1G69950.1; |
| Chr1 | PQ4 | transcript | 28930709 | 28931032 | ID=TAR39;Note=intergenic; |
| Chr1 | PQ4 | transcript | 28987517 | 28987743 | ID=TAR40;Note=intergenic; |
| Chr1 | PQ4 | transcript | 30108578 | 30108715 | ID=TAR41;Note=EXON;Parent=AT1G80020.1; |
| Chr2 | PQ4 | transcript | 43298 | 43812 | ID=TAR42;Note=EXON;Parent=AT2G01034.1; |
| Chr2 | PQ4 | transcript | 171093 | 171419 | ID=TAR43;Note=intergenic; |
| Chr2 | PQ4 | transcript | 1337229 | 1337369 | ID=TAR44;Note=intergenic; |
| Chr2 | PQ4 | transcript | 1353821 | 1353964 | ID=TAR45;Note=intergenic; |
| Chr2 | PQ4 | transcript | 1548828 | 1548944 | ID=TAR46;Note=intergenic; |
| Chr2 | PQ4 | transcript | 1935718 | 1936000 | ID=TAR47;Note=intergenic; |
| Chr2 | PQ4 | transcript | 3249300 | 3249527 | ID=TAR48;Note=intergenic; |
| Chr2 | PQ4 | transcript | 3298297 | 3298408 | ID=TAR49;Note=EXON;Parent=AT2G07783.1; |
| Chr2 | PQ4 | transcript | 3322914 | 3323048 | ID=TAR50;Note=intergenic; |
| Chr2 | PQ4 | transcript | 3445672 | 3445815 | ID=TAR51;Note=intergenic; |
| Chr2 | PQ4 | transcript | 3467321 | 3467542 | ID=TAR52;Note=EXON;Parent=AT2G07731.1; |
| Chr2 | PQ4 | transcript | 3481225 | 3481332 | ID=TAR53;Note=EXON;Parent=AT2G07737.1; |
| Chr2 | PQ4 | transcript | 3484867 | 3484997 | ID=TAR54;Note=intergenic; |
| Chr2 | PQ4 | transcript | 3569664 | 3569816 | ID=TAR55;Note=intergenic; |
| Chr2 | PQ4 | transcript | 4829946 | 4830057 | ID=TAR56;Note=EXON;Parent=AT2G11950.1; |
| Chr2 | PQ4 | transcript | 6088997 | 6089104 | ID=TAR57;Note=EXON;Parent=AT2G14350.1; |
| Chr2 | PQ4 | transcript | 6280599 | 6280961 | ID=TAR58;Note=intergenic; |
| Chr2 | PQ4 | transcript | 6517415 | 6517684 | ID=TAR59;Note=EXON;Parent=AT2G15040.1; |
| Chr2 | PQ4 | transcript | 8779493 | 8779637 | ID=TAR60;Note=intergenic; |
| Chr2 | PQ4 | transcript | 9200271 | 9200454 | ID=TAR61;Note=EXON;Parent=AT2G21460.1; |
| Chr2 | PQ4 | transcript | 9200773 | 9200881 | ID=TAR62;Note=EXON;Parent=AT2G21460.1; |
| Chr2 | PQ4 | transcript | 9875188 | 9875367 | ID=TAR63;Note=intergenic; |
| Chr2 | PQ4 | transcript | 9930150 | 9930427 | ID=TAR64;Note=intergenic; |
| Chr2 | PQ4 | transcript | 12039762 | 12039992 | ID=TAR65;Note=intergenic; |
| Chr2 | PQ4 | transcript | 13057652 | 13057943 | ID=TAR66;Note=EXON;Parent=AT2G30640.1; |
| Chr2 | PQ4 | transcript | 13537101 | 13537248 | I D=TAR67; Note=intergenic; |
| Chr2 | PQ4 | transcript | 13696749 | 13696943 | ID=TAR68;Note=intergenic; |
| Chr2 | PQ4 | transcript | 14190472 | 14190596 | ID=TAR69;Note=intergenic; |
| Chr2 | PQ4 | transcript | 14997744 | 14997896 | I D=TAR70; Note=intergenic; |
| Chr2 | PQ4 | transcript | 15471977 | 15472104 | ID=TAR71;Note=intron;Parent=AT2G36850.1; |
| Chr2 | PQ4 | transcript | 15476590 | 15476699 | ID=TAR72;Note=intergenic; |
| Chr2 | PQ4 | transcript | 15887002 | 15887114 | ID=TAR73;Note=intergenic; |
| Chr2 | PQ4 | transcript | 16026588 | 16026762 | ID=TAR74;Note=intergenic; |
| Chr2 | PQ4 | transcript | 17273007 | 17273262 | ID=TAR75;Note=intergenic; |
| Chr2 | PQ4 | transcript | 17938590 | 17938771 | ID=TAR76;Note=intergenic; |
| Chr2 | PQ4 | transcript | 18173485 | 18173588 | ID=TAR77;Note=intergenic; |
| Chr3 | PQ4 | transcript | 1284836 | 1284978 | ID=TAR78;Note=EXON;Parent=AT3G04717.1; |
| Chr3 | PQ4 | transcript | 1285129 | 1285277 | ID=TAR79;Note=EXON;Parent=AT3G04717.1; |
| Chr3 | PQ4 | transcript | 1745188 | 1745474 | ID=TAR80;Note=EXON;Parent=AT3G05850.1; |
| Chr3 | PQ4 | transcript | 2027119 | 2027229 | ID=TAR81;Note=intron;Parent=AT3G06530.1; |
| Chr3 | PQ4 | transcript | 2031178 | 2031284 | ID=TAR82;Note=intron;Parent=AT3G06530.1; |
| Chr3 | PQ4 | transcript | 2191886 | 2192164 | ID=TAR83;Note=EXON;Parent=AT3G06940.1; |
| Chr3 | PQ4 | transcript | 3963549 | 3963916 | ID=TAR84;Note=EXON;Parent=AT3G12502.1; |
| Chr3 | PQ4 | transcript | 4701930 | 4702221 | ID=TAR85;Note=intergenic; |
| Chr3 | PQ4 | transcript | 5258935 | 5259044 | ID=TAR86;Note=intergenic; |
| Chr3 | PQ4 | transcript | 5838460 | 5838564 | ID=TAR87;Note=EXON;Parent=AT3G17110.1; |
| Chr3 | PQ4 | transcript | 7104312 | 7104478 | ID=TAR88;Note=EXON;Parent=AT3G20365.1; |
| Chr3 | PQ4 | transcript | 7493078 | 7493401 | ID=TAR89;Note=intergenic; |
| Chr3 | PQ4 | transcript | 7864201 | 7864348 | ID=TAR90;Note=intron;Parent=AT3G22240.1; |
| Chr3 | PQ4 | transcript | 8211626 | 8211728 | ID=TAR91;Note=intergenic; |
| Chr3 | PQ4 | transcript | 8937608 | 8937717 | ID=TAR92;Note=intergenic; |
| Chr3 | PQ4 | transcript | 12047115 | 12047292 | ID=TAR93;Note=EXON;Parent=AT3G30416.1; |
| Chr3 | PQ4 | transcript | 12513937 | 12514047 | ID=TAR94;Note=EXON;Parent=AT3G30817.1; |
| Chr3 | PQ4 | transcript | 13373195 | 13373606 | ID=TAR95;Note=EXON;Parent=AT3G32415.1; |
| Chr3 | PQ4 | transcript | 13600838 | 13600960 | ID=TAR96;Note=intergenic; |
| Chr3 | PQ4 | transcript | 13620240 | 13620347 | ID=TAR97;Note=intergenic; |
| Chr3 | PQ4 | transcript | 14810535 | 14810673 | ID=TAR98;Note=intergenic; |
| Chr3 | PQ4 | transcript | 15615992 | 15616134 | ID=TAR99;Note=intergenic; |
| Chr3 | PQ4 | transcript | 15784281 | 15784396 | ID=TAR100;Note=EXON;Parent=AT3G43955.1; |
| Chr3 | PQ4 | transcript | 16223301 | 16223406 | ID=TAR101;Note=intergenic; |
| Chr3 | PQ4 | transcript | 17200993 | 17201244 | ID=TAR102;Note=EXON;Parent=AT3G46660.1; |
| Chr3 | PQ4 | transcript | 18221160 | 18221276 | ID=TAR103;Note=intergenic; |
| Chr3 | PQ4 | transcript | 22060835 | 22061134 | ID=TAR104;Note=EXON;Parent=AT3G59695.1; |
| Chr3 | PQ4 | transcript | 22061356 | 22061467 | ID=TAR105;Note=EXON;Parent=AT3G59695.1; |
| Chr3 | PQ4 | transcript | 22061858 | 22062139 | ID=TAR106;Note=EXON;Parent=AT3G59695.1; |
| Chr3 | PQ4 | transcript | 22561326 | 22561538 | ID=TAR107;Note=EXON;Parent=AT3G60965.1; |
| Chr3 | PQ4 | transcript | 22659806 | 22659978 | ID=TAR108;Note=EXON;Parent=AT3G61185.1; |
| Chr4 | PQ4 | transcript | 1270801 | 1271002 | ID=TAR109;Note=intergenic; |
| Chr4 | PQ4 | transcript; | 2191910 | 2192020 | ID=TAR110;Note=EXON;Parent=AT4G04410.1; |
| Chr4 | PQ4 | transcript | 3964456 | 3964678 | ID=TAR111;Note=EXON;Parent=AT4G06708.1; |
| Chr4 | PQ4 | transcript | 6534652 | 6534858 | ID=TAR112;Note=EXON;Parent=AT4G10580.1; |
| Chr4 | PQ4 | transcript | 7512355 | 7512799 | ID=TAR113;Note=intergenic; |
| Chr4 | PQ4 | transcript | 7652127 | 7652246 | ID=TAR114;Note=intron;Parent=AT4G13150.1; |
| Chr4 | PQ4 | transcript | 8036799 | 8037139 | ID=TAR115;Note=EXON;Parent=AT4G13900.1; |
| Chr4 | PQ4 | transcript | 8199493 | 8199599 | ID=TAR116;Note=intergenic; |
| Chr4 | PQ4 | transcript | 8691795 | 8691900 | ID=TAR117;Note=intron;Parent=AT4G15233.1; |
| Chr4 | PQ4 | transcript | 8693266 | 8693442 | ID=TAR118;Note=intron;Parent=AT4G15233.1; |
| Chr4 | PQ4 | transcript | 9694115 | 9694330 | ID=TAR 119;Note=intergenic; |
| Chr4 | PQ4 | transcript | 9694696 | 9694808 | ID=TAR120;Note=intergenic; |
| Chr4 | PQ4 | transcript | 9695064 | 9695208 | ID=TAR121; Note=intergenic; |
| Chr4 | PQ4 | transcript | 9696801 | 9696944 | ID=TAR122;Note=intergenic; |
| Chr4 | PQ4 | transcript | 11852077 | 11852219 | ID=TAR123;Note=EXON;Parent=AT4G22505.1; |
| Chr4 | PQ4 | transcript | 11852395 | 11852697 | ID=TAR124;Note=EXON;Parent=AT4G22505.1; |
| Chr4 | PQ4 | transcript | 12284949 | 12285142 | ID=TAR125;Note=intergenic; |
| Chr4 | PQ4 | transcript | 12285391 | 12285816 | ID=TAR126;Note=intergenic; |
| Chr4 | PQ4 | transcript | 12285929 | 12286302 | ID=TAR127;Note=intergenic; |
| Chr4 | PQ4 | transcript | 12473584 | 12473794 | ID=TAR128;Note=intergenic; |
| Chr4 | PQ4 | transcript | 16891478 | 16891793 | ID=TAR129;Note=intergenic; |
| Chr5 | PQ4 | transcript | 84435 | 84592 | ID=TAR130;Note=EXON;Parent=AT5G01215.1; |
| Chr5 | PQ4 | transcript | 1164586 | 1164766 | ID=TAR131;Note=EXON;Parent=AT5G04235.1; |
| Chr5 | PQ4 | transcript | 1166221 | 1166360 | ID=TAR132;Note=EXON;Parent=AT5G04235.1; |
| Chr5 | PQ4 | transcript | 4150542 | 4150679 | ID=TAR133;Note=intron;Parent=AT5G13080.1; |
| Chr5 | PQ4 | transcript | 4709525 | 4709773 | ID=TAR134;Note=intergenic; |
| Chr5 | PQ4 | transcript | 5304974 | 5305082 | ID=TAR135;Note=EXON;Parent=AT5G16235.1; |
| Chr5 | PQ4 | transcript | 5305601 | 5305740 | ID=TAR136;Note=EXON;Parent=AT5G16235.1; |
| Chr5 | PQ4 | transcript | 5771411 | 5771522 | ID=TAR137;Note=intron;Parent=AT5G17510.1; |
| Chr5 | PQ4 | transcript | 5893629 | 5893746 | ID=TAFt138;Note=intron;Parent=AT5G17830.1; |
| Chr5 | PQ4 | transcript | 6937393 | 6937568 | ID=TAR139; Note=intergenic; |
| Chr5 | PQ4 | transcript | 7828239 | 7828571 | ID=TAR140;Note=EXON;Parent=AT5G23240.1; |
| Chr5 | PQ4 | transcript | 8213452 | 8213665 | ID=TAR141;Note=EXON;Parent=AT5G24206.1; |
| Chr5 | PQ4 | transcript | 8218090 | 8218204 | ID=TAR142;Note=intron;Parent=AT5G24210.1; |
| Chr5 | PQ4 | transcript | 8958642 | 8958862 | ID=TAR143;Note=intergenic; |
| Chr5 | PQ4 | transcript | 10019022 | 10019176 | ID=TAR144;Note=intergenic; |
| Chr5 | PQ4 | transcript | 10019359 | 10019652 | ID=TAR145;Note=intergenic; |
| Chr5 | PQ4 | transcript | 13105430 | 13105592 | ID=TAR146;Note=EXON;Parent=AT5G34851.1; |
| Chr5 | PQ4 | transcript | 14567664 | 14567777 | ID=TAR147;Note=intergenic; |
| Chr5 | PQ4 | transcript | 14906568 | 14906819 | ID=TAR148;Note=intergenic; |
| Chr5 | PQ4 | transcript | 15649299 | 15649545 | ID=TAR149;Note=intergenic; |
| Chr5 | PQ4 | transcript | 16054202 | 16054301 | ID=TAR150;Note=EXON;Patent=AT5G40060.1; |
| Chr5 | PQ4 | transcript | 16745478 | 16745587 | ID=TAR151;Note=intergenic; |
| Chr5 | PQ4 | transcript | 17152037 | 17152359 | ID=TAR152;Note=intergenic; |
| Chr5 | PQ4 | transcript | 17971508 | 17971660 | ID=TAR153;Note=intergenic; |
| Chr5 | PQ4 | transcript | 18002454 | 18002633 | ID=TAR154;Note=intergenic; |
| Chr5 | PQ4 | transcript | 19159268 | 19159412 | ID=TAR155;Note=intergenic; |
| Chr5 | PQ4 | transcript | 19873174 | 19873392 | ID=TAR156;Note=EXON;Parent=AT5G48965.1; |
| Chr5 | PQ4 | transcript | 19873538 | 19873653 | ID=TAR157;Note=EXON;Parent=AT5G48965.1; |
| Chr5 | PQ4 | transcript | 20921928 | 20922221 | ID=TAR158;Note=intergenic; |
| Chr5 | PQ4 | transcript | 21549835 | 21549947 | ID=TAR159;Note=intergenic; |
| Chr5 | PQ4 | transcript | 21717504 | 21717608 | ID=TAR160;Note=intergenic; |
| Chr5 | PQ4 | transcript | 22355958 | 22356226 | ID=TAR161;Note=EXON;Parent=AT5G55050.1; |
| Chr5 | PQ4 | transcript | 22479122 | 22479371 | ID=TAR162;Note=EXON;Parent=AT5G55420.1; |
| Chr5 | PQ4 | transcript | 22479505 | 22479770 | ID=TAR163;Note=intergenic; |
| Chr5 | PQ4 | transcript | 23766482 | 23766590 | ID=TAR164;Note=EXON;Parent=AT5G58810.1; |
| Chr5 | PQ4 | transcript | 24186552 | 24186699 | ID=TAR165;Note=EXON;Parent=AT5G60022.1; |
| Chr5 | PQ4 | transcript | 24186836 | 24187127 | ID=TAR166;Note=EXON;Parent=AT5G60022.1; |
| Chr5 | PQ4 | transcript | 24279446 | 24279822 | ID=TAR167;Note=EXON;Parent=AT5G60285.1; |
| Chr5 | PQ4 | transcript | 24422361 | 24422543 | ID=TAR168;Note=intergenic; |
| Chr5 | PQ4 | transcript | 24788305 | 24788415 | ID=TAR169;Note=intergenic; |
| ChrM | PQ4 | transcript | 40160 | 40440 | ID=TAR170;Note=intergenic; |
| ChrM | PQ4 | transcript | 245983 | 246093 | ID=TAR171;Note=intergenic; |
| ChrM | PQ4 | transcript | 246645 | 246745 | ID=TAR172;Note=intergenic; |
| ChrM | PQ4 | transcript | 286404 | 286544 | ID=TAR173;Note=intergenic; |
| ChrM | PQ4 | transcript | 288554 | 288724 | ID=TAR174;Note=intron;Parent=ATMG01120.1; |
| ChrM | PQ4 | transcript | 316831 | 316937 | ID=TAR175;Note=intergenic; |

### EXAMPLES

The following examples are provided for the purpose of illustrating the present invention and should in no way be interpreted as limiting the scope thereof.

### Example 1. Identification of OSIP genes in A. thaliana using tiling arrays.

*Plant material, growth conditions and treatments -* Seeds of *Arabidopsis thaliana* wild-type ecotype Columbia-0 (Col-0) were obtained from the European *A. thaliana* stock Centre (NASC; http://nasc.nott.ac.uk/home.html). After sowing, they were grown in a growth chamber with a 21°C daytime temperature, 18°C night-time temperature, 75% humidity and a 12h day-light cycle with a light intensity of approximately 120µmol/m²s.

Rosette leaves from soil-grown *A. thaliana* plants were sprayed with paraquat (25µM methylviologendichlorodehydrate 98%) to obtain an equal and proportional distribution. Control plants were mock treated by spraying with H₂O. The treated plants were then kept at 100% relative humidity for 48 hours. Then, all plant material located above ground surface was harvested in liquid nitrogen and stored at -80°C prior to further analysis.

*Isolation of total RNA* - RNA was isolated from approximately 100 mg plant material per sample by the guanidinium isothiocyanate method (TRIzol reagent; Invitrogen, Carlsbad, USA) followed by purification using the RNeasy Mini Kit (Qiagen, Valencia, USA) according to the manufacturers' instructions.

*Target preparation and tiling array hybridization -* The mRNA samples were labeled and hybridized onto GeneChip® Arabidopsis Tiling 1.0R Arrays (Affymetrix, Santa Clara, USA). Two independent biological samples were used for each condition to create biological repeats. Scanning and image processing were performed using Affymetrix equipment and software.

*Identification of short unannotated and paraquat-induced genomic regions -* Background correction, log₂ transformation and interslide normalization were performed with the Affymetrix Tiling Analysis Software - Version 1.1, Build 2. The resulting tiling array data (consisting of chromosome positions of probes linked to normalized log₂ transformed intensities for both paraquat and control samples), the analysis results and all required information (e.g. *A. thaliana* genome annotation) were stored in a MySQL database. Since the tiling arrays were based on the *A*. *thaliana* genome sequence of the 2007's version of the TAIR database (20), the corresponding TAIR7 genome annotation was used for the analysis of the tiling array data.

Selection and identification of transcriptionally active regions included the following steps:
(i) intensities of biological replicates were combined and a threshold value of 8.6 was chosen to identify transcriptionally active probes based on both the tiling array intensities of genomic regions with known expression status during the paraquat and control treatment and the results of control qRT-PCR experiments. Individual probes with intensities below the threshold that were surrounded by transcriptionally active probes were considered active and vice versa to remove the effect of probes with poor hybridization properties.
(ii) groups of 4-13 successive transcriptionally active probes were combined into short transcriptionally active regions. Selection of even smaller regions has several disadvantages, including a high number of false positives and the identification of very small genes that are difficult to amplify in later stages. All active regions of 14-15 successive probes were already annotated.
(iii) transcriptionally active regions were withheld if the number of transcriptionally active probes was smaller in the control than in the paraquat treatment and if the average intensity of all probes in the smallest of the two regions was at least 1.4 higher in the paraquat treatment than in the control. Both rules were based on the results for genomic regions that were known to be induced upon paraquat treatment. The former rule was added because of the frequent occurrence of probes having zero intensity in one of the four hybridizations. In small regions these zero values can have substantial influence on the average intensity leading to false positive calls.
(iv) each selected region was compared to the *A*. *thaliana* TAIR7 genome annotation and only regions that were located in intergenic regions, introns or pseudogenes were withheld.

*Selection of open reading frames-* Using the TAIR7 genome sequence, a fasta file containing the corresponding DNA sequences of all selected short unannotated and paraquat-induced regions were retrieved. On each side of the active region an extra sequence of 50 bp was selected to minimize the chance of missing the start or stop codon.

Results - Leaf samples were collected at 48h after paraquat treatment from both 4-weeks old treated *A. thaliana* Col0 plants and their corresponding control plants upon mock-treatment with water. Two biological replicates were performed. From all plant samples high quality mRNA was isolated, labeled and used for hybridization on GeneChip® Arabidopsis tiling 1.0R arrays (Affymetrix). Log₂ transformed normalized microarray data of the two biological replicates were combined and used to identify transcriptionally active regions (TARs) in the *Arabidopsis* genome based on a threshold that was defined using control genomic regions with known expression status in the treatments under study. This way, a total of 92,844 and 86,272 TARs were identified in the paraquat and the control data set respectively. Short unannotated TARs that were induced by the paraquat treatment as compared to the control treatment were selected, again using a threshold that was based on the transcriptional behaviour of a set of appropriate controls, resulting in the identification of 176 Oxidative Stress Induced Peptides (OSIP)-encoding regions. The bulk of these TARs were intergenic, although 70 and 18 OSIP-encoding regions were located in pseudogenes and introns of annotated genes respectively. The corresponding DNA sequences of the selected TARs were translated in the six possible reading frames with the ExPaSy translate tool of the Swiss Institute of Bioinformatics (http://www.expasy.ch/tools/dna.html). Open reading frames (ORFs) of at least 30 bp (10 amino acids) were retained, resulting in a list of 575 potential OSIP-encoding genes, ordered based on their induction level (Fig. 1, Table 1). Peptide sequences shorter than 10 amino acids or located entirely in the outer 50 bp were discarded.

### Example 2. Overexpression of OSIPs results in increased peroxide MIC values of yeast.

*Primer Design -* The DNA sequences of the open reading frames were extended with 50 extra nucleotides after the stop codon to increase the choice possibilities for the reverse primer. The design of specific primers for each selected ORF was done with Primer3 software (21). To allow for transformation using Gateway® technology the sequence CACCAAAA was added to each forward primer.

*Materials and yeast strains -* Yeast strain used was *Saccharomyces cerevisiae* strain BY4741 (wild-type, WT) (Euroscarf, Germany), which was cultivated in Yeast Peptone Dextrose (YPD) (1% yeast extract, 2% peptone, 2% glucose). Yeast strains transformed with the pYES-DEST52 Gateway^{®} vector (Invitrogen, Carlsbad, USA) were cultured in SC-ura (0.8 g/l CSM-ura, complete amino acid supplement mixture minus uracil, Bio 101 Systems; 6.5 g/l YNB, yeast nitrogen base; 20 g/l glucose). Oxidative stress induced peptides (OSIPs) were synthesized by the addition of galactose to the growth medium.

*Construction of a mini library in yeast -* The coding sequences of the potential OSIP genes were cloned into the pENTR^{™} plasmid (Invitrogen, Carlsbad, USA) using the pENTR^{™}/D-TOPO^{®} cloning system according to the manufacturer's instructions (Invitrogen) and the presence of each ORF in the resulting pool of *E*. *coli* transformants was investigated by PCR with the individual primers. The inserts were then transferred to the pYES-DEST52 Gateway^{®} vector and subsequently used for transformation to *Sacharomyces cerevisiae* BY4741 using the Gietz protocol (22).

*Oxidative stress assays in yeast-* Oxidative stress tolerance of individual yeast transformants was examined by determination of de minimal inhibitory concentration (MIC) for H₂O₂, using a twofold dilution serie (0-20mM) in SC-ura (Gal) and 1/200 yeast inoculum of an overnight culture in SC-ura (Glu).

To investigate an active role in oxidative stress resistance, the full coding sequences of the potential OSIP genes were individually amplified by PCR, pooled, and used for transformation of yeast using the pENTR^{™}/D-TOPO^{®} cloning system (Invitrogen, Carlsbad, USA). Overexpression of the OSIP gene was activated by the addition of galactose to the growth medium. As paraquat induces oxidative stress in yeast during respiration, thus upon cultivation in glycerol, it was not possible to screen for yeast transformants with increased tolerance against paraquat. Therefore, we screened the corresponding yeast transformants for improved tolerance against oxidative stress induced by peroxide in galactose-containing medium by determining the minimal inhibitory concentration (MIC) of peroxide. As positive controls, we cloned two known *A. thaliana* peptides that govern oxidative stress tolerance in gist, namely LEA5 and AtMtATP6 (8, 9). To this end, for at least 500 individual yeast transformants. In this way, we selected 9 yeast transformants that were characterized by a consistent 2-fold increased peroxide MIC value in galactose-containing medium (MIC = 5mM peroxide), compared to the other yeast transformants (MIC = 2.5mM peroxide). A similar increase in MIC value was observed for yeast transformants overexpressing LEA5 or AtMtATP6.

Sequence analysis revealed that these nine transformants contained seven different constructs, with two constructs occurring twice in our selection. The sequences of these seven constructs, SIP108_4 (further SIP108 or OSIP108), SIP152_2 (further SIP152 or OSIP152), SIP163_3 (further SIP163 or OSIP163), SIP14_2, SIP14_1, SIP11_0 (further SIP11 or OSIP11) and SIP37_1 (further SIP37 or OSIP37) are shown in Table 3 and 4.

The log₂ ratios of OSIP108, OSIP163 and OSIP152 upon paraquat treatment in the tiling array were 5.42; 5.07 and 4.74, respectively. To validate these expression data, we next assessed expression levels of the selected 3 OSIPs using qRT-PCR analysis under the same conditions and at the same time point as used in the tiling array analysis. The log₂ ratios of OSIP108, OSIP163 and OSIP152 assessed were 5.32, 5.68 and 5.00, respectively. Hence, the resulting qRT-PCR data are in line with the normalized log ratios from the tiling arrays, thereby confirming the observed gene expression patterns.

### Example 3. Overexpression of OSIP108 governs oxidative stress tolerance in yeast

Resistance of the selected yeast transformants and the two positive control transformants against oxidative stress induced by H₂O₂ was additionally assayed in a halo test, by spotting 15 µL of 240 mM H₂O₂ on SC-ura agar, containing either glucose or galactose, and inoculated with 1/200 of overnight yeast culture as decribed previously (23). After 48 h of incubation at 30 °C, diameters of the halos were measured.

The halo-test comprises a predefined but continuous concentration gradient of H₂O₂, applied to inoculated agar plates. Upon incubation, circles (halos) of growth inhibition are visible. By comparing the diameter of the halos of plates with yeast grown on glucose (not allowing for expression of the OSIP) and galactose (inducing overexpression of the OSIP) the effect of the OSIP on oxidative stress tolerance was quantified. The average difference in halo diameter obtained on galactose-containing agar inoculated with OSIP108-overexpressing yeast was significantly lower as compared to yeast transformants overexpressing negative controls or SIP152 or SIP163 (Fig. 2). SIP14_2, SIP14_1 and SIP11 also showed significant enhanced tolerance in this assay.

### Example 4. Exogenous application of OSIP108 to yeast cultures reduces the ROS accumulation and increases survival upon peroxide treatment.

*ROS accumulation assay and survival assay* - ROS, after H₂O₂ treatment in the absence or presence of 500µM OSIP, were determined using 2,7-dichlorodihydrofluorescein diacetate (H₂DCFDA) staining as described previously (24). Survival of WT yeast in PBS following H₂O₂ treatment in the absence or presence of 500µM OSIP was assessed as described (25).

The effect of OSIP108 on endogenous ROS levels induced by peroxide in yeast were evaluated. Peroxide-induced ROS in yeast in the absence and presence of OSIP108 were measured using 2,7-dichlorodihydrofluorescein diacetate (H₂DCFDA) (Fig. 3A). The fluorescence intensity of 2',7'-dichlorofluorescein (DCF) yeast cultures treated with different concentrations of peroxide was significantly lower in the presence of 500 µM OSIP108 compared to the mock treatment. Moreover, we found the survival of peroxide-treated yeast cells (5mM) to increase 27-fold upon incubation with 500 µM OSIP108 (Fig. 3B).

SIP14_2, SIP14_1 and SIP11 also resulted in a decrease in the amount of endogenous ROS in the yeast cells. However, they showed less effect than SIP108 on the survival of the yeast cells upon addition of the peptides and after treatment with peroxide.

### Example 5. Effect of OSIP108 on yeast growth inhibition induced by apoptosis-inducing compounds

As induction of oxidative stress and apoptosis are closely linked, we investigated the effect of OSIP108 and its inactive variant OSIP(C3A) on *Saccharomyces cerevisiae* growth in the presence of various apoptosis-inducers and mitochondrial toxins like copper, valproic acid, tunicamycin, doxycyclin and cisplatin. In this respect, in parallel with mitochondrial copper toxicity, the use of the other mitochondrial toxins, *in casu* valproic acid, tunicamycin, doxycyclin and cisplatin, expand the range of causes of mitochondrial dysfunction and help find a broader basis for compounds rescuing mitochondrial dysfunction. Optical density (OD600) of yeast BY4741 was monitored upon treatment with different apoptosis-inducing compounds in the presence or absence of 200µM OSIP108. OSIP108 and apoptosis-inducers were added simultaneously to the yeast cells. Percentage growth was calculated as the ratio of the OD600 of the treated culture over an unstressed yeast culture after 10h or 48h of incubation. Treatment of yeast cells with 200uM OSIP108 results in significant increase in growth, compared to control treatment, in presence of 250 and 125µM cisplatin whereas OSIP108 fails to rescue yeast growth inhibition upon treatment with other apoptosis-inducers (Fig. 4). Also, OSIP108 but not OSIP(C3A) can rescue copper- and cisplatin-induced growth inhibition of yeast

### Example 6. Exogenous application of OSIP908 to A. thaliana leaves increases paraquat tolerance.

At least twelve soil grown, 4 week old Col-0 *A*. *thaliana* plants, were used in which one leaf of each plant was syringe-infiltrated with 100 µM OSIP108 dissolved in 1% dimethyl sulphoxide (DMSO). At least 12 control plants were mock treated with 1% DMSO only. After 24h incubation in the growth chamber, the plants were inoculated with a drop of 5 µl paraquat (100 µM). The lesion diameters caused by the paraquat treatment were measured and compared 2 and 3 days after the treatment.

Three days after paraquat application, the average lesion diameter of plants pretreated with OSIP108 (2.18±1.60mm, p=0.002) was significantly lower than the average lesion diameter of the control plants (4.45±1.37mm). These data confirm the ability of OSIP108 to increase oxidative stress tolerance not only in yeast, but also in plants.

### Example 7. Effect of OSIP108 on cell survival of HepG2 cells in the presence of copper or cisplatin.

The human hepatoma cell line HepG2 which naturally expresses hATP7B was incubated with 100 µM of peptide OSIP108. The viability of cells was determined after 48h exposure to toxic copper concentrations of 0.75 mM by MTT assay. The viability was calculated relative to cells that receive no copper (100%).

Pre-incubation of the hepatoma cell line HepG2 with OSIP108 resulted in increased survival of the cells in the presence of copper or cisplatin (Fig. 5). The inactive OSIP108(C3A) was used as a negative control.

### Example 8. Effect of endogenous or exogenous application of OSIP108 on copper-induced growth inhibition of Δccc2.

Copper ions are known to induce apoptosis of hepatocytes, one of the underlying mechanisms of copper-induced toxicity in Wilson's Disease (WD). WD is caused by mutations in the ATPase ATP7b. We found that overexpression of the gene encoding OSIP108 in a yeast Δccc2 mutant, which is devoid of the ortholog of ATP7b, could alleviate copper-induced growth inhibition of this mutant.

Exogenous addition of OSIP108 to Δccc2 yeast mutant cultures in the presence of a lethal copper dose also induces survival of the yeast mutant. OSIP108 reduced the copper-induced accumulation of reactive oxygen species (ROS) and increased viability of the yeast mutant. This was tested by spotting 5µL of 10mM. OSIP108 onto agar plates containing a yeast Δccc2 mutant, 500µM CuSO₄ and a viability dye based on MTT. Plates were incubated for 24h and placed at 4°C for 2 days. Blue halos were indicative of surviving yeast cells.

### Example 9. Effect of OSIP108 on Wilson's Disease model cell lines

CHO cell lines expressing either ATP7b or ATP7b mutant H1069Q (a mutation frequently found in patients having Wilson's Disease) were incubated with OSIP108 (100 µM). The viability of cells was determined after 48h exposure to toxic copper concentrations of 0.75 mM by MTT assay and calculated relative to cells that receive no copper (100%). Incubation of CHO cell lines expressing either ATP7b or ATP7b mutant H1069Q with OSIP108 enhanced the copper resistance of the cell lines and increased their viability in the presence of copper (Fig. 6)

### References

1. Berliner, J. A., Navab, M., Fogelman, A. M., Frank, J. S., Demer, L. L., Edwards, P. A., Watson, A. D., and Lusis, A. J. (1995) Circulation 91, 2488-2496
2. Dexter, D. T., Carter, C. J., Wells, F. R., Javoy-Agid, F., Agid, Y., Lees, A., Jenner, P., and Marsden, C. D. (1989) J Neurochem 52, 381-389
3. Low, P. S., and Merida, J. R. (1996) Physiologia Plantarum 96, 533-542
4. Smith, M. A., Perry, G., Richey, P. L., Sayre, L. M., Anderson, V. E., Beal, M. F., and Kowall, N. (1996) Nature 382, 120-121
5. Lee, C. Y., Lee, C. H., Shih, C. C., and Liou, H. H. (2008) Biochem Pharmacol 76, 1155-1164
6. Yang, W., and Tiffany-Castiglioni, E. (2005) J Toxicol Environ Health A 68, 1939-1961
7. Cunningham, T. J., Jing, H., Akerblom, I., Morgan, R., Fisher, T. S., and Neveu, M. (2002) Exp Neurol 177, 32-39
8. Zhang, X., Liu, S., and Takano, T. (2008) Biotechnol Lett 30, 1289-1294
9. Mowla, S. B., Cuypers, A., Driscoll, S. P., Kiddle, G., Thomson, J., Foyer, C. H., and Theodoulou, F. L. (2006) Plant J 48, 743-756
10. Fromenty, B., Robin M.A., Igoudjil, A., Mansouri, A., Pessayre, D. (2004), Diabetes Metab 30: 121-138.
11. Krens et al. (1982) Nature 296, 72-74
12. Shillito et al. (1985) Bio/Technol. 3, 1099-1102
13. Crossway et al. (1986) Mol. Gen. Genet. 202, 179-185
14. Klein et al. (1978) Nature 327, 70
15. Caruthers et al. (1982) Cold Spring Harbor Symp. Quant. Biol. 47, 411-418
16. Adams et al. (1983) J. Am. Chem. Soc. 105, 661.
17. Buchman and Berg (1988) Mol. Cell biol. 8: 4395-4405
18. Callis et al. (1987) Genes Dev 1:1 183-1200)
20. Swarbreck, D., Wilks, C., Lamesch, P., Berardini, T. Z., Garcia-Hernandez, M., Foerster, H., Li, D., Meyer, T., Muller, R., Ploetz, L., Radenbaugh, A., Singh, S., Swing, V., Tissier, C., Zhang, P., and Huala, E. (2008) Nucleic Acids Res 36, D1009-1014
21. Rozen, S., and Skaletsky, H. (2000) Methods Mol Biol 132, 365-386
22. Gietz, R. D., Schiestl, R. H., Willems, A. R., and Woods, R. A. (1995) Yeast 11, 355-360
23. Aerts, A. M., Francois, I. E., Bammens, L., Cammue, B. P., Smets, B., Winderickx, J., Accardo, S., De Vos, D. E., and Thevissen, K. (2006) FEBS Lett 580, 1903-1907
24. Francois, I. E., Bink, A., Vandercappellen, J., Ayscough, K. R., Toulmay, A., Schneiter, R., van Gyseghem, E., Van den Mooter, G., Borgers, M., Vandenbosch, D., Coenye, T., Cammue, B. P., and Thevissen, K. (2009) J Biol Chem 284, 32680-32685
25. Bink, A., Govaert, G., Francois, I. E., Pellens, K., Meerpoel, L., Borgers, M., Van Minnebruggen, G., Vroome, V., Cammue, B. P., and Thevissen, K. (2010) FEMS Yeast Res 10, 812-818

## Claims

1. An isolated peptide comprising an amino acid sequence with at least 70% sequence identity to amino acid sequence SEQ ID No. 1; wherein the isolated peptide increases the oxidative stress tolerance of a cell or organism under oxidative stress conditions.

2. The isolated peptide according to claim 1, wherein the isolated peptide increases the viability of cell under oxidative stress conditions or in the presence of an agent inducing mitochondrial dysfunction.

3. An isolated nucleic acid encoding for the peptide according to claims 1 or 2.

4. The isolated nucleic acid according to claim 4 comprising a nucleotide sequence with at least 70% sequence identity to nucleotide sequence SEQ ID NO:576.

5. A genetic construct comprising the following operably linked DNA elements: (a) a nucleic acid according to claims 3 or 4; (b) one or more control sequences capable of driving expression of said nucleic acid (c) a 3' end region comprising a transcription termination sequence.

6. A method for increasing the oxidative stress tolerance of a cell or organism comprising contacting or transfecting said cell or organism with a peptide according to claims 1 or 2, a peptidomimetic thereof or a nucleic acid encoding therefor.

7. The method according to claim 6, comprising introducing and expressing into a cell or non-human organism a genetic construct according to claim 5.

8. The method according to claim 6 or 7 wherein said cell or organism is an eukaryotic cell or organism.

9. The method according to claim 8 wherein said cell or organism is a plant cell or plant, or an eukaryotic microbial cell or organism.

10. A transgenic cell or non-human organism having increased tolerance to oxidative stress or having increased viability under oxidative stress conditions or in the presence of an agent inducing mitochondrial dysfunction relative to the corresponding wild-type cell or non-human organism wherein said transgenic cell or non-human organism is transfected with and expresses the genetic construct according to claim 5.

11. The transgenic cell or non-human organism according to claim 10 wherein said cell or organism is a plant cell or plant or part thereof, or an eukaryotic microbial cell or organism.

12. Harvestable parts, including seeds, of the transgenic plant according to claim 11.

13. The peptide according to claims 1 or 2 or peptidemimetic thereof for use in the treatment or prevention of a mammal having an oxidative stress related disorder.

14. The peptide according to claim 12 wherein said oxidative stress related disorder is a mitochondrial dysfunction related disorder.

15. A pharmaceutical composition comprising (i) a peptide according to claims 1 or 2, or peptidomimetic thereof and (ii) one or more pharmaceutically acceptable compounds, carriers and/or adjuvants.

## Patentansprüche

1. Ein isoliertes Peptid, das eine Aminosäuresequenz enthält, welche eine Sequenzidentität von mindestens 70 % mit Aminosäuresequenz SEQ ID Nr. 1 aufweist, wobei das isolierte Peptid die oxidative Stresstoleranz von unter oxidativem Stress stehenden Zellen oder Organismen steigert.

2. Das isolierte Peptid gemäß Anspruch 1, wobei das isolierte Peptid die Viabilität von Zellen steigert, die unter oxidativem Stress oder unter dem Einfluss potenzieller Auslöser einer mitochondrialen Dysfunktion stehen.

3. Eine isolierte Nukleinsäure, die für das Peptid der Ansprüche 1 oder 2 kodiert.

4. Die isolierte Nukleinsäure gemäß Anspruch 4, die eine Nukleotidsequenz enthält, welche eine Sequenzidentität von mindestens 70 % mit Nukleotidsequenz SEQ ID Nr. 576 aufweist.

5. Ein genetisches Konstrukt, das die folgenden, operativ verbundenen DNA-Elemente aufweist: (a) eine Nukleinsäure gemäß den Ansprüchen 3 oder 4, (b) mindestens eine Kontrollsequenz, welche die Expression der betreffenden Nukleinsäure zu steuern vermag und (c) einen 3'-Endbereich, der eine Transkriptionsterminationssequenz enthält.

6. Ein Verfahren zur Steigerung der oxidativen Stresstoleranz von Zellen oder Organismen, das eine Kontaktierung oder Transfektion der betreffenden Zellen und Organismen mit einem Peptid gemäß den Ansprüchen 1 oder 2, eine Peptidomimetikum desselben oder eine zur entsprechenden Kodierung vorgesehene Nukleinsäure enthält.

7. Das Verfahren gemäß Anspruch 6, das die Einführung oder Expression eines genetischen Konstrukts gemäß Anspruch 5 in eine Zelle oder einen nicht-menschlichen Organismus umfasst.

8. Das Verfahren gemäß einem der Ansprüche 6 oder 7, wobei es sich bei den betreffenden Zellen und Organismen um eukaryontische Zellen bzw. Organismen handelt.

9. Das Verfahren gemäß Anspruch 8, wobei es sich bei den betreffenden Zellen und Organismen um pflanzliche Zellen bzw. Organismen oder um eukaryontische Mikrobenzellen bzw. - Organismen handelt.

10. Eine transgene Zelle oder ein transgener, nicht-menschlicher Organismus, die / der im Vergleich mit entsprechenden Wildtypzellen oder -organismen eine gesteigerte Toleranz gegenüber oxidativem Stress oder eine gesteigerte Viabilität unter oxidativem Stress oder unter dem Einfluss potenzieller Auslöser einer mitochondrialen Dysfunktion aufweist, wobei die betreffenden transgenen Zellen oder nicht-menschlichen Organismen mit einem genetischen Konstrukt gemäß Anspruch 5 transfiziert wurden und dieses Konstrukt exprimieren.

11. Die transgene Zelle oder der transgene, nicht-menschliche Organismus gemäß Anspruch 10, wobei es sich bei den betreffenden Zellen und Organismen um pflanzliche Zellen und Organismen oder um Teile pflanzlicher Zellen und Organismen oder um eukaryontische Mikrobenzellen bzw. -organismen handelt.

12. Erntbare Teile, einschließlich von Samen, von transgenen Pflanzen gemäß Anspruch 11.

13. Das Peptid gemäß einem der Ansprüche 1 oder 2 sowie dessen Peptidemimetika zur Verwendung für die Behandlung oxidativer Stresssymptome bei Säugetieren oder für eine einschlägige Vorbeugung.

14. Das Peptid gemäß Anspruch 12, wobei es sich bei den besagten oxidativen Stresssymptomen um eine mitochondriale Dysfunktion oder eine verwandte Störung handelt.

15. Eine pharmazeutische Zusammensetzung, die aus den folgenden Elementen besteht: (i) einem Peptid gemäß einem der Ansprüche 1 oder 2 bzw. dessen Peptidemimetika und (ii) einem oder mehreren pharmazeutisch annehmbaren Verbindungen, Träger- und/oder Hilfsstoffen.

## Revendications

1. Un peptide isolé comprenant une séquence d'acide aminé avec au moins 70 % d'identité de séquence d'acides aminés SEQ ID No. 1; où un peptide isolé augmente la tolérance au stress oxydatif d'une cellule ou d'un organisme sous des conditions de stress oxydatif.

2. Le peptide isolé selon la revendication 1, où le peptide isolé augmente la viabilité de la cellule sous des conditions de stress oxydatif ou en présence d'un agent induisant un dysfonctionnement mitochondrial.

3. Un encodage d'acide nucléique isolé pour le peptide selon les revendications 1 ou 2.

4. L'acide nucléique isolé selon la revendication 4 comprenant la séquence nucléotidique 70 % de l'identité de la séquence SEQ ID NO :576.

5. Une construction génétique comprenant les éléments d'ADN fonctionnellement liés : (a) un acide nucléique selon la revendication 3 ou 4 ; (b) une ou plusieurs séquences de contrôle capable de diriger l'expression dudit acide nucléique (c) une zone d'extrémité 3' comprenant une séquence de terminaison de transcription.

6. Une méthode pour augmenter la tolérance au stress oxydatif d'une cellule ou d'un organisme comprenant le contact ou la transfection de ladite cellule ou organisme avec un peptide selon la revendication 1ou 2, un peptidomimétique d'un encodage d'acide nucléique.

7. La méthode selon la revendication 6, comprenant l'introduction et l'expression dans une cellule ou dans un organisme non humain d'une construction génétique selon la revendication 5.

8. La méthode selon les revendications 6 ou 7 ou ladite cellule ou organisme est une cellule ou un organisme eucaryote.

9. La méthode selon la revendication 8 ou ladite cellule ou organisme est une cellule végétale ou une plante, ou une cellule ou un organisme microbien eucaryote.

10. Une cellule transgénique ou un organisme non humain ayant une tolérance plus importante au stress oxydatif ou en ayant une meilleure viabilité sous des conditions de stress oxydatif ou en présence d'un agent induisant un dysfonctionnement mitochondrial relatif à la cellule sauvage correspondante ou à l'organisme non humain ou ladite cellule antigénique ou l'organisme non humain et transmet et exprime la construction génétique selon la revendication 5.

11. La cellule transgénique ou l'organisme non humain selon la revendication 10 ou ladite cellule ou ledit organisme est une cellule végétale ou une plante ou une partie de celle-ci, où une cellule ou un organisme microbien eucaryote.

12. Des parties récoltables, y compris des graines, de la plante transgénique selon la réclamation 11.

13. Le peptide selon la réclamation 1 ou 2 ou peptidomimétique pour l'utilisation dans le traitement ou la prévention des mammifères ayant un trouble lié au stress oxydatif.

14. Le peptide selon la réclamation 12 ou le trouble lié au stress oxydatif est un dysfonctionnement mitochondrial lié à ce trouble.

15. Une composition pharmaceutique comprenant (i) un peptide selon les réclamations 1 ou 2, ou un peptidomimétique et (ii) un ou plusieurs composés pharmaceutiques acceptables, excipients et/ou adjuvants.
